(19) Europäisches Patentamt
European Patent Office
Office européen des brevets

(11) EP 2 234 987 B1

(12) # EUROPÄISCHE PATENTSCHRIFT

(45) Veröffentlichungstag und Bekanntmachung des Hinweises auf die Patenterteilung:
**12.12.2012 Patentblatt 2012/50**

(21) Anmeldenummer: **08864093.3**

(22) Anmeldetag: **19.12.2008**

(51) Int Cl.:
***C07D 241/44*** *(2006.01)*

(86) Internationale Anmeldenummer:
**PCT/EP2008/068000**

(87) Internationale Veröffentlichungsnummer:
**WO 2009/080745 (02.07.2009 Gazette 2009/27)**

(54) **PERHYDROCHINOXALIN-DERIVATE ALS ANALGETIKA**

PERHYDROQUINOXALINE DERIVATIVES AS ANALGESICS

DÉRIVÉS DE PERHYDROQUINOXALINE EN TANT QUE ANLGÉSIQUES

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **20.12.2007 DE 102007062550**

(43) Veröffentlichungstag der Anmeldung:
**06.10.2010 Patentblatt 2010/40**

(73) Patentinhaber: **Dr. August Wolff GmbH & Co. KG Arzneimittel**
**33611 Bielefeld (DE)**

(72) Erfinder:
- **WÜNSCH, Bernhard**
  **48161 Münster (DE)**
- **SCHEPMANN, Dirk**
  **48147 Münster (DE)**
- **BOURGEOIS, Christian**
  **59379 Selm (DE)**

(74) Vertreter: **Hollatz, Christian**
**ter Meer Steinmeister & Partner GbR**
**Patentanwälte**
**Mauerkircherstrasse 45**
**81679 München (DE)**

(56) Entgegenhaltungen:
**US-A1- 2002 042 399 US-B1- 6 303 611**

- **REES ET AL: "Synthesis of perhydro-2 (1H)-quinoxalinones and perhydropyrrolo[1,2-a] quinoxalin-45H)-one derivatives" JOURNAL OF HETEROCYCLIC CHEMISTRY, HETEROCORPORATION. PROVO, US, Bd. 24, 1. Januar 1987 (1987-01-01), Seiten 1297-1300, XP009121213 ISSN: 0022-152X**
- **GIARDIN A D ET AL: "Synthesis and biological profile of the enantiomers of [4-(4-amino-6,7-dimethoxyquinazolin -2-yl)-cis-octahydroquinoxalin - 1-yl]furan-2-ylmethanone (cyclazosin), a potent competitive alpha 1B-adrenoceptor antagonist" JOURNAL OF MEDICINAL CHEMISTRY, AMERICAN CHEMICAL SOCIETY, WASHINGTON, US, Bd. 39, Nr. 23, 8. November 1996 (1996-11-08), Seiten 4602-4607, XP002152418 ISSN: 0022-2623**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Perhydrochinoxalin-Derivate sowie Arzneimittel enthaltend Perhydrochinoxalin-Derivaie.

Schmerzen sind unangenehme Sinnes- oder Gefühlserlebnisse, die eine lebenswichtige Schutz- und Warnfunktion haben und mit einer tatsächlichen oder drohenden Gewebeschädigung einhergehen können. Nach ihrer Entstehung unterscheidet man die Schmerzwahmehmung, beispielsweise in periphere oder zentrale Schmerzen.

Dem Körper werden Schmerzen über Rezeptoren im Nervensystem signalisiert, wobei das Schmerzempfinden des Patienten subjektiv ist.

Die Behandlung von Schmerzen hat in der Medizin große Bedeutung. Schmerzstillende Mittel wirken in der Regel durch eine Blockierung von Opioid-Rezeptoren. So sind klassische Opioide wie Morphin Opioid-Analgetika, die wegen ihrer starken analgetischen Wirkung in der klinischen Schmerztherapie häufig eingesetzt werden. Diese aktivieren selektiv den $\mu$-Rezeptor. Unerwünschte Nebenwirkungen einer solchen Schmerztherapie sind jedoch zum Teil erhebliche zentral vermittelte Nebenwirkungen wie Atemdepression, Erbrechen und Bradykardie. Nachteilig sind weiterhin mögliche psychische Abhängigkeiten.

Angesichts der Vielzahl an Schmerzen und mit Schmerzen verbundenen Erkrankungen besteht ein großer Bedarf an wirksamen Schmerzmitteln.

UD 40120 / SAM:AL

US 2002/0042399 A1 offenbart Komponenten mit $\kappa$-Opiat-Agonist-Aktivität, Zusammensetzungen, welche die vorstehenden Komponenten enthalten, und 5 Verfahren in denen die Komponenten als schmerz- und juckreizlindernde Mittel eingesetzt werden.

US 6303611 B 1 offenbart Komponenten mit $\kappa$-Opiat-Agonist-Aktivität, Zusammensetzungen, welche die vorstehenden Komponenten enthalten, und ein Verfahren, in dem die Komponenten als Analgetikum eingesetzt werden.

In Rees et al: Journal of hetereocyclic chemistry, Bd. 24, 1. Januar 1987, Seiten 1297 -1300 wird die Herstellung von neuen *trans*-Bicyclo-perhydro-2(1*H*)-chinoxalinonen beschrieben, welche zuerst eine Ringöffnungsreaktion und anschließenden spontanen Ringschluss von bestimmten Aziridinen in Gegenwart von einer $\alpha$-Aminosäure (Glycin, L-Alanin, L-Prolin und L-Phenylalanin) gewonnen werden.

Der Erfindung lag die Aufgabe zugrunde, ein Mittel zur Verfügung zu stellen, das wenigstens einen der vorgenannten Nachteile des Standes der Technik überwindet. Insbesondere bestand die Aufgabe, neue Verbindungen zur Verfügung zu stellen, die als pharmazeutische Wirkstoffe insbesondere zur Schmerzbekämpfung verwendbar sind.

Diese Aufgabe wird gelöst durch Verbindungen gemäß der allgemeinen Formel (1) wie nachstehend angegeben und/oder deren Racemate, Enantiomere, Diastereomere, Solvate, Hydrate sowie deren pharmazeutisch verträgliche Salze und/oder Ester:

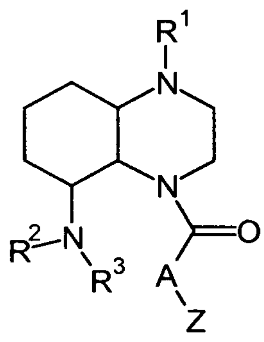

(1)

worin:

$R^1$ ist ausgewählt aus der Gruppe umfassend H; $C_1$-$C_{10}$-Alkyl; $C_3$-$C_{10}$-Cycloalkyl; COO($C_1$-$C_{10}$-Alkyl); $C_1$-$C_6$-Alkoxycabonyl; $C_1$-$C_6$-Oxocarbonyl; Phenylalkyl mit $C_1$-$C_6$-Alkyl wobei der Phenylrest mit einer oder mehreren gleichen oder verschiedenen Gruppen ausgewählt aus der Gruppe umfassend Halogen, $C_1$-$C_6$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N($C_1$-$C_5$-Alkyl)$_2$, $SO_2NH_2$, $SO_2$NH($C_1$-$C_5$-Alkyl), $SO_2$NH(Aryl) $SO_2$NH(Phenyl) und/oder $SO_2$NH (Heteroaryl) substituiert sein kann; $C_1$-$C_{10}$-Acyl; $C_3$-$C_{10}$-Cycloacyl; Phenylacyl, wobei der Acylrest ein $C_1$-$C_6$-Acylrest ist und der Phenylrest mit einer oder mehreren gleichen oder verschiedenen Gruppen ausgewählt aus der Gruppe umfassend Halogen, $C_1$-$C_6$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N($C_1$-$C_5$-Alkyl)$_2$, $SO_2NH_2$, $SO_2$NH($C_1$-$C_5$-Al-

kyl), $SO_2NH$(Aryl), $SO_2NH$(Phenyl) und/oder $SO_2NH$(Heteroaryl) substituiert sein kann; mono-, bi- oder tricyclisches Heteroaryl umfassend ein, zwei, drei oder vier Heteroatome ausgewählt aus der Gruppe umfassend N, O und/oder S; mono-, bi- oder tricyclisches Heteroarylalkyl umfassend ein, zwei, drei oder vier Heteroatome ausgewählt aus der Gruppe umfassend N, O und/oder S, wobei der Alkylrest ein $C_1$-$C_6$-Alkylrest ist; mono-, bi- oder tricyclisches Heteroarylacyl umfassend ein, zwei, drei oder vier Heteroatome ausgewählt aus der Gruppe umfassend N, O und/oder S, wobei der Acylrest ein $C_1$-$C_6$-Acylrest ist; C(O)($C_1$-$C_{10}$-Alkyl); C(O)N($C_1$-$C_{10}$-Alkyl)$_2$; C(O)($C_3$-$C_{10}$-Cycloalkyl); COO($C_1$-$C_{10}$-Alkyl); COO(Aryl); COO($C_3$-$C_{10}$-Cycloalkyl); C(O)COO($C_1$-$C_{10}$-Alkyl); C(O)-$(CH_2)_q$-COOH wobei q ist 0, 1, 2, 3 oder 4; C(O)-$(CH_2)_r$-COO($C_1$-$C_{10}$-Alkyl) wobei r ist 0, 1, 2, 3 oder 4; C(O)-CH($NH_2$)-$(CH_2)_s$-COOH wobei s ist 0, 1, 2, 3 oder 4; C(O)-CH($NH_2$)-$(CH_2)_t$-COO($C_1$-$C_{10}$-Alkyl) wobei t ist 0, 1, 2, 3 oder 4; C(O)-$(CH_2)_u$-CH($NH_2$)-COOH wobei u ist 0, 1, 2, 3 oder 4 und/oder C(O)-$(CH_2)_v$-CH($NH_2$)-COO($C_1$-$C_{10}$-Alkyl) wobei v ist 0, 1, 2, 3 oder 4;

$R^2$, $R^3$ sind jeweils gleich oder unabhängig voneinander ausgewählt aus der Gruppe umfassend H; $C_1$-$C_{10}$-Alkyl; $C_3$-$C_{10}$-Cycloalkyl; Phenylalkyl mit $C_1$-$C_6$-Alkyl und wobei der Phenylrest mit einer oder mehreren gleichen oder verschiedenen Gruppen ausgewählt aus der Gruppe umfassend Halogen, $C_1$-$C_5$-Alkyl, $C_1$-$C_4$-Alkyloxy, $NH_2$, NH($C_1$-$C_5$-Alkyl), N($C_1$-$C_5$-Alkyl)$_2$, OH, COOH, COO($C_1$-$C_{10}$-Alkyl), $CONH_2$, CONH($C_1$-$C_{10}$-Alkyl), CON($C_1$-$C_{10}$-Alkyl)$_2$, $SO_2$($C_1$-$C_5$-Alkyl), $SO_2HN$($C_1$-$C_5$-Alkyl), $CF_3$, CN und/oder $NO_2$ substituiert sein kann, oder

$R^2$ und $R^3$ bilden gememsam mit dem Stickstoff, an den sie gebunden sind, einen gesättigten 3- bis 8-gliedrigen N-Heterocvclus aus, wobei dieser substituiert sein kann mit einer oder mehreren gleichen oder verschiedenen Gruppen ausgewählt aus der Gruppe umfassen OH, $C_1$-$C_4$-Alkyloxy, Carbonyl-Sauersioff, $NH_2$, KM($C_1$-$C_5$-Alkyl), N($C_1$-$C_5$-Alkyll)$_2$, COOH, COO($C_1$-$C_{10}$-Alkyl), $CONH_2$, CONH($C_1$-$C_{10}$-Alkyl), CON($C_1$-$C_{10}$-Alkyl)$_2$, $OPO_3H_2$, $OSO_3H$, $SO_2$($C_1$-$C_5$-Alkyl), $SO_2HN$($C_1$-$C_3$-Alkyl), CN, O-Arylacetyl, O-Phenylacetyl, Arylacetoxyund/oder Acetylbenzyl, das mit zwei Cl-Gruppen substituiert sein kann;

A ist ausgewählt aus der Gruppe umfassend $(CH_2)_n$ wobei n ist ;

Z ist ausgewählt aus der Gruppe umfassend Phenyl, das mit einer oder mehreren gleichen oder verschiedenen Gruppen ausgewählt aus der Gruppe umfassend Halogen, $C_1$-$C_5$-Alkyl, $C_1$-$C_5$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2N$($C_1$-$C_5$-Alkyl)$_2$, $SO_2NH_2$, $SO_2NH$($C_1$-$C_5$-Alkyl), $SO_2NH$(Aryl), $SO_2NH$(Phenyl) und/oder $SO_2NH$(Heteroaryl) substituiert sein kann; ein mono-oder bicyclisches Aryl oder Heteroaryl umfassend ein oder zwei Heteroatome ausgewählt aus der Gruppe umfassend N, O und/oder S, wobei die Aryl- oder Heteroarylgruppe substituiert sein kann mit einer oder mehreren gleichen oder verschiedenen Gruppen ausgewählt aus der Gruppe umfassend Halogen, $C_1$-$C_4$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2N$($C_1$-$C_5$-Alkyl)$_2$, $SO_2NH_2$, $SO_2NH$($C_1$-$C_5$-Alkyl), $SO_2NH$(Aryl), $SO_2NH$(Phenyl) und/oder $SO_2NH$(Heteroaryl).

Es wurde überraschend gefunden, dass die erfindungsgemäßen Verbindungen eine analgetische Wirkung aufweisen können. Ein besonderer Vorteil der erfindungsgemäßen Verbindungen ergibt sich hierbei dadurch, dass die Verbindungen überwiegend in der Peripherie analgetisch wirken können.

Ohne auf eine bestimmte Theorie festgelegt zu sein, wird angenommen, dass die Perhydrochinoxalin-Ringstruktur der erfindungsgemäßen Verbindungen erheblichen Einfluss auf die vorteilhaften Eigenschaften der Verbindungen aufweist.

Im Sinne der vorliegenden Erfindung sind, wenn nicht abweichend angegeben, unter dem Begriff "Heteroaryl" mono-, bi- oder tricyclische Heteroarylgruppen umfassend ein, zwei, drei oder vier Heteroatome ausgewählt aus der Gruppe umfassend N, O und/oder S zu verstehen.

Bevorzugte Heteroarylreste sind ausgewählt aus der Gruppe umfassend Pyridinyl, Pyrimidinyl, Pyrazinyl, Triazolyl, Pyridazinyl, 1,3,5-Triazinyl, Chinolyl, Isochinolyl, Chinolinyl, Isochinolinyl, Chinoxalinyl, Imidazolyl, Pyrazolyl, Benzimidazolyl, Benzooxazolyl, Benzothiazolyl, Thiazolyl, Oxazolyl, Isoxazolyl, Oxazolidinyl, Pyrrolyl, Carbazolyl, Indolyl, Isoindolyl, Furyl, Benzofuryl, Benzofuranyl, 1,3-Benzodioxolyl, Thienyl und/oder Benzothienyl.

Besonders bevorzugte Heteroarylreste sind ausgewählt aus der Gruppe umfassend Pyridinyl, Thiazolyl, Oxazolyl, Isoxazolyl, Imidazolyl, Indolyl, Chinolyl, Isochinolyl, Benzofuranyl, 1,3-Benzodioxolyl, Benzothienyl, Benzimidazolyl, Benzooxazolyl, Benzothiazolyl, Furyl und/oder Thienyl.

Bevorzugte Heteroarylreste sind einkernige Heteroarylreste. Besonders bevorzugte Heteroarylreste sind einkernige Heteroarylreste mit 4, 5 oder 6 Kohlenstoffatomen.

Weiter bevorzugte Heteroarylreste sind einkernige Heteroarylreste, insbesondere ausgewählt aus der Gruppe umfassend 2-Pyridyl, 3-Pyridyl, 4-Pyridyl, Furyl, Thienyl, Imidazolyl, Pyrimidinyl und/oder Oxazolyl.

Im Rahmen dieser Erfindung wird für den Substituenten Pyridin die Bezeichnung "Pyridinyl" wie auch die gebräuchlichere Kurzform "Pyridyl" synonym verwendet

In bevorzugten Ausführungsformen des Strukturelementes $R^1$ ist die Heteroarylalkyl-Gruppe $-(CH_2)_{m-}$Heteroaryl wobei m 0, 1, 2, 3 oder 4 ist.

In weiter bevorzugten Ausführungsformen des Strukturelementes $R^1$ ist die Heteroarylacyl-Gruppe $-CO-(CH_2)_p$-Heteroaryl wobei p 0, 1, 2, 3 oder 4 ist.

In weiterhin bevorzugten Ausführungsformen des Strukturelementes $R^1$ ist $C(O)-(CH_2)_q-COOH$ wobei q ist 0, 1, 2, 3 oder 4 ausgewählt aus der Gruppe umfassend $C(O)COOH$, $C(O)-CH_2-COOH$ und/oder $C(O)-(CH_2)_2-COOH$.

In weiter bevorzugten Ausführungsformen des Strukturelementes $R^1$ ist $C(O)-(CH_2)_r-COO(C_1-C_{10}$-Alkyl) wobei r ist 0, 1, 2, 3 oder 4 ausgewählt aus der Gruppe umfassend $C(O)-CH_2-COO-CH_3$, $C(O)-CH_2-COO-C_2H_5$, $C(O)-(CH_2)_2-COO-CH_3$ und/oder $C(O)-(CH_2)_2-COO-C_2H_5$.

In weiter bevorzugten Ausführungsformen des Strukturelementes $R^1$ ist $C(O)-CH(NH_2)-(CH_2)_s-COOH$ wobei s ist 0, 1, 2, 3 oder 4 $C(O)-CH(NH_2)-CH_2-COOH$.

In weiter bevorzugten Ausführungsformen des Strukturelementes $R^1$ ist $C(O)-CH(NH_2)-(CH_2)_t-COO(C_1-C_{10}$-Alkyl) wobei t ist 0, 1, 2, 3 oder 4 ausgewählt aus der Gruppe umfassend $C(O)-CH(NH_2)-CH_2-COO-CH_3$ und/oder $C(O)-(CH_2)_2-COO-C_2H_5$.

In auch weiter bevorzugten Ausführungsformen des Strukturelementes $R^1$ ist $C(O)-(CH_2)_u-CH(NH_2)-COOH$ wobei u ist 0, 1, 2, 3 oder 4 $C(O)-CH_2-CH(NH_2)-COOH$.

In noch weiter bevorzugten Ausführungsformen des Strukturelementes $R^1$ ist $C(O)-(CH_2)_v-CH(NH_2)-COO(C_1-C_{10}$-Alkyl) wobei v ist 0, 1, 2, 3 oder 4 ausgewählt aus der Gruppe umfassend $C(O)-CH_2-CH(NH_2)-COO-CH_3$ und/oder $C(O)-(CH_2)_2-COO-C_2H_5$.

Der Begriff "$C_1-C_{10}$-Alkyl" umfasst, wenn nicht anders angegeben, geradkettige, verzweigte oder cyclische Alkylgruppen, vorzugsweise ausgewählt aus der Gruppe umfassend Methyl, Ethyl, Propyl, Butyl, Pentyl, Neopentyl, Undecyl, Dodecyl, Pentadecyl, Hexadecyl, Heptadecyl, Octadecyl und/oder Cyclohexyl. Der Begriff "$C_1-C_{10}$-Alkyl" umfasst bevorzugt geradkettige, verzweigte oder cyclische Alkylgruppen, vorzugsweise ausgewählt aus der Gruppe umfassend Methyl, Ethyl, Propyl, Butyl, Pentyl, Neopentyl, Hexyl, Heptyl, Octyl, Nonyl und/oder Decyl.

Bevorzugt sind $C_1-C_5$-Alkylgruppen. $C_1-C_5$-Alkylgruppen sind vorzugsweise ausgewählt aus der Gruppe umfassend Methyl, Ethyl, n-Propyl, Isopropyl, n-Butyl, tert.-Butyl und/oder n-Pentyl. Besonders bevorzugt sind $C_1-C_5$-Alkylgruppen ausgewählt aus der Gruppe umfassend Methyl, Ethyl, n-Propyl und/oder Isopropyl.

Betreffend Monoalkyl- und Dialkylaminosubstituenten $NH(C_1-C_5$-Alkyl), und/oder $N(C_1-C_5$-Alkyl$)_2$ sind $C_1-C_5$-Alkylgruppen vorzugsweise ausgewählt aus der Gruppe umfassend Methyl und/oder Ethyl.

$C_1-C_6$-Alkyloxygruppen sind vorzugsweise ausgewählt aus der Gruppe umfassend Methoxy, Ethoxy, lineares oder verzweigtes Propoxy und/oder Butoxy.

Der Begriff "Halogen" umfasst Fluor, Chlor, Brom und Jod, wobei Fluor oder Chlor insbesondere Chlor bevorzugt sind.

Unter dem Begriff "Aryl" sind bevorzugt aromatische Reste mit 6 bis 20 C-Atomen zu verstehen, vorzugsweise Phenyl, Naphthyl, Indenyl, Biphenyl sowie 5-oder 6-gliedrige heterocyclische Ringe, die 1 bis 3 Heteroatome ausgewählt aus O, N oder S enthalten und gegebenenfalls mit einem Benzolring anelliert sind wie Indolyl. Bevorzugt sind Phenyl und Indolyl, insbesondere Phenyl. Der Begriff "Aryl" umfasst bevorzugt Carbocyclen. Weiter bevorzugte Arylgruppen sind ausgewählt aus der Gruppe umfassend Phenyl, Naphthyl und/oder Indenyl.

Der Begriff "Phenylalkyl" umfasst im Sinne der vorliegenden Erfindung die Gruppe -AlkylPhenyl, wobei Phenylalkyl beispielsweise Phenylethyl und Benzyl umfasst.

Ein Vorteil der erfindungsgemäßen Verbindungen ist, dass diese eine hohe Affinität für den $\kappa$-Rezeptor aufweisen können. Von besonderem Vorteil ist weiterhin, dass die erfindungsgemäßen Verbindungen in bevorzugten Ausführungsformen eine hohe Selektivität der Bindung an den $\kappa$-Rezeptor gegenüber der Bindung an $\mu$-, $\delta$-, $\sigma_1$- und $\alpha_2$-Rezeptoren sowie gegenüber der Phencyclidin-(PCP) Bindungsstelle des NMDA-Rezeptors (NMDA: N-Methyl-D-aspartat) aufweisen.

Ein Vorteil einer hohen Selektivität der Bindung an den $\kappa$-Rezeptor kann dadurch zur Verfügung gestellt werden, dass keine oder nur geringfügig zentral vermittelte Nebenwirkungen auftreten. Ein besonderer Vorteil einer hohe Selektivität der Bindung an den $\kappa$-Rezeptor kann dadurch zur Verfügung gestellt werden, dass die Gefahr einer psychischen Abhängigkeiten reduziert werden kann.

In bevorzugten Ausführungsformen der Strukturelemente $R^2$ und $R^3$ bilden diese gemeinsam mit dem Stickstoff, an den sie gebunden sind, einen gesättigten 3- bis 8-gliedrigen N-Heterocyclus aus. Der gesättigten 3- bis 8-gliedrigen N-Heterocyclus ist vorzugsweise ausgewählt aus der Gruppe umfassend Pyrrolidinyl, Piperazinyl, Piperidinyl, Morpholinyl und/oder Azepanyl. Bevorzugte gesättigte N-Heterocyclen sind 5- oder 6-gliedrige heterocyclische Ringe ausgewählt aus der Gruppe umfassend Pyrrolidinyl, Piperazinyl, Piperidinyl und/oder Morpholinyl.

Die Strukturelemente $R^2$ und $R^3$ bilden in bevorzugten Ausfuhrunssformen gemeinsam mit dem Stickstoff, an den sie gebunden sind, einen Pyrrolidinvlrest aus, wobei der Pyrrolidinylrest mit einer oder mehreren gleichen oder verschiedenen Gruppen ausgewählt aus der Gruppe umfassend $C_1$-$C_5$-Alkyl, $C_1$-$C_5$-Alkyloxy und/oder OH substituiert sein kann. Bevorzugt ist der Pyrrolidinylrest mit einer oder zwei OH-Gruppen substituiert. Besonders bevorzugt bilden die Strukturelemente $R^2$ und $R^3$ gemeinsam mit dem Stickstoff, an den sie gebunden sind, einen Pyrrolidin- oder einen 3-Hydroxy-pyrrolidinring aus.

Das Strukturelement A ist eine Gruppe $(CH_2)_n$, wobei n 1 ist.

Das Strukturelement Z ist bevorzugt ein Phenylrest, der mit einer oder mehreren gleichen oder verschiedenen Gruppen ausgewählt aus der Gruppe umfassend $C_1$-$C_5$-Alkyl, $C_1$-$C_5$-Alkyloxy, OH, Halogen, bevorzugt ausgewählt aus F, Cl, Br und/oder I, $CF_3$, CN, $SO_2$($C_1$-$C_5$-Alkyl), $NO_2$, $NH_2$, NH($C_1$-$C_5$-Alkyl), und/oder N($C_1$-$C_5$-Alkyl)$_2$ substituiert sein kann. Vorzugsweise ist der Phenylrest mit einem oder zwei Halogenatomen, bevorzugt ausgewählt aus F, Cl, Br und/oder I, vorzugsweise Cl substituiert.

Eine Substitution des Phenylrestes mit einem, vorzugsweise zwei Chloratomen kann eine erhebliche Steigerung der Wirksamkeit der Verbindung zur Folge haben.

In bevorzugten Ausführungsformen bildet das Strukturelement C(O)AZ eine Phenylacetyl - oder eine Dichlorphenylacetylgruppe aus.

Bevorzugte Verbindungen und/oder deren Racemate, Enantiomere, Diastereomere, Solvate, Hydrate sowie deren pharmazeutisch verträgliche Salze und/oder Ester weisen die nachstehende allgemeine Formel (2) auf:

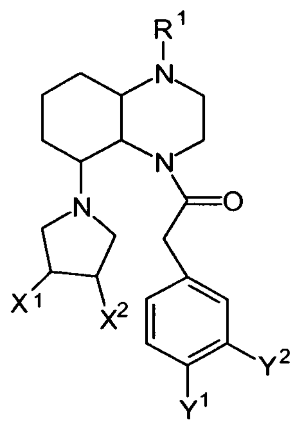

(2)

worin:

$R^1$ ist ausgewählt aus der Gruppe umfassend H; $C_1$-$C_{10}$-Alkyl; $C_3$-$C_{10}$-Cycloalkyl; COO($C_1$-$C_{10}$-Alkyl); $C_1$-$C_6$-Alkoxycabonyl; $C_1$-$C_6$-Oxocarbonyl; Phenylalkyl mit $C_1$-$C_6$-Alkyl wobei der Phenylrest mit einer oder mehreren gleichen oder verschiedenen Gruppen ausgewählt aus der Gruppe umfassend Halogen, $C_1$-$C_6$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N($C_1$-$C_5$-Alkyl)$_2$, $SO_2NH_2$, $SO_2$NH($C_1$-$C_5$-Alkyl), $SO_2$NH(Aryl), $SO_2$NH(Phenyl) und/oder $SO_2$NH(Heteroaryl) substituiert sein kann; $C_1$-$C_{10}$-Acyl; $C_3$-$C_{10}$-Cycloacyl; Phenylacyl, wobei der Acylrest ein $C_1$-$C_6$-Acylrest ist und der Phenylrest mit einer oder mehreren gleichen oder verschiedenen Gruppen ausgewählt aus der Gruppe umfassend Halogen, $C_1$-$C_6$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N($C_1$-$C_5$-Alkyl)$_2$, $SO_2NH_2$, $SO_2$NH($C_1$-$C_5$-Alkyl), $SO_2$NH(Aryl), $SO_2$NH(Phenyl) und/oder $SO_2$NH(Heteroaryl) substituiert sein kann; mono-, bi- oder tricyclisches Heteroaryl umfassend ein, zwei, drei oder vier Heteroatome ausgewählt aus der Gruppe umfassend N, O und/oder S; mono-, bi- oder tricyclisches Heteroarylalkyl umfassend ein, zwei, drei oder vier Heteroatome ausgewählt aus der Gruppe umfassend N, O und/oder S, und der Alkylrest ein $C_1$-$C_6$-Alkylrest ist; mono-, bi- oder tricyclisches Heteroarylacyl umfassend ein, zwei, drei oder vier Heteroatome ausgewählt aus der Gruppe umfassend N, O und/oder S, und der Acylrest ein $C_1$-$C_6$-Acylrest ist; C(O)($C_1$-$C_{10}$-Alkyl); C(O)N($C_1$-$C_{10}$-Alkyl)$_2$; C(O)($C_3$-$C_{10}$-Cycloalkyl); COO($C_1$-$C_{10}$-Alkyl); COO(Aryl); COO($C_3$-$C_{10}$-Cycloalkyl); C(O)COO($C_1$-$C_{10}$-Alkyl), C(O)-$(CH_2)_q$-COOH wobei q ist 0, 1, 2, 3 oder 4, C(O)-$(CH_2)_r$-COO($C_1$-$C_{10}$-Alkyl) wobei r ist 0, 1, 2, 3 oder 4, C(O)-CH($NH_2$)-$(CH_2)_s$-COOH wobei s ist 0, 1, 2, 3 oder 4, C(O)-CH($NH_2$)-$(CH_2)_t$-COO($C_1$-$C_{10}$-Alkyl) wobei t ist 0, 1, 2, 3 oder 4, C(O)-$(CH_2)_u$-CH($NH_2$)-COOH wobei u ist 0, 1, 2, 3 oder 4, und/oder C(O)-$(CH_2)_v$-CH($NH_2$)-COO($C_1$-$C_{10}$-Alkyl) wobei v ist 0, 1, 2, 3 oder 4;

$X^1$, $X^2$ sind jeweils gleich oder unabhängig voneinander ausgewählt aus der Gruppe umfassend H, OH, Carbonyl-

Sauerstoff, $NH_2$, $NH(C_1\text{-}C_5\text{-Alkyl})$, $N(C_1\text{-}C_5\text{-Alkyl})_2$, COOH, $COO(C_1\text{-}C_{10}\text{-Alkyl})$, $CONH_2$, $CONH(C_1\text{-}C_{10}\text{-Alkyl})$, $CON(C_1\text{-}C_{10}\text{-Alkyl})_2$, $OPO_3H_2$, $OSO_3H$, $SO_2(C_1\text{-}C_5\text{-Alkyl})$, $SO_2HN(C_1\text{-}C_5\text{-Alkyl})$, $C_1\text{-}C_4$-Alkyloxy, O-Arylacetyl, O-Phenylacetyl, Arylacetoxy und/oder Acetylbenzyl, das mit zwei Cl-Gruppen substituiert sein kann;

$Y^1$, $Y^2$ sind jeweils gleich oder unabhängig voneinander ausgewählt aus der Gruppe umfassend H, Halogen, $C_1\text{-}C_5$-Alkyl, $C_1\text{-}C_5$-Alkyloxy, $NH_2$, $NH(C_1\text{-}C_5\text{-Alkyl})$, NH(Aryl), NH(Phenyl), NH(Heteroaryl), $N(C_1\text{-}C_5\text{-Alkyl})_2$, OH, $SO_2(C_1\text{-}C_5\text{-Alkyl})$, $SO(C_1\text{-}C_5\text{-Alkyl})$, $CF_3$, CN, $NO_2$, $SO_2N(C_1\text{-}C_5\text{-Alkyl})_2$, $SO_2NH_2$, $SO_2NH(C_1\text{-}C_5\text{-Alkyl})$.

Das Strukturelement $R^1$ ist bevorzugt ausgewählt aus der Gruppe umfassend H, $C_1\text{-}C_5$-Alkyl, Phenylalkyl mit $C_1\text{-}C_3$-Alkyl und wobei der Phenylrest mit einer oder mehreren gleichen oder verschiedenen Gruppen ausgewählt aus der Gruppe umfassend Cl, OH und/oder $C_1\text{-}C_4$-Alkyloxy substituiert sein kann und/oder N-Heteroarylalkyl, wobei der N-Heteroarylrest ausgewählt ist aus Pyridinyl, Pyrimidinyl, Pyrazinyl und/oder Pyrrolyl, und der Alkylrest ein $C_1\text{-}C_3$-Alkylrest ist.

Das Strukturelement $R^1$ ist weiter bevorzugt ausgewählt aus der Gruppe umfassend $C_1\text{-}C_3$-Acyl, Benzoyl, $COO(C_1\text{-}C_3\text{-Alkyl})$, C(O)COOH, $C(O)\text{-}CH_2\text{-}COOH$, $C(O)\text{-}CH_2\text{-}COO\text{-}CH_3$ und/oder $C(O)\text{-}CH_2\text{-}COO\text{-}C_2H_5$.

Die Strukturelemente $X^1$ und $X^2$ sind bevorzugt ausgewählt aus der Gruppe umfassend H, OH und/oder O-Acetylphenyl, das mit zwei Cl-Gruppen substituiert ist.

Vorzugsweise ist wenigstens ein Strukturelement $X^1$ oder $X^2$ H. Weiter bevorzugt ist ein Strukturelement $X^1$ oder $X^2$ OH. In bevorzugten Ausführungsformen der Verbindung der Formel (2) ist das Strukturelement $X^1$ H und das Strukturelement $X^2$ OH.

Die Strukturelemente $Y^1$ und $Y^2$ sind bevorzugt ausgewählt aus der Gruppe umfassend OH, F und/oder Cl. In bevorzugten Ausführungsformen der Verbindung der Formel (2) sind die Strukturelemente $Y^1$ und $Y^2$ Cl. Eine Substitution mit zwei Gruppen Cl kann eine erhebliche Steigerung der Wirksamkeit der Verbindung zur Folge haben. Ein großer Vorteil, der dadurch zur Verfügung gestellt werden kann, dass die Strukturelemente $Y^1$ und $Y^2$ Chlor sind, ist, dass die Verbindungen eine besonders gute Affinität zum κ-Rezeptor aufweisen können.

Besonders bevorzugte Verbindungen und/oder deren Racemate, Enantiomere, Diastereomere, Solvate, Hydrate sowie deren pharmazeutisch verträgliche Salze und/oder Ester weisen die nachstehende allgemeine Formel (3) auf:

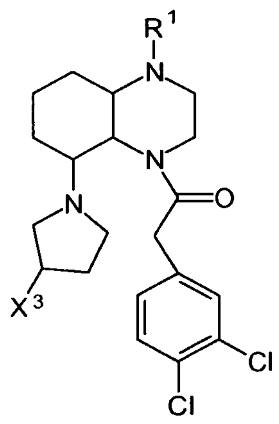

(3)

worin:

$R^1$ ist ausgewählt aus der Gruppe umfassend H; $C_1\text{-}C_5$-Alkyl; Phenylalkyl mit $C_1\text{-}C_4$-Alkyl und wobei der Phenylrest mit einer oder mehreren gleichen oder verschiedenen Gruppen ausgewählt aus der Gruppe umfassend Cl, OH und/oder $C_1\text{-}C_4$-Alkyloxy substituiert sein kann; N-Heteroarylalkyl, wobei der N-Heteroarylrest ausgewählt ist aus Pyridinyl, Imidazolyl, Pyrimidinyl, Pyrazinyl und/oder Pyrrolyl, und der Alkylrest ein $C_1\text{-}C_4$-Alkylrest ist; $C_1\text{-}C_5$-Acyl; Benzoyl; $COO(C_1\text{-}C_5\text{-Alkyl})$; COO(Aryl); $C(O)\text{-}(CH_2)_q\text{-}COOH$ wobei q ist 0, 1, 2, 3 oder 4 und/oder $C(O)\text{-}(CH_2)_r\text{-}COO(C_1\text{-}C_5\text{-Alkyl})$ wobei r ist 0, 1, 2, 3 oder 4;

$X^3$ ist ausgewählt aus der Gruppe umfassend H, OH, Benzyl und/oder O-Arylacetyl, das mit zwei Cl-Gruppen substituiert sein kann.

Das Strukturelement $X^3$ ist besonders bevorzugt ausgewählt aus der Gruppe umfassend H und/oder OH.

Das Strukturelement $R^1$ ist besonders bevorzugt ausgewählt aus der Gruppe umfassend H, Methyl, Butyl, Pentyl, Benzyl, p-Methoxybenzyl, Pyridinylmethyl, insbesondere 2-Pyridinylmethyl, 3-Pyridinylmethyl und/oder Imidazolylmethyl. Das Strukturelement $R^1$ ist ganz besonders bevorzugt H.

Das Strukturelement $R^1$ ist weiter besonders bevorzugt ausgewählt aus der Gruppe umfassend Benzoyl, Acetyl, Propionyl, $COOCH_3$, $COOC_2H_5$, C(O)COOH, C(O)-$CH_2$-COOH, C(O)-$(CH_2)_2$-COOH, C(O)-$CH_2$-COO-$CH_3$, C (O)-$CH_2$-COO-$C_2H_5$, C(O)-$(CH_2)_2$-COO-$CH_3$ und/oder C(O)-$(CH_2)_2$-COO-$C_2H_5$.

In bevorzugten Ausführungsformen ist das Strukturelement $R^1$ ein Acylrest ausgewählt aus der Gruppe umfassend Benzoyl, Acetyl, Propionyl, $COOCH_3$, $COOC_2H_5$, C(O)COOH, C(O)-$CH_2$-COOH und/oder C(O)-$CH_2$-COO-$CH_3$ und das Strukturelement $X^3$ ist ausgewählt aus der Gruppe umfassend H und/oder OH.

Ohne auf eine bestimmte Theorie festgelegt zu sein wird angenommen, dass die Wirkung der erfindungsgemäßen Verbindungen insbesondere auf der sterischen Wirkung der Perhydrochinoxalin-Gruppe, insbesondere in Verbindung mit dem Strukturelement $R^1$, beruht. Insbesondere eine Kombination der Perhydrochinoxalin-Gruppe mit einem Strukturelement $R^1$, das ein Acylrest oder ein Alkylrest ist, kann eine vorteilhafte analgetische Wirkung zur Verfügung stellen.

Von Vorteil bei den Ausführungsformen, in denen das Strukturelement $R^1$ ein Acylrest ausgewählt aus der Gruppe umfassend Benzoyl, Acetyl, Propionyl, $COOCH_3$, $COOC_2H_5$, C(O)COOH, C(O)-$CH_2$-COOH und/oder C (O)-$CH_2$-COO-$CH_3$ und das Strukturelement $X^3$ ausgewählt ist aus der Gruppe umfassend H und/oder OH ist, dass diese eine gute Affinität zum κ-Rezeptor aufweisen können. Beispielsweise kann der $K_i$-Wert als Maß für die Affinität zum κ-Rezeptor im Bereich von ≥ 1 nM bis ≤ 800 nM, vorzugsweise im Bereich von ≥ 5 nM bis ≤ 600 nM, bevorzugt im Bereich von ≥ 9 nM bis ≤ 500 nM, liegen.

Der $K_i$-Wert wurde nach der Methode gemäß Hunter et al., Br. J. Pharmacol. 1990, 1001. 183-189 und Smith et al., J. Neuoch. 1989, 53, 27-36 bestimmt, wobei eine Präparation aus Meerschweinchenganzhirn verwendet wurde und als Radioligand [$^3$H]-U-69,593 (Amersham) verwendet wurde, wie in Beispiel 30 beschrieben.

Ein besonderer Vorteil der Ausführungsformen, in denen das Strukturelement $R^1$ ein Acylrest ausgewählt aus der Gruppe umfassend Benzoyl, Acetyl, Propionyl, $COOCH_3$, $COOC_2H_5$, C(O)COOH, C(O)-$CH_2$-COOH und/oder C (O)-$CH_2$-COO-$CH_3$ und das Strukturelement $X^3$ ausgewählt ist aus der Gruppe umfassend H und/oder OH ist, dass diese eine gute Selektivität der Bindung zum κ-Rezeptor gegenüber der Bindung zum μ-Rezeptor aufweisen können.

In weiter bevorzugten Ausführungsformen ist das Strukturelement $R^1$ ein Phenylalkyl-, Alkyl- , Heteroaryl- oder Heteroarylalkylrest ausgewählt aus der Gruppe umfassend H, Methyl, Butyl, Pentyl, Benzyl, p-Methoxybenzyl, 2-Pyridinylmethyl, 3-Pyridinylmethyl und/oder Imidazolylmethyl und das Strukturelement $X^3$ ist H.

Ein Vorteil dieser Ausführungsformen ist, dass diese eine besonders gute Affinität zum κ-Rezeptor aufweisen können. Beispielsweise kann der $K_i$-Wert als Maß für die Affinität zum κ-Rezeptor im Bereich von ≥ 0,01 nM bis ≤ 50 nM, vorzugsweise im Bereich von 0,5 nM bis ≤ 20 nM, bevorzugt im Bereich von ≥ 1 nM bis ≤ 10 nM, liegen.

In weiter bevorzugten Ausführungsformen ist das Strukturelement $R^1$ H.

Insbesondere bevorzugte Verbindungen und/oder deren Racemate, Enantiomere, Diastereomere, Solvate, Hydrate sowie deren pharmazeutisch verträgliche Salze und/oder Ester weisen die nachstehende allgemeine Formel (4) auf:

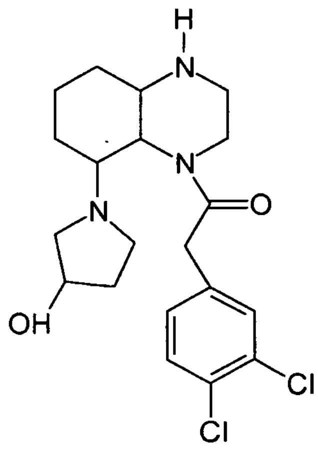

(4).

Verbindungen der Formel (4) können den Vorteil einer besonders guten analgetischen Wirkung, insbesondere peripheren analgetischen Wirkung, zur Verfugung stellen.

Die erfindungsgemäßen Verbindungen der Formel (1) insbesondere die Verbindungen der Formel (4) können in Form der Racemate, Diastereomere oder Enantiomerenpaare vorliegen. Die Racemate, Diastereomere oder Enantiomere eines jeden Paares können nach klassischen Methoden, vorzugsweise mittels Hochleistungsflüssigkeitchromatographie (high performance liquid chromatography, HPLC) getrennt werden.

In bevorzugten Ausführungsformen umfasst die Verbindung (1) ein Gemisch umfassend Enantiomere gemäß

den nachstehenden Formeln (1a) und/oder (1b):

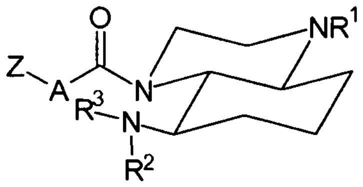
(1a)

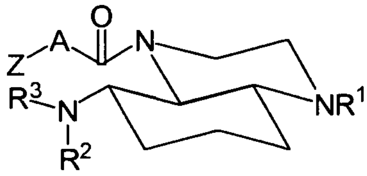
(1b).

Vorzugsweise liegen die Enantiomere (1a) und (1b) der Verbindung (1) als Racemat vor.

Es kann bevorzugt sein, dass die erfindungsgemäßen Verbindungen in Form eines Enantiomers ausgewählt aus den Formeln (1a) und/oder (1b) vorliegen.

In bevorzugten Ausführungsformen kann die Verbindung (4) die nachstehenden Diastereomere gemäß den nachstehenden Formeln (4a) und/oder (4b) aufweisen:

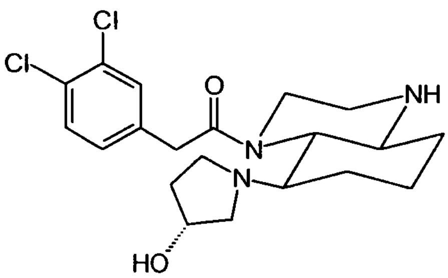
(4a)

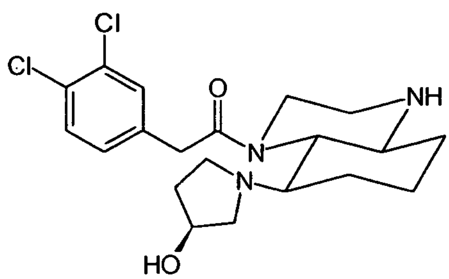
(4b).

Es versteht sich, dass, wenn nicht ausdrücklich abweichend angegeben, wenn im Rahmen der vorliegenden Erfindung die Struktur von lediglich einem Stereoisomer insbesondere

Enantiomer dargestellt ist, jeweils das oder die weiteren Stereoisomere insbesondere Enantiomere umfasst sind.

Weiter bevorzugte Verbindungen und/oder deren Racemate, Enantiomere, Diastereomere, Solvate, Hydrate sowie deren pharmazeutisch verträgliche Salze und/oder Ester weisen die nachstehende Formel (6) auf:

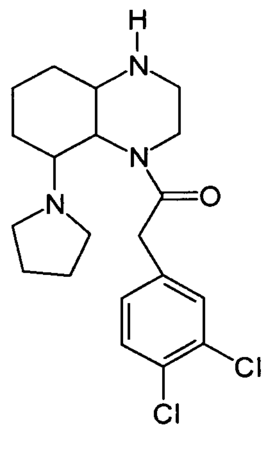
(6).

Es konnte festgestellt werden, dass Verbindungen der Formel (6) eine besonders gute analgetischen Wirkung, insbesondere peripheren analgetischen Wirkung, zur Verfügung stellen können.

Die erfindungsgemäßen Verbindungen sind verwendbar in Form ihrer Racemate, ihrer reinen Stereoisomeren, insbesondere Enantiomeren oder Diastereomeren, oder in Form von Mischungen der Stereoisomeren, insbesondere der Enantiomeren oder Diastereomeren.

Bevorzugte Verbindungen sind ausgewählt aus der Gruppe umfassend 2-(3,4-Dichlorphenyl)-1-[(4aRS,8SR, 8aRS)-8-(pyrrolidin-1-yl)-perhydrochinoxalin-1-yl]-ethan-1-on, und/oder die Diastereomeren 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on sowie deren Diastereomeren-Gemisch.

Weiter bevorzugte Verbindungen sind ausgewählt aus der Gruppe umfassend 1-[(*4aRS*, *8SR*, *8aRS*)-4-Benzoyl-8-(pyrrolidin-1-yl)-perhydrochinoxalin-1-yl]-2-(3,4-dichlorphenyl)ethan-1-on, 1-[(*4aRS,8SR,8aRS*)-4-Acetyl-8-(pyrrolidin-1-yl)-perhydrochinoxalin-1-yl]-2-(3,4-dichlorphenyl)ethan-1-on, *1*-{(*4aRS,8SR,8aRS)*-1-[2-(3,4-Dichlorphenyl) acetyl]-8-(pyrrolidin-1-yl)-perhydrochinoxalin-4-yl}propan-1-on, Methyl-{(*4aRS*,*8SR*,*8aRS*)-1-[2-(3,4-dichlorphenyl)acetyl]-8-(pyrrolidin-1-yl)-perhydrochinoxalin-4-yl}carboxylat, Ethyl-{(*4aRS*,*8SR*,*8aRS*)-1-[2-(3,4-dichlorphenyl)acetyl]-8-(pyrrolidin-1-yl)-perhydrochinoxalin-4-yl}carboxylat, 3-{(*4aRS,8SR,8aRS*)-1-[2-(3,4-Dichlorphenyl)acetyl]-8-(pyrrolidin-1-yl)-perhydrochinoxalin-4-yl}-3-oxopropionsäure, 4-{(*4aRS,8SR,8aRS*)-1-[2-(3,4-Dichlorphenyl)acetyl]-8-(pyrrolidin-1-yl)perhydrochinoxalin-4-yl}-4-oxobuttersäure, Methyl-3-{(*4aRS,8SR,8aRS*)-1-[2-(3,4-Dichlorphenyl)acetyl]-8-(pyrrolidin-1-yl)-perhydrochinoxalin-4-yl}-3-oxopropionat, 1-{(*4aRS,8SR,8aRS*)-4-Benzoyl-8-[(3SR)-und (*3RS*)-3-hydroxypyrrolidin-1-yl]-perhydrochinoxalin-1-yl}-2-(3,4-dichlorphenyl)ethan-1-on, Methyl-{(4aRS,8SR,8aRS)-1-[2-(3,4-dichlorphenyl)acetyl]-8-[(*3SR*)- und (*3RS*)-3-hydroxypyrrolidin-1-yl]-perhydrochinoxalin-4-yl}carboxylat, 3-{(*4aRS,8SR,8aRS)*-1-[*2*-(3,4-Dichlorphenyl)acetyl]-8-[(*3SR*)- und (*3RS*)-3-hydroxypyrrolidin-1-yl]-perhydrochinoxalin-4-yl}-3-oxopropionsäure, Methyl-3-{(*4aRS,8SR,8aRS*)-1-[2-(3,4-Dichlorphenyl)acetyl]-8-[(*3SR*)- und (*3RS*)-3-hydroxypyrrolidin-1-yl]-perhydrochinoxalin-4-yl}-3-oxopropionat, 2-(3,4-Dichlorphenyl)-1-[(*4aRS,8SR,8aRS*)-4-methyl-8-(pyrrolidin-1-yl)-perhydrochinoxalin-1-yl]ethan-1-on, 1-[(*4aRS,8SR,8aRS*)-4-Butyl-8-(pyrrolidin-1-yl)perhydrochinoxalin-1-yl]-2-(3,4-dichlorphenyl)-ethan-1-on, 1-[(*4aRS,8SR,8aRS*)-4-Benzyl-8-(pyrrolidin-1-yl)perhydrochinoxalin-1-yl]-2-(3,4-dichlorphenyl)ethan-1-on, 2-(3,4-Dichlorphenyl)-1-[(*4aRS*,*8SR*,*8aRS*)-4-(4-Methoxybenzyl)-8-(pyrrolidin-1--yl)-perhydrochinoxalin-1-yl] ethan-1-on, 2-(3,4-Dichlorphenyl)-1-{(*4aRS,8SR,8aRS*)-4-[(pyridin-2-yl)methyl]-8-(pyrrolidin-1-yl)perhydrochinoxalin-1-yl}ethan-1-on, 2-(3,4-Dichlorphenyl)-1-{(*4aRS,8SR,8aRS*)-4-[(pyridin-3-yl)methyl]-8-(pyrrolidin-1-yl)perhydrochinoxalin-1-yl}ethan-1-on, 2-(3,4-Dichlorphenyl)-1-{(*4aRS,8SR,8aRS*)-4-[(1*H*-imidazol-5-yl)methyl]-8-(pyrrolidin-1-yl)perhydrochinoxalin-1-yl}ethan-1-on, 2-(3,4-Dichlorphenyl)-1-{(*4aRS,8SR,8aRS*)-4-methyl-8-[(*3SR*)- und (*3RS*)-3-hydroxypyrrolidin-1-yl]-perhydrochinoxalin-1-yl} ethan-1-on, 1-{(*4aRS,8SR,8aSR*)-4-Benzyl-8-[(3SR)- und (*3RS*)-3-hydroxypyrrolidin-1-yl]perhydrochinoxalin-1-yl}-2-(3,4-dichlorphenyl)ethan-1-on und/oder 2-(3,4-Dichlorphenyl)-1-{(*4aRS,8SR, 8aSR*)-4-[(pyridin-3-yl)methyl]-8-[(*3SR*)- und (*3RS*)-3-hydroxypyrrolidin- 1 -yl]perhydrochinoxalin-1-yl}ethan-1-on.

Die erfindungsgemäßen Verbindungen sind weiterhin verwendbar in Form ihrer Säuren oder ihrer Basen oder in Form ihrer Salze oder Ester, insbesondere der physiologisch verträglichen Salze oder Ester, oder in Form ihrer Solvate, insbesondere der Hydrate.

Vorteilhafterweise verwendbar sind insbesondere pharmazeutisch verträgliche Additionssalze der erfindungsgemäßen Verbindungen.

Die pharmazeutisch verträglichen Salze können Basenadditionssalze sein. Dazu zählen Salze der erfindungsgemäßen Verbindungen mit anorganischen Basen, wie Alkalihydroxiden, Erdalkalihydroxiden oder mit organischen Basen, wie Mono-, Di- oder Triethanolamin.

Vorteilhafterweise verwendbar sind weiter Säureadditionssalze, insbesondere mit anorganischen Säuren wie Salzsäure, Schwefelsäure oder Phosphorsäure, oder mit geeigneten organischen Carbon- oder Sulfonsäuren, oder mit Aminosäuren.

Pharmazeutisch verträgliche Salze umfassen in bevorzugten Ausführungsformen nichttoxische Additionssalze der erfindungsgemäßen Verbindungen, beispielsweise in Form der freien Base, mit organischen oder anorganischen Säuren. Beispiele für anorganische Säuren umfassen HCl, HBr, Schwefelsäure und Phosphorsäure. Organische Säuren sind vorzugsweise ausgewählt aus der Gruppe umfassend Essigsäure, Propionsäure, Brenztraubensäure, Buttersäure, alpha-, beta- oder gamma-Hydroxybuttersäure, Valeriansäure, Hydroxyvaleriansäure, Capronsäure, Hydroxycapronsäure, Caprylsäure, Caprinsäure, Laurinsäure, Myristinsäure, Palmitinsäure, Stearinsäure, Glykolsäure, Milchsäure, D-Glucuronsäure, L-Glucuronsäure, D-Galacturonsäure, Glycin, Benzoesäure, Hydroxybenzoesäure, Gallussäure, Salicylsäure, Vanillinsäure, Cumarsäure, Kaffeesäure, Hippursäure, Orotsäure, L-Weinsäure, D-Weinsäure, D,L-Weinsäure, meso-Weinsäure, Fumarsäure, L-Aepfelsäure, D-Aepfelsäure, D,L-Aepfelsäure, Oxalsäure, Malonsäure, Bernsteinsäure, Maleinsäure, Oxalessigsäure, Glutarsäure, Hydroxyglutarsäure, Ketoglutarsäure, Adipinsäure, Ketoadipinsäure, Pimelinsäure, Glutaminsäure, Asparaginsäure, Phthalsäure, Propantricarbonsäure, Zitronensäure, Isozitronensäure, Methansulfonsäure, Toluolsulfonsäure, Benzolsulfonsäure, Camphersulfonsäure, Embonsäure und/oder Trifluormethansulfonsäure.

Pharmazeutisch verträgliche Salze der erfindungsgemäßen Verbindungen sind beispielsweise ausgewählt aus der Gruppe umfassend Chloride, Bromide, Iodide, Hydrochloride, Hydrobromide, Sulfonate, Methansulfonate, Sulfate, Hydrogensulfate, Sulfite, Hydrogensulfite, Phosphate, Nitrate, Methanoate, Acetate, Prioionate, Lactate, Citrate, Glutarate, Maleate, Malonate, Malate, Succinate, Tartrate, Oxalate, Fumarate, Benzoate, p-Toluolsulfonate und/oder Salze der Aminosäuren, vorzugsweise der proteinogenen Aminosäuren.

Verwendbare pharmazeutisch verträgliche Ester der Verbindungen sind insbesondere physiologisch leicht hydrolisierbare Ester, beispielsweise ausgewählt aus der Gruppe umfassend Alkyl-, Pivaloyloxymethyl-, Acetoxymethyl-, Phthalidyl-, Indanyl- und/oder Methoxymethylenester.

Ohne auf eine bestimmte Theorie festgelegt zu sein, wird angenommen, dass die erfindungsgemäßen Verbindungen geeignet sind eine analgetische, antipyretische, antiphlogistische, Juckreiz lindernde und/oder krampflösende Wirkung aufzuweisen.

Ein Vorteil der Verbindungen liegt in bevorzugten Ausführungsformen darin, dass diese Verbindungen lediglich in geringem Maße die Blut-Him-Schranke passieren. Dies ermöglicht, dass die erfindungsgemäßen Verbindungen insbesondere als peripher wirkende Schmerzmittel verwendbar sind.

Aufgrund ihrer vorteilhaften Eigenschaften sind die erfindungsgemäßen Verbindungen zur Verwendung als Arzneimittel geeignet.

Die erfindungsgemäßen Verbindungen sind vorzugsweise toxikologisch unbedenklich und eignen sich daher als pharmazeutische Wirkstoffe und/oder Arzneimittel.

Die erfindungsgemäßen Verbindungen sind in vorteilhaften Ausführungsformen insbesondere zur therapeutischen und/oder prophylaktischen Behandlung, Diagnose und/oder Therapie von

Erkrankungen ausgewählt aus der Gruppe umfassend Schmerzerkrankungen, entzündliche Erkrankungen und/oder gastrointestinale Erkrankungen verwendbar.

Die erfindungsgemäßen Verbindungen können insbesondere periphere Schmerzen positiv beeinflussen. Insbesondere wurde überraschend gefunden, dass bevorzugte Ausführungsformen der erfindungsgemäßen Verbindungen eine analgetische Wirksamkeit aufweisen.

Beispielsweise konnte experimentell in einem in vivo Modell festgestellt werden, dass die Verbindungen der Formeln (4) und (6) eine analgetische Wirksamkeit aufwiesen. Hierbei zeigte die Verbindung der Formel (6) eine noch bessere analgetische Wirkung als die Verbindung der Formel (4).

Ein weiterer Gegenstand der Erfindung betrifft die Verwendung der erfindungsgemäßen Verbindungen, insbesondere der Verbindungen der Formeln (4) und (6), zur Herstellung eines Arzneimittels zur therapeutischen und/oder prophylaktischen Behandlung von Erkrankungen ausgewählt aus der Gruppe umfassend Schmerzerkrankungen, entzündliche Erkrankungen und/oder gastrointestinale Erkrankungen.

Die erfindungsgemäßen Verbindungen sind allein oder in Kombination mit bekannten Substanzen zur Behandlung von Erkrankungen ausgewählt aus der Gruppe umfassend Schmerzerkrankungen, entzündliche Erkrankungen und/oder gastrointestinale Erkrankungen verwendbar.

Schmerzerkrankungen umfassen akute und chronische Schmerzen.

Schmerzerkrankungen können insbesondere ausgewählt sein aus der Gruppe umfassend Rückenschmerzen, Gesichtsschmerzen, Kopfschmerzen, Gelenkschmerzen, muskuläre

Schmerzsyndrome, entzündliche Schmerzerkrankungen, neuropathische Schmerzen, periphere Schmerzen, periphere Nervenschädigungen, viscerale Schmerzen, Bauchschmerzen, Menstruationsbeschwerden, Nieren- und Gallensteinschmerzen, Juckreiz, Krebs- und Tumorschmerzen, sympatische Schmerzen, postoperative Schmerzen, posttraumatische Schmerzen, Hyperalgesie und/oder inflammatorische Schmerzen.

Gesichtsschmerzen sind vorzugsweise ausgewählt aus der Gruppe umfassend Trigeminusneuralgie, Zahnschmerzen, Ohrenschmerzen, kraniomandibuläre Dysfunktion und/oder chronisch-idiopatischer Gesichtsschmerz.

Kopfschmerzen sind vorzugsweise ausgewählt aus Schmerzen der Kopforgane wie Schädeldecke, Hirnhäute, Blutgefäße im Gehirn, Hirnnerven, und oberste Spinalnerven. Bevorzugte Kopfschmerzformen sind ausgewählt aus der Gruppe Migränekopfschmerz, Spannungskopfschmerz, Cluster-Kopfschmerz (Horton-Syndrom) und substanzinduzierter Kopfschmerzen beispielsweise durch Einnahme von Medikamenten.

Rückenschmerzen sind vorzugsweise ausgewählt aus der Gruppe umfassend Wirbelsäulensyndrom der Hals-, Brust oder Lendenwirbelsäule, Steißbeinschmerzen und/oder Ischiasschmerzen.

Entzündliche Schmerzerkrankungen sind vorzugsweise ausgewählt aus der Gruppe umfassend Polyarthritis und/oder rheumatoide Arthritis.

Periphere Nervenschädigungen sind vorzugsweise ausgewählt aus der Gruppe umfassend Stumpf- und Phantomschmerzen, neuropathische Schmerzen, Polyneuropathie, postzosterische Neuralgie und/oder Zwischenrippenneuralgie

Bauchschmerzen umfassen vorzugsweise Reizdarmsyndrom (RDS).

Menstruationsbeschwerden umfassen Schmerzen und Krämpfe.

Unter einer Hyperalgesie wird das gesteigerte Empfinden eines Schmerzreizes verstanden.

Insbesondere bei der Behandlung von chronischen peripheren Schmerzen des Menschen kann durch Verwendung der erfindungsgemäßen Verbindungen ein vorteilhafter Effekt auf den Krankheitsverlauf erzielt werden. Ein weiterer Vorteil der erfindungsgemäßen Verbindungen kann sich daraus ergeben, dass keine oder nur geringfügig zentral vermittelte Nebenwirkungen wie Atemdepression, Erbrechen, Bradykardie oder Obstipation auftreten können.

Insbesondere sind die erfindungsgemäßen Verbindungen in bevorzugten Ausführungsformen als periphere Analgetika geeignet.

Von besonderem Vorteil ist, dass die erfindungsgemäßen Verbindungen vorzugsweise keine euphorische Wirkung zeigen. Dies kann den Vorteil zur Verfügung stellen, dass eine Verabreichung der erfindungsgemäßen Verbindungen zu geringerer oder keiner psychischer Abhängigkeit führen. Dies ermöglicht, dass die erfindungsgemäßen Verbindungen über einen längeren Zeitraum verabreicht werden können. Beispielsweise wird eine langfristige Verabreichung, insbesondere eine tägliche Verabreichung ermöglicht. Dies ermöglicht beispielsweise eine Verabreichung zur Behandlung von Schmerzerkrankungen, deren Therapie unter Umständen über Monate oder Jahre fortgesetzt werden muss.

Vorzugsweise sind die erfindungsgemäßen Verbindungen zur Behandlung chronischer Schmerzen geeignet.

Versuche konnten beispielsweise im "Writhing Test" an Mäusen zeigen, dass die erfindungsgemäßen Verbindungen eine analgetischen Wirksamkeit aufweisen können, wie in Beispiel 28 beschrieben. Die Untersuchungen wurden an der Maus durchgeführt, wie von 1. C. Hendershot, J. Forsaith, J. Pharmacol. Exp. Ther. 125,237-240 (1959) beschrieben.

Weiterhin können die erfindungsgemäßen Verbindungen als Lokalanästhetiküm geeignet sein. Beispielsweise können die erfindungsgemäßen Verbindungen zur Linderung der Schmerzen bei Insektenstich wie Mückenstich oder Verbrennungen geeignet sein. Insbesondere können die erfindungsgemäßen Verbindungen bei schmerzhaften Insektenstichen wie Wespen- oder Bienenstich zur Schmerzlinderung geeignet sein.

Weiterhin sind die erfindungsgemäßen Verbindungen zur Behandlung von Schmerzreizen wie Juckreiz verwendbar.

Die erfindungsgemäßen Verbindungen, insbesondere die Verbindungen der Formeln (4) und (6), können insbesondere den Vorteil zur Verfugung stellen für die Behandlung von Juckreiz geeignet sein.

Juckreiz, auch Pruritus genannt, ist ein häufiges Symptom in der Hautheilkunde und stellt auch in anderen medizinischen Fachgebieten ein großes Problem dar. Üblicher Weise wird Juckreiz als eine Art von Schmerzreiz erfahren. Die Juckempfindung löst das Verlangen aus, die betroffene Stelle zu Kratzen. Kratzen verstärkt jedoch den Juckreiz. Durch das Kratzen geschädigte Haut bietet weiter Infektionserregern einen guten Nährboden und Entzündungen aufgekratzter Hautpartien sind nicht selten. So leiden beispielsweise Dialyse-Patienten häufig unter Juckreiz und dessen Folgeschäden. Insbesondere chronischer Juckreiz ist häufig schwer zu therapieren und eine schwere körperliche und seelische Belastung.

Ein besonders bevorzugter Gegenstand der Erfindung betrifft daher die Verwendung der erfindungsgemäßen Verbindungen zur Herstellung eines Arzneimittels zur therapeutischen und/oder prophylaktischen Behandlung von Juckreiz.

Insbesondere kann eine vorbeugende Verabreichung der erfindungsgemäßen Verbindungen von Vorteil sein, wenn ein Juckreiz erwartet wird, beispielsweise nach eine Dialyse.

Die erfindungsgemäßen Verbindungen oder diese enthaltenden Mittel können systemisch oder topisch verabreichbar sein. Vorzugsweise werden die erfindungsgemäßen Verbindungen oder Mittel topisch, insbesondere in Form von Cremes, Salben, Pflastern oder Tinkturen verabreicht.

Entzündlichen Erkrankungen können insbesondere ausgewählt sein aus der Gruppe umfassend entzündliche Erkrankungen des Gastrointestinaltraktes, entzündliche Darmerkrankungen wie Morbus Crohn und/oder Colitis ulcerosa, akute oder chronische entzündliche Veränderungen bei Gallenblasenentzündung, entzündliche Pseudopolypen, Colitis cystica profunda, Pneumatosis cystoides intestinales, Pankreatitis, Appendizitis, entzündlichen Erkrankungen der Gelenke wie rheumatoide Arthritis und/oder entzündliche Erkrankungen der Haut und der Augen.

Eine Verwendung der erfindungsgemäßen Verbindungen ist insbesondere geeignet bei chronisch entzündlichen Darmerkrankungen wie Morbus Crohn oder Colitis ulcerosa.

Ein besonderer Vorteil der erfindungsgemäßen Verbindungen kann dadurch zur Verfügung gestellt werden, dass diese insbesondere zur Behandlung und/oder Prophylaxe entzündlicher gastrointestinalen Erkrankungen verwendbar sind.

Gastrointestinalen Erkrankungen können insbesondere ausgewählt sein aus der Gruppe umfassend Reizdarm (irritable bowel Syndrom), gastrische Läsionen, gastrointestinale Ulzerationen, exogene und endogene Schädigungen der Magen-Darmmukosa, Fehlfunktionen des Gastrointestinaltraktes, Adenomen, insbesondere im Darm und/oder juvenile Polypen.

Zu den Fehlfunktionen des Gastrointestinaltraktes werden im Sinne dieser Erfindung auch Passagestörungen und Koliken wie Gallenkoliken gezählt.

Die erfindungsgemäßen Verbindungen können weiterhin besonders geeignet für die Verwendung zur Behandlung entzündlicher gastrointestinalen Erkrankungen sein. Beispielsweise können die erfindungsgemäßen Verbindungen neben der analgetischen und entzündungshemmenden Wirkung geeignet sein, durch die Erkrankung hervorgerufene Störungen der Darmmotorik und/oder Fehlfunktionen des Gastrointestinaltraktes zu normalisieren.

Beispielsweise sind Reizdarmerkrankungen (irritable bowel Syndrom) die häufigste Ursache abdominaler Schmerzsyndrome. Ein Vorteil der erfindungsgemäßen Verbindungen kann dadurch zur Verfügung gestellt werden, dass die erfindungsgemäßen Verbindungen die mit dem Irritable Bowel Syndroms verbundenen Schmerzen lindern und/oder die Erkrankung heilen können. Von besonderem Vorteil ist, dass die erfindungsgemäßen Verbindungen vorzugsweise auf die normale Darmperistaltik keine negativen Auswirkungen aufweisen.

Bevorzugte Indikationen sind ausgewählt aus der Gruppe umfassend Schmerzzustände, Entzündungen, Hyperalgesie, neuropathische Schmerzen, viscerale Schmerzen, periphere Schmerzen, inflammatorische Schmerzen, rheumatoide Arthritis, Menstruationsbeschwerden umfassend Schmerzen und/oder Krämpfe, Nieren- und Gallensteinschmerzen, postoperative Schmerzen, Pruritus, gastrointestinale Beschwerden, wie irritable bowel syndrom, und/oder entzündliche Darmerkrankungen wie Morbus Crohn und Colitis ulcerosa.

Ein weiterer Gegenstand der Erfindung betrifft Arzneimittel umfassend wenigstens eine erfindungsgemäße Verbindung und/oder deren Racemate, Enantiomere, Diastereomere, Solvate, Hydrate sowie deren pharmazeutisch verträgliche Salze und/oder Ester. Bevorzugt sind Arzneimittel umfassend Verbindungen der Formeln (4) oder (6) und/oder deren Racemate, Enantiomere, Diastereomere, Solvate, Hydrate sowie deren pharmazeutisch verträgliche Salze und/oder Ester. Weiterhin können die erfindungsgemäßen Arzneimittel auch Mischungen von zwei oder mehr der erfindungsgemäßen Verbindungen enthalten.

Bevorzugte Arzneimittel sind Schmerzmittel. Besonders bevorzugt sind Arzneimittel zur Behandlung chronischer Schmerzen.

Insbesondere bevorzugte Arzneimittel sind weiterhin Arzneimittel zur Behandlung von Juckreiz, insbesondere chronischem Juckreiz.

Eine bevorzugte Verwendung der Arzneimittel umfassend erfindungsgemäße Verbindungen umfasst die therapeutische und/oder prophylaktische Behandlung von Erkrankungen ausgewählt aus der Gruppe umfassend Schmerzerkrankungen, entzündliche Erkrankungen und/oder gastrointestinale Erkrankungen. Vorzugsweise eignen sich die erfindungsgemäßen Arzneimittel zur Behandlung von Schmerzen. Weiter bevorzugt eignen sich die erfindungsgemäßen Arzneimittel zur Behandlung von Juckreiz.

Unter dem Begriff "prophylaktische Behandlung" wird im Sinne der vorliegenden Erfindung insbesondere verstanden, dass die erfindungsgemäßen Verbindungen prophylaktisch verabreicht werden können, bevor Symptome einer Erkrankung auftreten oder die Gefahr einer Erkrankung besteht. Insbesondere wird unter einer "prophylaktischen Behandlung" eine medikamentöse Vorbeugung verstanden.

Weiter bevorzugte Arzneimittel umfassen wenigstens eine erfindungsgemäße Verbindung und/oder deren Racemate, Enantiomere, Diastereomere, Solvate, Hydrate sowie deren pharmazeutisch verträgliche Salze und/oder Ester und wenigstens einen Opioid-Rezeptor-Antagonisten, vorzugsweise ausgewählt aus der Gruppe umfassend Naloxon, Naltrexon, Cyprodim, Naltrindol, Norbinaltorphimin Nalmefen, Nalorphin, Nalbuphin, Naloxonazin, Methylnaltrexon und/oder Ketylcyclazocin, bevorzugt ausgewählt aus der Gruppe umfassend Naloxon, Naltrexon, Cyprodim, Naltrindol und/oder Norbinaltorphimin. Weiter bevorzugt ist die Verwendung eines Arzneimittels umfassend wenigstens eine erfindungsgemäße Verbindung und/oder deren Racemate, Enantiomere, Diastereomere, Solvate, Hydrate sowie deren pharmazeutisch verträgliche Salze und/oder Ester und wenigstens einen Opioid-Rezeptor-Antagonisten, vorzugsweise ausgewählt aus der Gruppe umfassend Naloxon, Naltrexon, Cyprodim, Naltrindol, Norbinaltorphimin Nalmefen, Nalorphin, Nalbuphin, Naloxonazin, Methylnaltrexon und/oder Ketylcyclazocin. Insbesondere bevorzugt ist die Verwendung eines Arzneimittels umfassend wenigstens eine erfindungsgemäße Verbindung und/oder deren Racemate, Enantiomere, Diastereomere, Solvate, Hydrate sowie deren pharmazeutisch verträgliche Salze und/oder Ester und wenigstens einen Opioid-Rezeptor-Antagonisten, vorzugsweise ausgewählt aus der Gruppe umfassend Naloxon, Naltrexon, Cyprodim, Naltrindol, Norbinaltorphimin Nalmefen, Nalorphin, Nalbuphin, Naloxonazin, Methylnaltrexon und/oder Ketylcyclazocin zur therapeutischen und/oder prophylaktischen Behandlung von Erkrankungen ausgewählt aus der Gruppe umfassend Schmerzerkrankungen, entzündliche Erkrankungen und/oder gastrointestinale Erkrankungen, insbesondere Juckreiz.

Die erfindungsgemäßen Verbindungen sind entsprechend üblichen Methoden verabreichbar, beispielsweise oral, dermal, intranasal, transmucosal, pulmonal, enteral, buccal, rektal, durch Inhalation, mittels Injektion beispielsweise intravenös, parenteral, intraperitoneal, intradermal, subkutan und/oder intramuskulär und/oder örtlich, beispielsweise auf schmerzende Bereiche des Körpers. Besonders bevorzugt ist eine orale Verabreichung.

Die erfindungsgemäßen Verbindungen und/oder deren Racemate, Enantiomere, Diastereomere, Solvate, Hydrate sowie deren pharmazeutisch verträgliche Salze und/oder Ester sind insbesondere zur Herstellung von Arzneimitteln verwendbar, indem man sie zusammen mit mindestens einem Träger- oder Hilfsstoff in eine geeignete Darreichungsform bringt.

Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, vorliegen und/oder verabreicht werden.

Für eine orale Verabreichung eignen sich bevorzugt Zubereitungen in Form von Tabletten, Dragees, Kapseln, Granulaten, Pellets, Tropfen, Säften und Sirupen.

Für eine parenterale, topische oder inhalative Verabreichung eignen sich bevorzugt Lösungen, vorzugsweise ölige oder wäßrige Lösungen, Suspensionen, Emulsionen, Implantate sowie Sprays. Die erfindungsgemäßen Verbindungen können auch als leicht rekonstituierbare Trockenzubereitungen verwendbar sein, beispielsweise lyophilisiert, wobei die erhaltenen Lyophilisate beispielsweise zur Herstellung von Injektionspräparaten verwendbar sind.

Zur perkutanen Verabreichung geeignete Zubereitungen können beispielsweise in einem Depot in gelöster Form oder in einem Pflaster umfasst sein, gegebenenfalls unter Zusatz von die Hautpenetration fördernden Mitteln. Oral oder perkutan anwendbare Zubereitungsformen können die entsprechenden Verbindungen auch verzögert freisetzen.

Bevorzugt sind weiterhin pharmazeutische Darreichungsform mit verzögerter Freisetzung (Retardformulierung) für die orale Applikation der erfindungsgemäßen Verbindungen. Es können magensaftresistente Formulierungen bevorzugt sein. Beispiele für Formulierungen mit verzögerter Freisetzung sind Retardmatrixtabletten, mehrschichtige Tabletten, deren Beschichtung beispielsweise magensaftresistent ausgebildet sein kann, wie Beschichtungen auf Schellackbasis, Retard-Kapseln oder Formulierungen unter Verwendung biologisch abbaubarer Polymere, beispielsweise Poly(milchsäure)-Polymere.

Die erfindungsgemäßen Verbindungen können für eine intravenöse Verabreichung formuliert sein. Bevorzugt sind sterile Suspensionen zur parenteralen Verabreichung, insbesondere zur intravenösen Injektion. Insbesondere für Injektionslösungen sind Hilfsstoffe und/oder Lösemittel vorzugsweise ausgewählt aus der Gruppe umfassend Dimethylsulfoxid (DMSO), Alkohole, bevorzugt mehrwertige Alkohole, vorzugsweise ausgewählt aus der Gruppe umfassend Glycerol und/oder Propylenglykol und/oder pflanzliche Öle geeignet.

Mittel für die topische Anwendung können beispielsweise als pharmazeutisch verträgliche Puder, Lotionen, Salben, Cremes, Gele oder als therapeutische Systeme vorliegen, die therapeutisch wirksame Mengen der erfindungsgemäßen Verbindungen enthalten.

Die erfindungsgemäßen Verbindungen sind als einzelne therapeutische Wirkstoffe oder als Mischungen mit anderen therapeutischen Wirkstoffen verabreichbar. Sie können alleine verabreicht werden, vorzugsweise werden sie in Form von Arzneimitteln verabreicht, insbesondere als Mischungen mit geeigneten pharmazeutischen Trägem.

Zur Herstellung der Arzneimittel sind übliche physiologisch verträgliche pharmazeutische Hilfsstoffe, vorzugsweise ausgewählt aus der Gruppe umfassend Trägermaterialien, Füllstoffe, Lösungsmittel, Verdünnungsmittel, Netzmittel, Emulgatoren, Farbstoffe, Konservierungsmittel, Sprengmittel, Gleitmittel, Salze zur Beeinflussung des osmotischen Druckes, Puffersubstanzen, Aromen und/oder Bindemittel verwendbar.

Als Trägersubstanzen sind organische oder anorganische Stoffe verwendbar, die sich für eine enterale, beispielsweise orale oder rektale, oder parenterale Applikation eignen und mit den Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlenhydrate wie Lactose oder Stärke, Magnesiumstearat, Talkum oder Cellulose.

Die angegebenen Arzneimittel können sterilisiert sein.

Die Verbindungen sind nach üblichen Synthesemethoden herstellbar.

Die erfindungsgemäßen Verbindungen sind besonders bevorzugt herstellbar durch ein Verfahren zur Herstellung der erfindungsgemäßen Verbindungen, umfassend die folgenden Schritte:

a) Cyclisierung von Nitromethan und Glutaraldehyd zu 2-Nitrocyclohexan-1,3-diol;
b) Aminierung des in Schritt a) erhaltenen Nitrodiols mit primären oder sekundären Amine;
c) Reduktion der Nitrogruppe des Nitrodiamins zu einem primären Amin;
d) Umsetzung des in Schritt c) erhaltenen Cyclohexantriamins mit Dialkyloxalat;
e) Abspaltung eines Aminrestes der in Schritt d) erhaltenen Verbindung;
f) Alkylierung der in Schritt e) erhaltenen Verbindung unter Einführen der Gruppen $R^2$ und $R^3$;
g) Reduktion des Perhydrochinoxalindion-Rings der in Schritt f) erhaltenen Verbindung zum Perhydrochinoxalin;
h) Acylierung des in Schritt g) erhaltenen sekundären Amins unter Einführen einer Gruppe C(O)-A-Z;
i) Einführung eines Restes $R^1$, vorzugsweise durch Alkylierung, Acylierung oder hydrogenolytische Einführung von H.

Für die Gruppen A, Z, $R^1$, $R^2$ und $R^3$ wird in vollem Umfang auf die vorstehende Beschreibung Bezug genommen.

Die Cyclisierung von Nitromethan und Glutaraldehyd zu 2-Nitrocyclohexan-1,3-diol gemäß Schritt a) des erfindungsgemäßen Verfahrens erfolgt vorzugsweise basenkatalysiert, bevorzugt unter Verwendung von Natronlauge als Base. Bevorzugt wird die Reaktion in einem protischen Lösemittel durchgeführt, vorzugsweise in Methanol.

Zur Aminierung des in Schritt a) erhaltenen Nitrodiols sind vorzugsweise primäre Amine verwendbar, bevorzugt ausgewählt aus der Gruppe umfassend Pyrrolidin, Benzylamin, p-Methoxybenzylamin, p-Chlorbenzylamin und/oder 3,4-Dichlorbenzylamin. Vorzugsweise verwendbar ist Benzylamin. Bevorzugt wird die Reaktion in einem protischen Lösemittel durchgeführt, vorzugsweise in Wasser.

In bevorzugten Ausführungsformen wird die Reduktion der Nitrogruppe des Nitrodiamins zu einem primären Amin in Schritt c) des erfindungsgemäßen Verfahrens mit Methanol über Raney-Nickel oder mit Wasserstoff in Gegenwart des Katalysators Raney-Nickel ausgeführt. Bevorzugt verwendbar ist frisch aktiviertes Raney-Nickel. Vorzugsweise wird Wasserstoffgas der Reaktion zugeführt. Ein bevorzugter Druck des Wasserstoffs liegt im Bereich von 0,2 bar bis 100 bar, vorzugsweise im Bereich von 0,5 bar bis 8 bar, besonders bevorzugt bei 1 bar.

Die Reduktion kann in einem aprotischen Lösungsmittel durchgeführt werden. Bevorzugt wird die Reduktion in einem protischen Lösungsmittel durchgeführt. Die Reduktion wird vorzugsweise in einem Lösungsmittel ausgewählt

aus der Gruppe umfassend Methanol, Ethylacetat, Wasser und/oder Tetrahydrofuran durchgeführt, bevorzugt in Methanol. Bevorzugte Reaktionstemperaturen liegen in einem Bereich von 20°C bis 40 °C.

Die Umsetzung des in Schritt c) erhaltenen Cyclohexantriamins mit Dialkyloxalat erfolgt vorzugsweise in einem Lösungsmittel ausgewählt aus der Gruppe umfassend Methanol und/oder Ehtylacetat. Verwendbar sind vorzugsweise Dimethyl- und Diethyloxalat, besonders bevorzugt verwendbar ist Dimethyloxalat. Durch diese Umsetzung des Cyclohexantriamins mit Dialkyloxalat kann ein Ringschluss zu einem Perhydrochinoxalindion-Derivat erfolgen.

Die durch die Aminierung des in Schritt a) erhaltenen Nitrodiols mit primären oder sekundären Aminen vorhandenen Aminreste werden in Schritt e) abgespalten. Eine bevorzugt für die Aminierung verwendete Verbindung ist Benzylamin, daher erfolgt bevorzugt eine Debenzylierung des Benzylamin-Substituenten. Bevorzugt erfolgt eine Debenzylierung mit Wasserstoff bei 1 bar unter Verwendung von Palladium auf Aktivkohle als Katalysator. Wasserstoff kann auch in situ aus chemischen Wasserstoffquellen wie Ammoniumformiat, Hydrazin oder Ameisensäure gewonnen werden. Bevorzugt erfolgt eine Abspaltung des Benzylrestes durch katalystische Transferhydrogenolyse mit Ammoniumformiat und Palladium auf Aktivkohle. Vorzugsweise erfolgt die Reaktion unter Rückfluss. Ein bevorzugtes Lösemittel ist Methanol. Bevorzugt erfolgt die Erzeugung eines primären Amins.

In einem weiteren Schritt f) erfolgt eine Alkylierung der in Schritt e) erhaltenen Verbindung unter Einführen der Gruppen $R^2$ und $R^3$. Bevorzugt ist eine reduktive Alkylierung eines primären Amins. Beispielsweise kann eine Umsetzung mit Formalin und Natriumcyanoborhydrid ($NaBH_3CN$) in einem protischen Lösemittel, bevorzugt in Methanol, erfolgen.

Bevorzugt erfolgt eine Alkylierung des Amins mit Halogenalkanen. Vorzugsweise erfolgt eine Umsetzung mit Iod- oder Bromalkanen und $NaHCO_3$ in Acetonitril unter Rückfluss. Bevorzugt sind Iodalkane, vorzugsweise ausgewählt aus der Gruppe umfassend Iodmethan, Iodethan, 1,4-Diiodbutan, 1,5-Diiodpentan. Auch bevorzugt sind Bromalkane, insbesondere 1,4-Dibrombutan-2-ol. Bevorzugt verwendbar sind Iodmethan oder Iodethan.

Zur Bildung eines stickstoffhaltigen Rings sind vorzugsweise endständig halogenierte Dihalogenalkane verwendbar. Besonders bevorzugt sind Dihalogenalkane umfassend zwei bis sechs C-Atome. Diese können einfach oder zweifach substituiert sein mit OH- und/oder CarbonylGruppen. Ganz besonders bevorzugt sind die Dihalogenalkane mit vier C-Atomen. Bevorzugt sind die Dihalogenalkane ausgewählt aus der Gruppe umfassend 1,4-Diiodbutan, 1,4-Dibrombutan-2-ol und/oder 1,5-Diiodpentan.

Die Alkylierung kann unter Verwendung von Hilfsbasen erfolgen. Bevorzugte Hilfsbasen sind ausgewählt aus der Gruppe umfassend Kaliumcarbonat, Natriumcarbonat, Kaliumhydrogencarbonat und/oder Natriumhydrogencarbonat. Die Alkylierung kann in einem aprotischen Lösungsmittel durchgeführt werden. Bevorzugt wird die Alkylierung in einem protischen Lösungsmittel durchgeführt. Verwendbare Lösungsmittel sind vorzugsweise ausgewählt aus der Gruppe umfassend Aceton, Acetonitril und/oder Methanol, insbesondere Acetonitril.

Die Reduktion des Perhydrochinoxalindion-Rings der in Schritt f) erhaltenen Verbindung zum Perhydrochinoxalin erfolgt vorzugsweise unter Verwendung des Reduktionsmittels Lithiumaluminiumhydrid ($LiAlH_4$). Weiter bevorzugt ist eine Kombination mit Lewis-Säuren, beispielsweise Aluminiumchlorid. Bevorzugt ist ein 3:1-Gemisch von Lithiumaluminiumhydrid ($LiAlH_4$) und Aluminiumchlorid. Die Reduktion kann in einem aprotischen Lösungsmittel durchgeführt werden. Bevorzugt wird die Reduktion in einem protischen Lösungsmittel durchgeführt. Ein bevorzugtes Lösungsmittel ist Tetrahydrofuran (THF). Vorzugsweise wird unter Stickstoff-Schutzgasatmosphäre reduziert.

In einem weiteren Schritt erfolgt eine Acylierung des in Schritt g) erhaltenen sekundären Amins unter Einführen einer Gruppe C(O)-A-Z.

Die Acylierung kann mit Acylierungsmitteln wie Säurechloriden oder analogen freien Carbonsäuren erfolgen. Bevorzugt erfolgt die Acylierung mit Säurechloriden. Besonders bevorzug verwendbar sind Phenylacetylchloridderivate. Besonders bevorzugt sind 2-(3,4-Dichlorphenyl)acetylchlorid und 2-Phenylacetylchlorid. Die Acylierung mit Carbonsäuren erfolgt bevorzugt mit Katalysatoren. Besonders bevorzugt sind diese ausgewählt aus der Gruppe umfassend Dicyclohexylcarbodiimid (DCC) und N'-(3-Dimethylaminopropyl)-N-ethylcarbodiimid (EDC).

Vorzugsweise erfolgt die Acylierung von Hydroxypyrrolidin-Derivaten mit Acylierungsmitteln im Verhältnis von 1:1.

In einem weiteren Verfahrensschritt erfolgt eine Einführung eines Restes $R^1$, vorzugsweise durch Alkylierung, Acylierung oder hydrogenolytische Einführung von H.

In bevorzugten Ausführungsformen kann ein vorhandener Rest hydrogenolytisch abgespalten werden, wodurch eine hydrogenolytische Einführung von H als Rest $R^1$ erfolgt. Anschließend können in einem Schritt weitere Reste $R^1$ eingeführt werden.

Für eine hydrogenolytischen Spaltung werden vorzugsweise elementarer Wasserstoff mit Palladium auf Kohlenstoff als Katalysator eingesetzt. Bevorzugt wird dem Gemisch Salzsäure zugegeben. Die hydrogenolytische Spaltung kann in einem aprotischen Lösungsmittel durchgeführt werden. Bevorzugt wird die hydrogenolytische Spaltung in einem protischen Lösungsmittel durchgeführt. Bevorzugte Lösungsmittel sind ausgewählt aus der Gruppe umfassend Wasser und/oder Tetrahydrofuran (THF). Bevorzugt sind 1:1-Gemische von Wasser und Tetrahydrofuran. Ein bevorzugter Druck des Wasserstoffs liegt im Bereich von 0,5 bar bis 8 bar, vorzugsweise bei 1 bar.

Der Rest $R^1$ kann insbesondere bevorzugt durch Alkylierung oder Acylierung des sekundären Amins eingeführt

werden.

Vorzugsweise wird eine Alkylierung mit Aldehyden als reduktive Alkylierung durchgeführt.

Als Katalysator werden vorzugsweise Natriumcyanoborhydrid oder Natriumtriacetoxyborhydrid verwendet. Besonders bevorzugt sind Aldehyde ausgewählt aus der Gruppe umfassend Formaldehyd, Butyraldehyd, Anisaldehyd, Pyridin-2-carbaldehyd, Nicotinaldehyd und/oder 1H-Imidazol-5-carbaldehyd.

Vorzugsweise erfolgt eine Acylierung mit Acylierungsmitteln wie Säurechloriden oder analogen freien Carbonsäuren. Besonders bevorzugt ist die Acylierung mit Säurechloriden. Ganz besonders bevorzugt sind Säurechloride ausgewählt sind aus der Gruppe umfassend Benzoylchlorid, Acetylchlorid, Propionylchlorid, Methylchlorformiat, Ethylchlorformiat, Malonsäuremonomethylesterchlorid und/oder Bernsteinsäureanhydrid.

Veresterte Reste können durch Esterspaltung in freie Säuren überführt werden.

Die unter Rückfluss durchführbaren Reaktionen können auch in einer Synthese-Mikrowelle durchgeführt werden.

Durch die Umsetzung des Cyclohexantriamins mit Dimethyloxalat zu einem Cinoxalinderivat in Schritt d) des erfindungsgemäßen Verfahrens entsteht ein Racemat umfassend zwei Enantiomere.

In bevorzugten Ausführungsformen des Verfahrens kann daher vorgesehen sein, Racemate zu trennen. In weiter bevorzugten Ausführungsformen des Verfahrens kann vorgesehen sein, Diastereomeren-Gemische zu trennen.

Die Trennung der Racemate, Diastereomere oder Enantiomere kann nach bekannten Methoden, insbesondere chromatographischen Methoden, vorzugsweise mittels Hochleistungsflüssigkeitchromatographie (high performance liquid chromatography, HPLC) oder Säulenchromatographie oder Flash-Chromatographie (FC) erfolgen.

Eine Trennung von Racematen, Diastereomeren oder Enantiomere erfolgt vorzugsweise mittels chiraler chromatographischer Verfahren, insbesondere chiraler Hochleistungsflüssigkeitchromatographie. Chirales Säulenmaterial ist kommerziell erhältlich.

Die Trennung eines Racemats kann auch durch die Umsetzung eines racemischen Gemischs einer organischen Säure mit einem reinen Enantiomer einer Säure erfolgen. Die entstandenen diastereomeren Salze lassen sich durch fraktionierende Kristallisation trennen. Die Spaltung des Racemats erfolgt vorzugsweise dadurch, dass das Racemat mit einer enantiomerenreinen Säure umgesetzt wird. Anschließend erfolgt die Trennung durch fraktionierende Umkristallisation oder chromatographische Verfahren, wobei die Verfahren kombiniert und mehrfach durchgeführt werden können.

Im Sinne der vorliegenden Erfindung ist die angegebene Reihenfolge der Verfahrensschritte a) bis i) nicht im Sinne einer festgelegten Abfolge zu verstehen. Abhängig von dem gewählten Verfahren kann die Reihenfolge der Verfahrensschritte entsprechend variieren. Es ist bevorzugt, dass die Verfahrensschritte in der angegebenen Reihenfolge durchgeführt werden.

In bevorzugten Ausführungsformen können die erhaltenen Verbindungen aufreinigt werden, beispielsweise mittels chromatographischer Verfahren, vorzugsweise beispielsweise mittels Hochleistungsflüssigkeitchromatographie oder Säulenchromatographie.

Beispiele, die der Veranschaulichung der vorliegenden Erfindung dienen, sind nachstehend angegeben.

Für die chemischen Reaktionen wurden Rundkolben verwendet. Fanden hydrolyse- und/oder oxidationsempfindliche Substanzen Anwendung oder wurden Hydrierungen mit elementarem Wasserstoff durchgeführt, so wurden trockene Schlenkkolben eingesetzt. Als Inertgas wurde Stickstoff der Firma Air Liquide, Düsseldorf eingesetzt. Beim Arbeiten mit Inertgas wurden Substanzen entweder im Gegenstrom oder durch Septen hinzugefiigt.

Reaktionen bei 0 °C wurden durch ein Eis-/Wassergemisch gekühlt.

Der Reaktionsverlauf und die damit verbundene Vollständigkeit einer Reaktion wurde durch Dünnschichtchromatographie überwacht.

Isolierte Substanzen wurden bei +5 °C gelagert.

Die eingesetzten Lösemittel wurden in p.A.- Qualität (p.A., zur Analyse) bezogen und ohne weitere Reinigung verwendet. Wasserfreie, absolute Lösemittel wurden durch Destillation über einem Trockenmittel in einer Intergasatmosphäre hergestellt. Wasser wurde in demineralisierter Form eingesetzt.

Eine Reinigung der Verbindungen erfolgte mittels Flash-Chromatographie, einer Variante der Säulenchromatographie. Als stationäre Phase wurde Kieselgel 60 (40 - 63 μm) der Firma Merck verwendet. Die mobile Phase, der Säulendurchmesser (0), die Kieselgel-Füllhöhe sowie das Fraktionsvolumen wurden den Versuchsbedingungen angepasst und werden bei den einzelnen Herstellungsvorschriften beschrieben.

Beispiel 1

Herstellung von 2-(3,4-Dichlorphenyl)-1-[(4aRS,8SR,8aRS)-8-(pyrrolidin-1-yl)-perhydrochinoxalin-1 -yl]-ethan-1-on

1.1 Herstellung von (2r)-2-Nitrocyclohexan-1,3-diol

In einen 11- Rundkolben wurden einer 25 %igen, wässrigen Glutaraldehyd-Lösung (182 ml, 460 mmol), Nitromethan (38 ml, 0,71 mol) und $CH_3OH$ (600 ml) gegeben. Bei 0 bis 5 °C wurde 2 M NaOH (12 ml) zugetropft. Das Eisbad wurde entfernt und der Ansatz 4 Stunden bei Raumtemperatur (20-23°C) gerührt. Die entstandene gelbe Lösung wurde durch Zugabe von saurem Kationentauscher (Merck) (16,8 g) neutralisiert und 20 Minuten lang gerührt. Das Austauscherharz wurde abfiltriert und mit wenig $CH_3OH$ gewaschen. Das Filtrat wurde im Vakuum bis zu einem halbfesten Zustand eingedampft. Der Rückstand wurde in EtOH (100 ml) und Toluol (250 ml) aufgenommen. Das entstandene Zweiphasengemisch wurde erneut im Vakuum eingedampft. Der entstanden Feststoff wurde in heißem (65°C bis 70°C) EtOH (100 ml) gelöst und mit Toluol (250 ml) versetzt. Die entstandenen farblosen Kristalle wurden abfiltriert und im Hochvakuum getrocknet.

1.2 Herstellung von (2*r*-)-$N^1$,$N^3$-Dibenzyl-2-nitrocyclohexan-1,3-diamin

In einem 250 ml Rundkolben wurde Benzylamin (26,4 ml, 0,24 mol) in $H_2O$ (60 ml) gelöst und mit (2r-)-2-Nitrocyclohexan-1,3-diol (19,3 g, 0,12 mol) versetzt. Die gelbe Lösung wurde 16 Stunden lang bei Raumtemperatur gerührt. Der entstandene gelbe Niederschlag wurde abfiltriert und anschließend mit $CH_3OH$ umkristallisiert. Man erhielt einen farblosen Feststoff.

1.3 Herstellung von (2*r*)-$N^1$,$N^3$-Dibenzylcyclohexan-1,2,3-triamin

(2r)-$N^1$,$N^3$-Dibenzyl-2-nitrocyclohexan-1,3-diamin (0,34 g, 1.,0 mmol) wurde in $CH_3OH$ (2,5 ml) gelöst und mit Raney-Nickel (Acros Organics, Geel, Belgien) versetzt (0,96 g; 1 ml abgesetzte Suspension enthielt etwa 0,6 g Raney-Nickel; vgl. Gattermann, L; Wieland, H.; Wieland, T.; Sucrow, W. Die Praxis des organischen Chemikers, 43. Auflage; Walter de Gryter: Berlin 1982; 555). Die Suspension wurde bei 1 bar $H_2$ 3 Stunden lang bei Raumtemperatur gerührt. Anschließend wurde vom Katalysator abfiltriert und die Lösung im Vakuum eingedampft. Man erhielt ein hellgelbes Öl.

1.4 Herstellung von (4aRS,5SR,8aRS)-1-Benzyl-5-(benzylamino)perhydrochinoxalin-2,3-dion

(2*r*)-$N^1$,$N^3$-Dibenzylcyclohexan-1,2,3-triamin (100 mg, 0,32 mmol) wurde in $CH_3OH$ (2,0 ml) gelöst und mit Dimethyloxalat (38 mg, 0,32 mmol) versetzt. Das Gemisch wurde 24 Stunden unter Rückfluss erhitzt. Anschließend wurde im Vakuum eingedampft. Der Rückstand wurde aus Ethylacetat umkristallisiert. Es wurde das Produkts als farbloser Feststoff erhalten.

1.5 Herstellung von (4aRS,5SR,8aRS)-5-Amino-1-benzylperhydrochinoxalin-2,3-dion

(4aRS,5SR,8aRS)-1-Benzyl-5-(benzylamino)perhydrochinoxalin-2,3-dion (1,19 g, 3,28 mmol) wurde in Methanol (40 ml) gelöst und mit $NH_4HCO_2$ (2,07 g, 32,8 mmol) versetzt. Des weiteren wurde 120 mg Palladium auf Kohlenstoff (Merck) zugegeben. Das Gemisch wurde 2 Stunden lang unter Rückfluss erhitzt. Anschließend wurde vom Katalysator abfiltriert und im Vakuum eingedampft. Der Rückstand wurde in $CH_2Cl_2$ aufgenommen und drei Mal mit 0,1 N NaOH gewaschen. Die organische Phase wurde über $Na_2SO_4$ getrocknet und im Vakuum eingedampft. Man erhielt einen farblosen Feststoff.

1.6 Herstellung von (4aRS,5SR,8aRS)-1-Benzyl-5-(pyrrolidin-1-yl)perhydrochinoxalin-2,3-dion

(4aRS,5SR,8aRS)-5-Amino-1-benzylperhydrochinoxalin-2,3-dion (3,06 g, 11,2 mmol) wurde in $CH_3CN$ (300 ml) gelöst. Es wurden $NaHCO_3$ (6,4 g, 76,2 mmol) und 1,4-Diiodbutan (13,9 g, 44,8 mmol, 5,9 ml) hinzu gegeben und 18 Stunden lang unter Rückfluss erhitzt. $NaHCO_3$ wurde mit Blaubandfilter (Schleicher&Schuell) abgetrennt und die gelbe Lösung im Vakuum eingeengt. Der Feststoff wurde in $CH_2Cl_2$ aufgenommen und drei Mal mit HCl (1 N) ausgeschüttelt. Anschließend wurde die wässrige Phase mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Man erhielt einen hellgelben Feststoff.

1.7 Herstellung von (4aRS,5SR,8aRS)-1-Benzyl-5-(pyrrolidin-1-yl)perhydrochinoxalin

Herstellung des $Al(AlH_4)_3$;

Getrocknetes $AlCl_3$ (45 mg, 0,33 mmol) wurde bei 0 °C unter Stickstoffatmosphäre in einen Schlenkkolben gegeben und mit absolutem THF (2,5 ml) versetzt. Die entstandene klare, farblose Lösung wurde 5 Minuten lang bei 0 °C gerührt. Anschließend wurde 1,0 M $LiAlH_4$-Lösung (1,0 ml, 1,0 mmol) hinzugetropft. Die Suspension wurde auf Raumtemperatur erwärmt und 20 Minuten lang gerührt. Es entstand eine Suspension mit 1,33 mmol $Al(AlH_4)_3$.

Reduktion:

(4aRS,5SR,8aRS)-1-Benzyl-5-(pyrrolidin-1-yl)perhydrochinoxalin-2,3-dion (59 mg, 0,18 mmol) wurde in abs. THF (3 ml) gelöst und zu der auf 0 °C abgekühlten $Al(AlH_4)_3$-Suspension gegeben. Die Suspension wurde 45 Minuten lang bei 0 °C gerührt, auf Raumtemperatur aufgewärmt und weitere 20 Minuten lang gerührt. Danach wurden unter Eiskühlung 2 N NaOH (2 ml) vorsichtig zugetropft. Die Aufschlämmung wurde fünf Mal mit $CH_2Cl_2$ (15 ml) ausgeschüttelt. Die vereinigten organischen Phasen wurden über $Na_2SO_4$ getrocknet und im Vakuum eingedampft. Das Produkt wurde als hellgelber Feststoff gewonnen.

1.8 Herstellung von 1-[(4aRS,8SR,8aRS)-4-Benzyl-8-(pyrrolidin-1-yl)-perhydrochinoxalin-1-yl]-2-(3,4-dichlorphenyl) ethan-1-on

(4aRS,5SR,8aRS)-1-Benzyl-5-(pyrrolidin-1-yl)perhydrochinoxalin (325 mg, 1,09 mmol) wurde in abs. $CH_2Cl_2$ (35 ml) gelöst. Es wurde 2-(3,4-Dichlorphenyl)acetylchlorid (291 mg, 1,3 mmol) hinzugetropft und bei Raumtemperatur gerührt. Nach 30 Minuten wurde 2 N NaOH (35 ml) zugegeben und 2 Stunden lang kräftig gerührt. Die wässrige Phase wurde abgetrennt. Die organische Phase wurde drei Mal mit HCl (1N) ausgeschüttelt. Anschließend wurde die wässrige Phase mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Man erhielt einen hellgelben Feststoff.

1.9 Herstellung von 2-(3,4-Dichlorphenyl)-1-[(4aRS,8SR,8aRS)-8-(pyrrolidin-1-yl)-perhydrochinoxalin-1-yl]-ethan-1-on

1-[(4aRS,8SR,8aRS)-4-Benzyl-8-(pyrrolidin-1-yl)perhydrochinoxalin-1-yl]-2-(3,4-dichlorphenyl)ethan-1-on (244 mg, 0,50 mmol) wurde in THF / $H_2O$ (1:1, 50 ml) gelöst und mit konz. HCl (5 ml) sowie Palladium auf Kohlenstoff (Pd/C) (Merck) (98,4 mg) versetzt. Der Ansatz wurde 30 Minuten bei 1 bar $H_2$ bei Raumtemperatur gerührt. Der Katalysator wurde abfiltriert und THF im Vakuum verdampft. Die wässrige Phase wurde mit NaOH (2 N) auf pH 8 gebracht und fünf Mal mit $CH_2Cl_2$ ausgeschüttelt. Die vereinigten organischen Phasen wurden über $Na_2SO_4$ getrocknet und eingedampft. Der gelbliche Rückstand wurde säulenchromatographisch über Kieselgel 60 (40 - 63 μm, (Merck) Säule Ø 3 cm, $CH_2Cl_2$ /MeOH / $NH_3$ 9:1:0,1, 1= 17 cm, V = 10 ml) gereinigt und ergab ein gelbes Harz.

Beispiel 2

Herstellung des Diastereomeren-Gemischs 2-(3,4-Dichlorphenyl)-1-{(4aRS,8SR,8aSR)-8-[(3SR)- 3-hydroxy-pyrrolidin-1-yl]perhydrochinoxalin-1-yl}ethan-1-on und 2-(3,4-Dichlorphenyl)-1-{(4aRS,8SR,8aSR)-8-[(3RS)-3-hydroxy-pyrrolidin-1-yl]perhydrochinoxalin-1-yl}ethan-1-on

Die Herstellung erfolgte ausgehend von (4aRS,5SR,8aRS)-5-Amino-1-benzylperhydrochinoxalin-2,3-dion, das wie in Beispiel 1.1 bis 1.5 beschrieben hergestellt wurde.

2.1 Herstellung von (4aRS,5SR,8aRS)-1-Benzyl-5-(3-hydroxypyrrolidin-1-yl)perhydrochinoxalin-2,3-dion

In Acetonitril (16 ml) wurde (4aRS,5SR,8aRS)-5-Amino-1-benzylperhydrochinoxalin-2,3-dion (144 mg, 0,53 mmol) gelöst und $NaHCO_3$ (300 mg, 3,57 mmol) und racemisches 1,4-Dibrombutan-2-ol (Reinheit 85 %, 1,15 g, 4,20 mmol, 0,57 ml) zugegeben. Nach 24 Stunden wurde $NaHCO_3$ abgetrennt und im Vakuum eingedampft Der Feststoff wurde in $CH_2Cl_2$ aufgenommen und drei Mal mit HCl (1N) ausgeschüttelt. Anschließend wurde die wässrige Phase mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Das Rohprodukt wurde flashchromatographisch über Kieselgel 60 (40 - 63 μm, (Merck) Ø 2 cm, Aceton / MeOH / $Et_2NH$ 9,5 : 0,5 : 0,1,1= 17 cm, V = 5 ml) gereinigt. Ein hellgelber Feststoff wurde isoliert.

2.2 Herstellung von (4aRS,5SR,8aRS)-1-Benzyl-5-((3SR)- und (3*RS*)-hydroxypyrrolidin-1-yl)-perhydrochinoxalin

Herstellung des $Al(AlH_4)_3$:

Getrocknetes $AlCl_3$ (940 mg, 6,8 mmol) wurde bei 0 °C unter Stickstoffatmosphäre in einen Schlenkkolben gegeben und mit abs. THF (52 ml) versetzt. Die entstandene klare, farblose Lösung wurde 5 Minuten lang bei 0 °C gerührt. Anschließend wurde 1,0 M $LiAlH_4$-Lösung (21 ml, 21 mmol) hinzugetropft. Die Suspension wurde auf Raumtemperatur erwärmt und 20 Minuten lang gerührt. Es entstand eine Suspension mit 27,8 mmol $Al(AlH_4)_3$.

Reduktion:

(*4aRS,5SR,8aRS*)-1-Benzyl-5-(3-hydroxypyrrolidin-1-yl)perhydrochinoxalin-2,3-dion (1,29 g, 3,8 mmol) wurde in abs. THF (65 ml) gelöst und zu der auf 0 °C abgekühlten $Al(AlH_4)_3$-Suspension gegeben. Die Suspension wurde 45 Minuten lang bei 0 °C gerührt, auf Raumtemperatur aufgewärmt und weitere 20 Minuten lang gerührt. Danach wurden unter Eiskühlung 2 N NaOH (13 ml) vorsichtig zugetropft. Die Aufschlämmung wurde fünf Mal mit $CH_2Cl_2$ (50 ml) ausgeschüttelt. Die vereinigten organischen Phasen wurden über $Na_2SO_4$ getrocknet und im Vakuum eingedampft. Das Produkt wurde als hellgelber Feststoff gewonnen.

2.3 Herstellung von 1-{(4aRS,8SR,8aSR)-4-Benzyl-8-[(3SR)- und (3RS)-3-hydroxypyrrolidin-1-yl]perhydrochinoxalin-1-yl}-2-(3,4-dichlorphenyl)ethan-1-on

(4aRS,5SR,8aRS)-1-Benyzl-5-((3SR)- und (3RS)-hydroxypyrrolidin-1-yl)-perhydrochinoxalin (2,6 g, 8,1 mmol) wurde in abs. $CH_2Cl_2$ (200 ml) gelöst, 2-(3,4-Dichlorphenyl)acetylchlorid (1,8 g, 8,1 mmol) hinzugetropft und bei Raumtemperatur gerührt. Nach 30 Minuten wurde NaOH (2 N, 200 ml) zugegeben und die Reaktionsmischung über Nacht kräftig gerührt. Die wässrige Phase wurde abgetrennt. Die organische Phase wurde drei Mal mit HCl (1N) ausgeschüttelt. Anschließend wurde die wässrige Phase mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Es wurde ein hellgelber Feststoff isoliert.

2.4 Herstellung von 2-(3,4-Dichlorphenyl)-1-{(4aRS,8SR,8aSR)-8-[(3SR)- und (3RS)-3-hydroxypyrrolidin-1-yl]perhydrochinoxalin-1-yl}ethan-1-on

1-{(4aRS,8SR,8aSR)-4-Benzyl-8-[(3SR)- und (3RS)-3-hydroxypyrrolidin-1-yl]perhydrochinoxalin-1-yl}-2-(3,4-dichlorphenyl)ethan-1-on (373 mg, 0,74 mmol) wurde in $THF/H_2O$ (1:1, 74 ml) gelöst und mit konzentrierter HCl (7,4 ml) sowie 158 mg Palladium auf Kohlenstoff (Merck) versetzt. Der Ansatz wurde 30 Minuten bei 1 bar $H_2$ bei Raumtemperatur gerührt. Der Katalysator wurde abfiltriert und THF im Vakuum verdampft. Die wässrige Phase wurde mit NaOH (2 N) auf pH 8 gebracht und fünf Mal mit $CH_2Cl_2$ ausgeschüttelt. Die vereinigten organischen Phasen wurden über $Na_2SO_4$ getrocknet und eingedampft. Der gelbliche Rückstand wurde säulenchromatographisch über Kieselgel 60 (40 - 63 $\mu$m, (Merck) 0 3 cm, $CH_2Cl_2$ / MeOH / $NH_3$ 9:1:0,1,1= 18 cm, V = 10 ml) gereinigt und ergab ein leicht gelbes Harz.

Beispiel 3

Herstellung von 1-[(4aRS,8SR,8aRS)-4-Benzyl-8-(pyrrolidin-1-yl)perhydrochinoxalin-1-yl]-2-(3,4-dichlorphenyl)ethan-1-on

Die Herstellung erfolgte ausgehend von (4aRS,5SR,8aRS)-1-Benzyl-5-(pyrrolidin-1-yl)perhydrochinoxalin-2,3-dion, das wie in Beispiel 1.1 bis 1.6 beschrieben hergestellt wurde.

3.1 Herstellung von (4aRS,5SR,8aRS)-1-Benzyl-5-(pyrrolidin-1-yl)perhydrochinoxalin

Herstellung des $Al(AlH_4)_3$:

Getrocknetes $AlCl_3$ (45 mg, 0,33 mmol) wurde bei 0 °C unter Stickstoffatmosphäre in einen Schlenkkolben gegeben und mit abolutem THF (2,5 ml) versetzt. Die entstandene klare, farblose Lösung wurde 5 Minuten lang bei 0 °C gerührt. Anschließend wurde 1,0 M $LiAlH_4$-Lösung (1,0 ml, 1,00 mmol) hinzugetropft. Die Suspension wurde auf Raumtemperatur erwärmt und 20 Minuten lang gerührt. Es entstand eine Suspension mit 1,33 mmol $Al(AlH_4)_3$:

Reduktion:

(4aRS,5SR,8aRS)-1-Benzyl-5-(pyrrolidin-1-yl)perhydrochinoxalin-2,3-dion (59 mg, 0,18 mmol) wurde in abolutem THF (3 ml) gelöst und zu der auf 0 °C abgekühlten $Al(AlH_4)_3$-Suspension gegeben. Die Suspension wurde 45 Minuten lang bei 0 °C gerührt, auf Raumtemperatur aufgewärmt und weitere 20 Minuten lang gerührt. Danach wurden unter Eiskühlung 2 N NaOH (2 ml) vorsichtig zugetropft. Die Aufschlämmung wurde fünf Mal mit $CH_2Cl_2$ (15 ml) ausgeschüttelt. Die vereinigten organischen Phasen wurden über $Na_2SO_4$ getrocknet und im Vakuum eingedampft. Das Produkt wurde als hellgelber Feststoff gewonnen.

3.2 Herstellung von 1-[(4aRS,8SR,8aRS)-4-Benzyl-8-(pyrrolidin-1-yl)perhydrochinoxalin-1-yl]-2-(3,4-dichlorphenyl) ethan-1-on

(4aRS,5SR,8aRS)-1-Benzyl-5-(pyrrolidin-1-yl)perhydrochinoxalin (325 mg, 1,09 mmol) wurde in abolutem $CH_2Cl_2$ (35 ml) gelöst. Es wurde 2-(3,4-Dichlorphenyl)acetylchlorid (291 mg, 1,3 mmol) hinzugetropft und bei Raumtemperatur gerührt. Nach 30 Minuten wurde 2 N NaOH (35 ml) zugegeben und 2 Stunden lang kräftig gerührt. Die wässrige Phase wurde abgetrennt. Die organische Phase wurde drei Mal mit HCl (1N) ausgeschüttelt. Anschließend wurde die wässrige Phase mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Das Produkt wurde als hellgelber Feststoff erhalten.

Beispiel 4

Herstellung von〈(*3SR*)- und (*3RS*)-1-{(*4aRS*,*5RS*,*8aSR*)-4-[2-(3,4-dichlorphenyl)acetyl]-perhydrochinoxalin-5-yl}pyrrolidin-3-yl〉-2-(3,4-dichtorphenyl)acetat

Die Herstellung erfolgte ausgehend von (4aRS,5SR,8aRS)-1-Benyzl-5-((3SR)- und (*3RS*)-hydroxypyrrolidin-1-yl)-perhydrochinoxalin, das wie in Beispiel 2.1 bis 2.2 beschrieben hergestellt wurde.

4.1 Herstellung von 〈(*3SR*)- und (*3RS*)-1-{(*4aRS*,*5RS*,*8aSR*)-1-Benzyl-4-[2-(3,4-dichlorphenyl)acetyl]-perhydrochinoxalin-5-yl}pyrrolidin-3-yl〉-2-(3,4-dichlorphenyl)acetat

(4aRS,5SR,8aRS)-1-Benyzl-5-((3SR)- und (3RS)-hydroxypyrrolidin-1-yl)-perhydrochinoxalin (0,70 g, 2,2 mmol)wurde in abolutem $CH_2Cl_2$ (100 ml) gelöst. Es wurde 2-(3,4-Dichlorphenyl)acetylchlorid (1,1 g, 4,9 mmol) hinzugetropft und der Ansatz bei Raumtemperatur gerührt. Nach 4 Stunden wurde 2 N NaOH (4,5 ml) zugegeben und über Nacht kräftig gerührt. Die organische Phase wurde abgetrennt und zwei Mal mit 2 N NaOH gewaschen. Anschließend wurden die organische Phase über $Na_2SO_4$ getrocknet und im Vakuum eingedampft. Der Rückstand wurde zwei Mal flashchromatographisch über Kieselgel 60 (40 - 63 $\mu$m, (Merck) Säule 0 2 cm, EE / $Et_2NH$ 10:0,1;1= 15 cm, V = 5 ml) gereinigt. Es wurde ein hellgelbes Harz isoliert.

4.2 Herstellung von〈(3SR)- und (3RS)-1-{(4aRS,5RS,8aSR)-4-[2-(3,4-dichlorphenyl)-acetyl]-perhydrochinoxalin-5-yl} pyrrolidin-3-yl〉-2-(3,4-dichlorphenyl)acetat

〈(*3SR*)- und (*3RS*)-1-{(*4aRS*,*5RS*,*8aSR*)-1-Benzyl-4-[2-(3,4-dichlorphenyl)acetyl]-perhydrochinoxalin-5-yl} pyrrolidin-3-yl〉-2-(3,4-dichlorphenyl)acetat (299 mg, 0,43 mmol) wurde in THF / $H_2O$ (1:1, 59 ml) gelöst und mit konzentrierter HCl (5,9 ml) sowie Pd/C (81 mg) versetzt. Der Ansatz wurde 35 Minuten bei 1 bar $H_2$ bei Raumtemperatur gerührt. Der Katalysator wurde abfiltriert und das Methanol des Filtrats im Vakuum verdampft. Die wässrige Phase wurde mit NaOH (2 N) auf pH 8 gebracht und fünf Mal mit $CH_2Cl_2$ ausgeschüttelt. Die organische Phase wurde über $Na_2SO_4$ getrocknet und eingedampft. Der gelbliche Rückstand wurde säulenchromatographisch gereinigt (0 2 cm, $CH_2Cl_2$ / MeOH /$NH_3$ 9.5:0.5:0.05, 1= 16 cm, V = 5 ml). Die Produktfraktionen wurden eingedampft, der Rückstand in $CH_2Cl_2$ aufgenommen und die Lösung drei Mal mit HCl (1 N) ausgeschüttelt. Anschließend wurde die wässrige Phase mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Der gelbliche Rückstand wurde säulenchromatographisch gereinigt (0 2 cm, $CH_2Cl_2$ / MeOH / $NH_3$ 9:1:0,1,1 = 15 cm, V = 5 ml) und ergab einen gelblichen Feststoff.

Beispiel 5

Herstellung von 1-{(4aRS,8SR,8aSR)-4-Benzyl-8-[(3SR)- und (3RS)-3-hydroxypyrrolidin-1-yl]perhydrochinoxalin-1-yl} -2-(3,4-dichlorphenyl)ethan-1-on

Die Herstellung erfolgte ausgehend von (4aRS,5SR,8aRS)-1-Benyzl-5-((3SR)- und (3RS)-hydroxypyrrolidin-1-yl)-perhydrochinoxalin, das wie in Beispiel 2.1 bis 2.2 beschrieben hergestellt wurde.

(4aRS,5SR,8aRS)-1-Benyzl-5-((3SR)- und (3RS)-hydroxypyrrolidin-1-yl)-perhydrochinoxalin (2,6 g, 8,1 mmol) wurde in abolutem $CH_2Cl_2$ (200 ml) gelöst, 2-(3,4-Dichlorphenyl)acetylchlorid (1,8 g, 8,1 mmol) hinzugetropft und bei Raumtemperatur gerührt. Nach 30 Minuten wurde NaOH (2 N, 200 ml) zugegeben und die Reaktionsmischung über Nacht kräftig gerührt. Die wässrige Phase wurde abgetrennt. Die organische Phase wurde drei Mal mit HCl (1N) ausgeschüttelt. Anschließend wurde die wässrige Phase mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Es wurde ein hellgelber Feststoff isoliert.

Beispiel 6

Herstellung von 1-[(4aRS,8SR,8aRS)-4-Benzoyl-8-(pyrrolidin-1-yl)-perhydrochinoxalin-1-yl]-2-(3,4-dichlorphenyl) ethan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Unter $N_2$ wurde 2-(3,4-Dichlorphenyl)-1-[(4aRS,8SR,8aRS)-8-(pyrrolidin-1-yl)-perhydrochinoxalin-1-yl]-ethan-1-on (111 mg, 0,28 mmol) in abolutem $CH_2Cl_2$ (14 ml) gelöst und Benzoylchlorid (47 mg, 0,35 mmol) zugetropft. Der Ansatz wurde über Nacht bei Raumtemperatur gerührt und anschließend im Vakuum eingedampft. Der Rückstand wurde flashchromatographisch gereinigt (0 2 cm, $CH_2Cl_2$ / MeOH / $NH_3$ 9,5:0,5:0,05, 1= 15 cm, V = 5 ml) und als gelbliches Harz gewonnen.

Beispiel 7

Herstellung von 1-[(4aRS,8SR,8aRS)-4-Acetyl-8-(pyrrolidin-1-yl)-perhydrochinoxalin-1-yl]-2-(3,4-dichlorphenyl)ethan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Unter $N_2$ wurde 2-(3,4-Dichlorphenyl)-1-[(4aRS,8SR,8aRS)-8-(pyrrolidin-1-yl)-perhydrochinoxalin-1-yl]-ethan-1-on (113 mg, 0,29 mmol) in abolutem $CH_2Cl_2$ (14 ml) gelöst und Acetylchlorid (27 mg, 0,34 mmol) zugetropft. Der Ansatz wurde über Nacht bei Raumtemperatur gerührt und anschließend im Vakuum eingedampft. Der Rückstand wurde flashchromatographisch gereinigt (Ø 2 cm, $CH_2Cl_2$ / MeOH / $NH_3$ 9,5:0,5:0,05, 1= 15 cm, V = 5 ml). Es wurde ein gelbliches Harz gewonnen.

Beispiel 8

Herstellung von 1-{(4*aRS*,*8SR*,*8aRS*)-1-[2-(3,4-Dichlorphenyl)acetyl]-8-(pyrrolidin-1-yl)-perhydrochinoxalin-4-yl} propan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Unter $N_2$ wurde 2-(3,4-Dichlorphenyl)-1-[(4aRS,8SR,8aRS)-8-(pyrrolidin-1-yl)-perhydrochinoxalin-1-yl]-ethan-1-on (40,7 mg, 0,10 mmol) in abolutem $CH_2Cl_2$ (5 ml) gelöst und Propionylchlorid (11,4 mg, 0,12 mmol) zugetropft. Der Ansatz wurde 2,5 Stunden bei Raumtemperatur gerührt und anschließend im Vakuum eingedampft. Der Rückstand wurde flashchromatographisch gereinigt (Ø 2 cm, $CH_2Cl_2$ / MeOH / $NH_3$ 9:1:0,1, 1= 16 cm, V = 3 ml). Es wurde ein gelbes Harz gewonnen.

Beispiel 9

Herstellung von Methyl-{(4*aRS*,*8SR*,*8aRS*)-1-[2-(3,4-dichlorphenyl)acetyl]-8-(pyrrolidin-1-yl)-perhydrochinoxalin-4-yl} carboxylat

Zur besseren Übersichtlichkeit wird bei dieser und den folgenden Verbindungen die Bezifferung des Chinoxalin-Rings von 2-(3,4-Dichlorphenyl)-1-[(4aRS,8SR,8aRS)-8-(pyrrolidin-1-yl)-perhydrochinoxalin-1-yl]-ethan-1-on übernommen

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

2-(3,4-Dichlorphenyl)-1-[(4aRS,8SR,8aRS)-8-(pyrrolidin-1-yl)-perhydrochinoxalin-1-yl]-ethan-1-on (100,9 mg, 0,25 mmol) wurde unter Stickstoffatmosphäre in abolutem $CH_2Cl_2$ (13 ml) gelöst und mit Methylchlorformiat (28,9 mg, 0,31 mmol) versetzt. Die Lösung wurde 2 Stunden lang bei Raumtemperatur gerührt. Danach wurde im Vakuum eingedampft und der Rückstand flashchromatographisch gereinigt (0 2 cm, $CH_2Cl_2$ / MeOH / $NH_3$ 9,5:0,5:0,05, 1=16 cm, V = 5 ml) und als gelbes Harz erhalten.

Beispiel 10

Herstellung von Ethyl-{(4*aRS*,*8SR*,*8aRS*)-1-[2-(3,4-dichlorphenyl)acetyl]-8-(pyrrolidin-1-yl)-perhydrochinoxalin-4-yl} carboxylat

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

2-(3,4-Dichlorphenyl)-1-[(4aRS,8SR,8aRS)-8-(pyrrolidin-1-yl)-perhydrochinoxalin-1-yl]-ethan-1-on (104,9 mg, 0,26 mmol) wurde unter $N_2$ in abolutem $CH_2Cl_2$ (13 ml) gelöst und mit Ethylchlorformiat (34,5 mg, 0,32 mmol) versetzt. Die Lösung wurde über Nacht bei Raumtemperatur gerührt, anschließend im Vakuum eingedampft. Der Rückstand wurde flashchromatographisch gereinigt (0 2 cm, $CH_2Cl_2$ / MeOH / $NH_3$ 9,5:0,5:0,05, 1= 18 cm, V = 5 ml). Es wurde ein gelbes Harz erhalten.

Beispiel 11

Herstellung von 3-{(4*aRS*,*8SR,*8*aRS)*-1-[2-(3,4-Dichlorphenyl)acetyl]-8-(pyrrolidin-1-yl)-perhydrochinoxalin-4-yl}-3-oxopropionsäure

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Unter $N_2$ wurde 2-(3,4-Dichlorphenyl)-1-[(4aRS,8SR,8aRS)-8-(pyrrolidin-1-yl)-perhydrochinoxalin-1-yl]-ethan-1-on (103 mg, 0,26 mmol) in abolutem $CH_2Cl_2$ (13 ml) gelöst und Malonsäuremonomethylesterchlorid (42 mg, 0,31 mmol) zugetropft. Der Ansatz wurde über Nacht bei Raumtemperatur gerührt und anschließend im Vakuum eingedampft. Der Rückstand wurde in 20 ml aufgenommen und mit 2 N NaOH (2 ml) versetzt. Die Lösung wurde über Nacht bei Raumtemperatur gerührt. Danach wurde im Vakuum eingedampft und der Rückstand durch Flashchromatographie gereinigt (Ø 2 cm, $CH_2Cl_2$ / MeOH /$NH_3$ 8:2:0,1, 1=15 cm, V = 5 ml). Das Rohprodukt wurde erneut in $CH_2Cl_2$ aufgenommen, mit einem Glasfiltertiegel G4 und Celite® (Kieselgur der Firma CELITE Corp., Lompoc, USA) filtriert und das Filtrat im Vakuum eingedampft. Es wurde ein farbloser Feststoff gewonnen.

Beispiel 12

Herstellung von 4- {(4*aRS, 8SR, 8aRS*)-1-[2-(3,4-Dichlorphenyl)acetyl]-8-(pyrrolidin-1-yl)perhydrochinoxalin-4-yl}-4-oxobuttersäure

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

In einen 50 ml Schlenkkolben wurde unter $N_2$ 2-(3,4-Dichlorphenyl)-1-[(4aRS,8SR,8aRS)-8-(pyrrolidin-1-yl)-perhydrochinoxalin-1-yl]-ethan-l-on (108,6 mg, 0,27 mmol) in abolutem $CH_2Cl_2$ (14 ml) gelöst. Die Lösung wurde mit Bernsteinsäureanhydrid (33 mg, 0,33 mmol) sowie einer Spatelspitze 4-(Dimethylamino)pyridin (DMAP) versetzt. Der Ansatz wurde über Nacht bei Raumtemperatur gerührt. Anschließend wurde im Vakuum fast bis zur Trockene eingedampft und flashchromatographisch gereinigt (Ø 2 cm, $CH_2Cl_2$ / MeOH / $NH_3$ 8:2:0,1, 1 = 17 cm, V = 5 ml). Die Fraktionen mit dem Produkt wurden im Vakuum eingedampft und erneut in $CH_2Cl_2$ aufgenommen. Es wurde mit einem Glasfiltertiegel

G4 und Celite filtriert und das Filtrat im Vakuum eingedampft. Die Ausbeute ergab einen gelblichen Feststoff.

Beispiel 13

Herstellung von Methyl-3-{(*4aRS,8SR,8aRS*)-1-[2-(3,4-Dichlorphenyl)acetyl]-8-(pyrrolidin-1-yl)-perhydrochinoxalin-4-yl}-3-oxopropionat

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Unter $N_2$ wurde 2-(3,4-Dichlorphenyl)-1-[(4aRS,8SR,8aRS)-8-(pyrrolidin-1-yl)-perhydrochinoxalin-1-yl]-ethan-1-on (101 mg, 0,26 mmol) in abolutem $CH_2Cl_2$ (13 ml) gelöst und Malonsäuremonomethylesterchlorid (42 mg, 0,31 mmol) zugetropft. Der Ansatz wurde über Nacht bei Raumtemperatur gerührt und anschließend im Vakuum eingedampft. Der Rückstand wurde durch Flashchromatographie gereinigt (Ø 2 cm, $CH_2Cl_2$ / MeOH / $NH_3$ 9,5:0,5:0,05,1= 15 cm, V = 5 ml). Es wurde ein gelbliches Harz gewonnen.

Beispiel 14

Herstellung von 1-{(*4aRS,8SR,8aRS*)-4-Benzoyl-8-[(3SR)- und (*3RS*)-3-hydroxypyrrolidin-1-yl]-perhydrochinoxalin-1-yl}-2-(3,4-dichlorphenyl)ethan-1-on

Die Herstellung erfolgte ausgehend von dem Diastereomeren-Gemisch 2-(3,4-Dichlorphenyl)-1-{(4aRS,8SR, 8aSR)-8-[(3SR)-3-hydroxypyrrolidin-1-yl]perhydrochinoxalin-1-yl}ethan-1-on und 2-(3,4-Dichlorphenyl)-1-{(4aRS,8SR, 8aSR)-8-[(3RS)-3-hydroxypyrrolidin-1-yl]perhydrochinoxalin-1-yl}ethan-1-on, das wie in Beispiel 2.1 bis 2.4 beschrieben hergestellt wurde.

Unter $N_2$ wurde das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on (120 mg, 0,29 mmol) in abolutem $CH_2Cl_2$ (18 ml) gelöst und Benzoylchlorid (23 mg, 0,29 mmol) zugetropft. Der Ansatz wurde 3 Stunden bei Raumtemperatur gerührt und anschließend im Vakuum eingedampft. Der Rückstand wurde durch Flashchromatographie gereinigt (Ø 2 cm, $CH_2Cl_2$ / MeOH / $NH_3$ 9:1:0,05, 1= 15 cm, V = 5 ml). Die Produktfraktionen wurden eingedampft und in $CH_2Cl_2$ aufgenommen. Die organische Phase wurde drei Mal mit HCl (1 N) ausgeschüttelt. Die wässrige Phase wurde mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Der Rückstand wurde durch flashchromatographisch gereinigt (Ø 2 cm, $CH_2Cl_2$ / MeOH / $NH_3$ 9,5:0,5:0,05, 1=15 cm, V = 5 ml). Die Produktfraktionen wurden eingedampft. Der Rückstand wurde durch präparative HPLC (MeOH / $H_2O$ / $Et_2NH$ 70:30:0,1) gereinigt wie nachfolgend beschrieben.

Hierzu wurde eine Pumpe L-7150, Autosampler L-7200, UV-Detektor L-7400, Interface D-7000 und Software HSM (alle Fa. Merck Hitachi) verwendet. Die Lösungen wurde individuell hergestellt oder es wurde ein Methanol / Wasser-Gemisch mit 0,1 % Diethylamin verwendet. Die Flussrate betrug 9,000 ml/min. Es wurde eine Säule Phenomenex Gemini 5μm C18 110A verwendet. Es wurde bei Raumtemperatur gearbeitet. Das Injektionsvolumen betrug 400 μl. Die Detektion erfolgte bei 225 nm. Der Rückstand wurde in MeOH (500 μl) gelöst. 400 μl wurden injiziert (80 % der gesamten Stoffmenge), die verbliebenen 100 μl mit MeOH auf 500 μl aufgefüllt. Aus dieser Lösung wurden in einem zweiten Lauf erneut 400 μl injiziert, so dass insgesamt 96 % der Probe in zwei Läufen chromatographisch gereinigt wurden.

Aus der Produktfraktion wurde MeOH verdampft, die wässrige Phase wurde drei Mal mit $CH_2Cl_2$ ausgeschüttelt, die vereinigten organischen Phasen wurden über $Na_2SO_4$ getrocknet und eingedampft. Es wurde ein farbloser Feststoff isoliert.

Beispiel 15

Herstellung von Methyl-{(*4aRS,8SR,8aRS*)-1-[2-(3,4-dichlorphenyl)acetyl]-8-[(*3SR*)-und (*3RS*)-3-hydroxypyrrolidin-1-yl]-perhydrochinoxalin-4-yl}carboxylat

Die Herstellung erfolgte ausgehend von dem Diastereomeren-Gemisch 2-(3,4-Dichlorphenyl)-1-{(4aRS,8SR, 8aSR)-8-[(3SR)-3-hydroxypyrrolidin-1-yl]perhydrochinoxalin-1-yl]ethan-1-on und 2-(3,4-Dichlorphenyl)-1-{(4aRS,8SR, 8aSR)-8-[(3RS)-3-hydroxypyrrolidin-1-yl]perhydrochinoxalin-1-yl)ethan-1-on, das wie in Beispiel 2.1 bis 2.4 beschrieben hergestellt wurde.

Unter $N_2$ wurde das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl} ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on (132 mg, 0,32 mmol) in abolutem $CH_2Cl_2$ (20 ml) gelöst und

Methylchlorformiat (30 mg, 0,32 mmol) zugetropft. Der Ansatz wurde 3 Stunden bei Raumtemperatur gerührt und anschließend im Vakuum eingedampft. Der Rückstand wurde durch Flashchromatographie gereinigt (Ø 2 cm, $CH_2Cl_2$ / MeOH / $NH_3$ 9:1:0,05, 1=15 cm, V = 5 ml). Die Produktfraktionen wurden eingedampft und der Rückstand durch präparative HPLC (wie unter Beispiel 14 beschrieben in MeOH / $H_2O$ / $Et_2NH$ 70:30:0,1) gereinigt. Aus der Produktfraktion wurde MeOH im Vakuum verdampft, die wässrige Phase wurde drei Mal mit $CH_2Cl_2$ ausgeschüttelt, die vereinigten organischen Phasen wurden über $Na_2SO_4$ getrocknet und eingedampft. Es wurde ein hellgelbes Harz isoliert.

Beispiel 16

Herstellung von 3-{(*4aRS,8SR,8aRS*)-1-[2-(3,4-Dichlorphenyl)acetyl]-8-[(*3SR*)- und (*3RS*)-3-hydroxypyrrolidin-1-yl]-perhydrochinoxalin-4-yl}-3-oxopropionsäure

Die Herstellung erfolgte ausgehend von dem Diastereomeren-Gemisch 2-(3,4-Dichlorphenyl)-1-{(4aRS,8SR, 8aSR)-8-[(3SR)-3-hydroxypyrrolidin-1-yl]perhydrochinoxalin-1-yl}ethan-1-on und 2-(3,4-Dichlorphenyl)-1-{(4aRS,8SR, 8aSR)-8-[(3RS)-3-hydroxypyrrolidin-1-yl]perhydrochinoxalin-1-yl}ethan-1-on, das wie in Beispiel 2.1 bis 2.4 beschrieben hergestellt wurde.

Unter $N_2$ wurde das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on (104 mg, 0,25 mmol) in abolutem $CH_2Cl_2$ (15 ml) gelöst und Malonsäuremonomethylesterchlorid (34 mg, 0,25 mmol) zugetropft. Der Ansatz wurde 3,5 Stunden bei Raumtemperatur gerührt und anschließend im Vakuum eingedampft. Der Rückstand wurde in MeOH (20 ml) aufgenommen und mit 2 N NaOH (2 ml) versetzt. Die Lösung wurde über Nacht gerührt und anschließend im Vakuum eingedampft. Der Rückstand wurde zweimal flashchromatographisch gereinigt (1.Ø 2 cm, $CH_2Cl_2$ / MeOH /$NH_3$ 8:2:0,1, 1= 15 cm, V = 5 ml; 2. Ø 1 cm, $CH_2Cl_2$ / MeOH/$NH_3$ 8:2:0,2, 1= 14 cm, V = 3 ml). Der Rückstand wurde durch präparative HPLC (wie unter Beispiel 14 beschrieben in MeOH / $H_2O$ / $Et_2NH$ 40:60:0,1) gereinigt. Aus der Produktfraktion wurde MeOH verdampft, die wässrige Phase wurde drei Mal mit $CH_2Cl_2$ ausgeschüttelt, die vereinigten organischen Phasen wurden über $Na_2SO_4$ getrocknet und eingedampft. Es wurde ein farbloses Öl isoliert.

Beispiel 17

Herstellung von Methyl-3-{(*4aRS,8SR,8aRS*)-1-[2-(3,4-Dichlorphenyl)acetyl]-8-[(*3SR*)-und (*3RS*)-3-hydroxypyrrolidin-1-yl]-perhydrochinoxalin-4-yl}-3-oxopropionat

Die Herstellung erfolgte ausgehend von dem Diastereomeren-Gemisch 2-(3,4-Dichlorphenyl)-1-{(4aRS,8SR, 8aSR)-8-[(3SR)-3-hydroxypyrrolidin-1-yl]perhydrochinoxalin-1-yl}ethan-1-on und 2-(3,4-Dichlorphenyl)-1-{(4aRS,8SR, 8aSR)-8-[(3RS)-3-hydroxypyrrolidin-1-yl]perhydrochinoxalin-1-yl}ethan-1-on, das wie in Beispiel 2.1 bis 2.4 beschrieben hergestellt wurde.

Unter $N_2$ wurde das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on (204 mg, 0,49 mmol) in abolutem $CH_2Cl_2$ (30 ml) gelöst und Malonsäuremonomethylesterchlorid (67 mg, 0,49 mmol) zugetropft. Der Ansatz wurde 3 Stunden lang bei Raumtemperatur gerührt und anschließend im Vakuum eingedampft. Der Rückstand wurde durch Flashchromatographie gereinigt (Ø 2 cm, $CH_2Cl_2$ /MeOH / $NH_3$ 9:1:0,05, 1= 15 cm, V = 5 ml). Es wurde ein gelbliches Harz gewonnen.

Beispiel 18

Herstellung von 2-(3,4-Dichlorphenyl)-1-[(*4aRS,8SR,8aRS*)-4-methyl-8-(pyrrolidin-1-yl)-perhydrochinoxalin-1-yl]ethan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Formalin (37 %, 223 mg, 2,7 mmol) wurde in 5 ml MeOH gelöst und mit $NaBH_3CN$ (17,2 mg, 0,27 mmol) versetzt. Der pH-Wert wurde mit konzentrierter Essigsäure auf 5 eingestellt. Anschließend wurde 2-(3,4-Dichlorphenyl)-1-[(4aRS,8SR,8aRS)-8-(pyrrolidin-1-yl)-perhydrochinoxalin-1-yl]-ethan-1-on (109 mg, 0,27 mmol), gelöst in MeOH (15 ml), zum Ansatz gegeben und die Mischung 1,5 Stunden lang gerührt. Nach Zugabe von gesättigter $Na_2CO_3$-Lösung (12 ml) wurde 15 Minuten bei Raumtemperatur gerührt. Der entstandene Niederschlag wurde abfiltriert und MeOH bei 100 mbar aus dem Filtrat abgedampft. Die wässrige Phase wurde fünfmal mit $CH_2Cl_2$ ausgeschüttelt, die vereinigten organischen Phasen wurden über $Na_2SO_4$ getrocknet und im Vakuum eingedampft. Der Rückstand wurde flashchromatographisch gereinigt (Ø 2 cm, $CH_2Cl_2$ / MeOH / $NH_3$ 9,5:0,5:0,05, 1 = 16 cm, V = 5 ml). Es wurde ein gelbliches Harz isoliert.

Beispiel 19

Herstellung von 1-[(*4aRS, 8SR,8aRS*)-4-Butyl-8-(pyrrolidin-1-yl)perhydrochinoxalin-1-yl]-2-(3,4-dichlorphenyl)-ethan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Butyraldehyd (93 mg, 1,3 mmol) wurde in 5 ml MeOH gelöst und mit $NaBH_3CN$ (82 mg, 1,3 mmol) versetzt. Der pH-Wert wurde mit konzentrierter Essigsäure auf 5 eingestellt. Anschließend wurde 2-(3,4-Dichlorphenyl)-1-[(4aRS, 8SR,8aRS)-8-(pyrrolidin-1-yl)-perhydrochinoxalin-1-yl]-ethan-1-on (101 mg, 0,25 mmol), gelöst in MeOH (15 ml), zum Ansatz gegeben und die Mischung über Nacht bei Raumtemperatur. Nach Zugabe von gesättigter $Na_2CO_3$-Lösung (15 ml) wurde der Ansatz 15 Minuten bei Raumtemperatur gerührt. Der entstandene Niederschlag wurde abfiltriert. Die wässrige Phase des Filtrats wurde dreimal mit $CH_2Cl_2$ extrahiert, die vereinigten organischen Phasen wurden über $Na_2SO_4$ getrocknet und im Vakuum eingedampft. Der Rückstand wurde flashchromatographisch gereinigt (Ø 2 cm, $CH_2Cl_2$ / MeOH / $NH_3$ 9,5:0,5:0,1, 1 = 15 cm, V = 5 ml). Es wurde ein farbloses Harz isoliert.

Beispiel 20

Herstellung von 2-(3,4-Dichlorphenyl)-1-[(*4aRS,8SR,8aRS*)-4-(4-Methoxybenzyl)-8-(pyrrolidin-1-yl)-perhydrochinoxalin-1-yl]ethan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Anisaldehyd (361 mg, 2,6 mmol) wurde in 5 ml MeOH gelöst und mit $NaBH_3CN$ (170 mg, 2,6 mmol) versetzt. Der pH-Wert wurde mit konzentrierter Essigsäure auf 5 eingestellt. Anschließend wurde 2-(3,4-Dichlorphenyl)-1-[(4aRS, 8SR,8aRS)-8-(pyrrolidin-1-yl)-perhydrochinoxalin-1-yl]-ethan-1-on (105 mg, 0,26 mmol), gelöst in MeOH (15 ml), zum Ansatz gegeben und die Mischung über Nacht gerührt. Nach Zugabe von gesättigter $Na_2CO_3$-Lösung (15 ml) wurde der Ansatz 15 Minuten bei Raumtemperatur gerührt. Der entstandene Niederschlag wurde abfiltriert. Die wässrige Phase des Filtrats wurde drei Mal mit $CH_2Cl_2$ gewaschen, die vereinigten organischen Phasen wurden über $Na_2SO_4$ getrocknet und im Vakuum eingedampft. Der Rückstand wurde flashchromatographisch gereinigt (0 2 cm, $CH_2Cl_2$ / MeOH / $NH_3$ 9,5:0,5:0,1, 1 = 15 cm, V = 5 ml). Die das Produkt enthaltenen Fraktionen wurden eingedampft, der Rückstand in $CH_2Cl_2$ aufgenommen und die Lösung drei Mal mit HCl (1 N) ausgeschüttelt. Anschließend wurde die wässrige Phase mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Es wurde ein gelbliches Harz isoliert.

Beispiel 21

Herstellung von 2-(3,4-Dichlorphenyl)-1-{(*4aRS,8SR,8aRS*)-4-[(pyridin-2-yl)methyl]-8-(pyrrolidin-1-yl)perhydrochinoxalin-1-yl}ethan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

Pyridin-2-carbaldehyd (268 mg, 2,5 mmol) wurde in MeOH (5 ml) gelöst und mit $NaBH_3CN$ (157 mg, 2,5 mmol) versetzt. Der pH-Wert wurde mit konzentrierter Essigsäure auf 5 eingestellt. Anschließend wurde 2-(3,4-Dichlorphenyl)-1-[(4aRS,8SR,8aRS)-8-(pyrrolidin-1-yl)-perhydrochinoxalin-1-yl]-ethan-1-on (98 mg, 0,25 mmol), gelöst in MeOH (15 ml), zum Ansatz gegeben und die Mischung über Nacht bei Raumtemperatur gerührt. Nach Zugabe von gesättigter $Na_2CO_3$-Lösung (15 ml) wurde 15 Minuten bei Raumtemperatur gerührt. Der entstandene Niederschlag wurde abfitriert. Die wässrige Phase des Filtrats wurde fünf Mal mit $CH_2Cl_2$ extrahiert, die vereinigten organischen Phasen wurden über $Na_2SO_4$ getrocknet und im Vakuum eingedampft. Der Rückstand wurde vier Mal flashchromatographisch gereinigt (jeweils Ø 2 cm, 1= 15 cm, V = 5 ml; 1. $CH_2Cl_2$ / MeOH / $NH_3$ 9,5:0,5:0,1, 2. $CH_2Cl_2$ / MeOH / $NH_3$ 9,5:0,5:0,1, 3. $CH_2Cl_2$ / MeOH/$NH_3$ 9,75:0,25:0,15, 4. $CH_2Cl_2$ /MeOH / $NH_3$ 9,5:0,5:0,15). Anschließend wurde das Rohprodukt durch präparative HPLC (wie unter Beispiel 14 beschrieben in MeOH / $H_2O$ / $Et_2NH$ 80:20:0,1) gereinigt. Aus der Produktfraktion wurde MeOH verdampft, die wässrige Phase wurde drei Mal mit $CH_2Cl_2$ ausgeschüttelt, die vereinigten organischen Phasen wurden über $Na_2SO_4$ getrocknet und eingedampft. Es wurde ein gelbliches Harz isoliert.

Beispiel 22

Herstellung von 2-(3,4-Dichlorphenyl)-1-{(*4aRS,8SR,8aRS*)-4-[(pyridin-3-yl)methyl]-8-(pyrrolidin-1-yl)perhydrochinoxalin-1-yl}ethan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

2-(3,4-Dichlorphenyl)-1-[(4aRS,8SR,8aRS)-8-(pyrrolidin-1-yl)-perhydrochinoxalin-1-yl]-ethan-1-on (120 mg, 0,30 mmol) wurde in abolutem $CH_2Cl_2$ (10 ml) gelöst und mit Nicotinaldehyd (65 mg, 0,61 mmol), $NaBH(OAc)_3$ (128 mg, 0,61 mmol) und Eisessig (36 mg, 0,61 mmol) versetzt. Das Gemisch wurde bei Raumtemperatur gerührt. Nach 21 Stunden wurden nochmals gleiche Mengen an Nicotinaldehyd, $NaBH(OAc)_3$ und Eisessig zugegeben und der Ansatz 3,5 Stunden gerührt. Anschließend wurde filtriert und die organische Phase drei Mal mit HCl (1N) ausgeschüttelt. Die wässrige Phase wurde mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Der Rückstand wurde durch präparative HPLC (wie unter Beispiel 14 beschrieben in MeOH / $H_2O$ / $Et_2NH$ 80:20:0,1) gereinigt. Aus der Produktfraktion wurde MeOH verdampft, die wässrige Phase wurde drei Mal mit $CH_2Cl_2$ ausgeschüttelt, die vereinigten organischen Phasen wurden über $Na_2SO_4$ getrocknet und eingedampft. Es wurde ein gelbliches Harz isoliert.

Beispiel 23

Herstellung von 2-(3,4-Dichlorphenyl)-1-{(*4aRS, 8SR, 8aRS*)-4-[(1H-imidazol-5-yl)methyl]-8-(pyrrolidin-1-yl)perhydrochinoxalin-1-yl}ethan-1-on

Die Herstellung erfolgte ausgehend von 2-(3,4-Dichlorphenyl)-1-[(4aRS,8SR,8aRS)-8-(pyrrolidin-1-yl)-perhydrochinoxalin-1-yl]-ethan-1-on, das wie in Beispiel 1.1 bis 1.9 beschrieben hergestellt wurde.

2-(3,4-Dichlorphenyl)-1-[(4aRS,8SR,8aRS)-8-(pyrrolidin-1-yl)-perhydrochinoxalin-1-yl]-ethan-1-on (134 mg, 0,34 mmol) wurde in abolutem $CH_2Cl_2$ (10 ml) gelöst und mit 1*H*-Imidazol-5-carbaldehyd (65 mg, 0,67 mmol), $NaBH(OAc)_3$ (143 mg, 0,67 mmol) und Eisessig (41 mg, 0,67 mmol) versetzt. Das Gemisch wurde bei Raumtemperatur 2,5 Stunden gerührt. Anschließend wurde filtriert und die organische Phase drei Mal mit HCl (1 N) ausgeschüttelt. Die wässrige Phase wurde mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Der Rückstand wurde durch präparative HPLC (wie unter Beispiel 14 beschrieben in MeOH / $H_2O$ / $Et_2NH$ 80:20:0,1) gereinigt. Aus der Produktfraktion wurde MeOH verdampft, die wässrige Phase wurde drei Mal mit $CH_2Cl_2$ ausgeschüttelt, die vereinigten organischen Phasen wurden über $Na_2SO_4$ getrocknet und eingedampft. Es wurde ein farbloser Feststoff isoliert.

Beispiel 24

Herstellung von 2-(3,4-Dichlorphenyl)-1-{(*4aRS,8SR,8aRS*)-4-methyl-8-[(*3SR*)- und (*3RS*)-3-hydroxypyrrolidin-1-yl]-perhydrochinoxalin-1-yl}ethan-1-on

Die Herstellung erfolgte ausgehend von dem Diastereomeren-Gemisch 2-(3,4-Dichlorphenyl)-1-{(4aRS,8SR, 8aSR)-8-[(3SR)-3-hydroxypyrrolidin-1-yl]perhydrochinoxalin-1-yl}ethan-1-on und 2-(3,4-Dichlorphenyl)-1-{(4aRS,BSR, 8aSR)-8-[(3RS)-3-hydroxypyrrolidin-1-yl]perhydrochinoxalin-1-yl}ethan-1-on, das wie in Beispiel 2.1 bis 2.4 beschrieben hergestellt wurde.

Formalin (37 %, 170 mg, 2,1 mmol) wurde in 5 ml MeOH gelöst und mit $NaBH_3CN$ (132 mg, 2,1 mmol) versetzt. Der pH-Wert wurde mit konzentrierter Essigsäure auf 5 eingestellt. Anschließend wurde das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl} ethan-1-on und 2-(3,4-Dichlorphenyl)-1-{(4aRS,8SR,8aSR)-8-[(3RS)-3-hydroxypyrrolidin-1-yl]perhydrochinoxalin-1-yl)ethan-1-on (86 mg, 0.21 mmol), gelöst in MeOH (15 ml), zu gegeben und der Ansatz 2 Stunden lang gerührt. Nach Zugabe von gesättigter $Na_2CO_3$-Lösung (15 ml) wurde 15 Minuten bei Raumtemperatur gerührt. Der entstandene Niederschlag wurde abfiltriert. MeOH wurde bei 100 mbar aus dem Filtrat abgedampft. Die wässrige Phase wurde fünfmal mit $CH_2Cl_2$ ausgeschüttelt, die vereinigten organischen Phasen wurden über $Na_2SO_4$ getrocknet und im Vakuum eingedampft. Der Rückstand wurde flashchromatographisch gereinigt (Ø 2 cm, $CH_2Cl_2$ / MeOH / $NH_3$ 9:1:0.1,1= 16 cm, V = 5 ml). Es wurde ein gelbliches Harz isoliert.

Beispiel 25

Herstellung von 2-(3,4-Dichlorphenyl)-1-{(*4aRS,8SR,8aSR*)-4-[(pyridin-3-yl)methyl]-8-[(*3SR*)- und (*3RS*)-3-hydroxypyrrolidin-1-yl]perhydrochinoxalin-1-yl}ethan-1-on

Die Herstellung erfolgte ausgehend von dem Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on, das wie in Beispiel 2.1 bis 2.4 beschrieben hergestellt wurde.

Eine Lösung des Diastereomeren-Gemischs 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on (103 mg, 0,25 mmol) in MeOH (15 ml) wurde tropfenweise zu einer Lösung aus Nicotinaldehyd (53 mg, 0,49 mmol) und $NaBH_3CN$ (157 mg, 2.5 mmol) in MeOH (5 ml) gegeben. Der pH-Wert wurde mit konzentrierter Essigsäure auf pH 5 eingestellt. Der Ansatz wurde 2 Stunden gerührt. Nach Zugabe von gesättigter $Na_2CO_3$-Lösung (15 ml) wurde 15 Minuten bei Raumtemperatur gerührt. Der entstandene Niederschlag wurde abfiltriert und das Filtrat im Vakuum eingedampft. Der Rückstand wurde flashchromatographisch gereinigt (Ø 2 cm, $CH_2Cl_2$ / MeOH / $NH_3$ 9,5:0,5:1, 1= 16 cm, V = 3 ml). Die das Produkt enthaltenen Fraktionen wurden eingedampft.

Der Rückstand wurde durch präparative HPLC (wie unter Beispiel 14 beschrieben in MeOH /$H_2O$ / $Et_2NH$ 70: 30:0,1) gereinigt. Aus der Produktfraktion wurde MeOH verdampft, die wässrige Phase wurde drei Mal mit $CH_2Cl_2$ ausgeschüttelt, die vereinigten organischen Phasen wurden über $Na_2SO_4$ getrocknet und eingedampft. Es wurde ein gelbliches Harz isoliert.

Beispiel 26

Herstellung von 2-(3,4-Dichlorphenyl)-1-{(*4aRS,8SR,8aSR*)-4-[(1*H*-Imidazol-5-yl)methyl]-8-[(*3SR*)- und (*3RS*)-3-hydroxypyrrolidin-1-yl]perhydrochinoxalin-1-yl}ethan-1-on

Die Herstellung erfolgte ausgehend von dem Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on, das wie in Beispiel 2.1 bis 2.4 beschrieben hergestellt wurde.

1*H*-Imidazol-5-carbaldehyd (262 mg, 2,7 mmol) wurde in MeOH (5 ml) gelöst und mit $NaBH_3CN$ (172 mg, 2,7 mmol) versetzt. Der pH-Wert wurde mit konzentrierter Essigsäure auf 5 eingestellt. Anschließend wurde das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on (113 mg, 0,27 mmol), gelöst in MeOH (15 ml), zum Ansatz gegeben und 5 Stunden gerührt. Nach Zugabe von gesättigter $Na_2CO_3$-Lösung (15 ml) wurde 15 Minuten bei Raumtemperatur gerührt. Der entstandene Niederschlag wurde abfiltriert. Das Filtrat wurde drei Mal mit $CH_2Cl_2$ gewaschen, die vereinigten organischen Phasen wurden über $Na_2SO_4$ getrocknet und im Vakuum eingedampft. Der Rückstand wurde zweimal flashchromatographisch gereinigt (Ø 2 cm, $CH_2Cl_2$ / MeOH / $NH_3$ 9:1:0.1, 1=15 cm, V = 5 ml). Die das Produkt enthaltenen Fraktionen wurden eingedampft. Der Rückstand wurde in $CH_2Cl_2$ aufgenommen und drei Mal mit HCl (1 N) ausgeschüttelt. Die wässrige Phase wurde mit NaOH (2 N) auf pH 8 gebracht und drei Mal mit $CH_2Cl_2$ ausgeschüttelt. Die organischen Phasen wurden über $Na_2SO_4$ getrocknet und im Vakuum eingedampft. Es wurde ein gelbliches Harz isoliert.

Beispiel 27

Herstellung von 〈(*3SR*)- und (*3RS*)-1-{(*4aRS,5RS,8aSR*)-1-Benzyl-4-[2-(3,4-dichlorphenyl)acetyl]-perhydrochinoxalin-5-yl}pyrrolidin-3-yl〉-2-(3,4-dichlorphenyl)acetat

Die Herstellung erfolgte ausgehend von (4aRS,5SR,8aRS)-1-Benyzl-5-((3SR)- und (*3RS*)-hydroxypyrrolidin-1 -yl)-perhydrochinoxalin, das wie in Beispiel 2.1 bis 2.2 beschrieben hergestellt wurde.

(4aRS,5SR,8aRS)-1-Benyzl-5-((3SR)- und (*3RS*)-hydroxypyrrolidin-1-yl)-perhydrochinoxalin (0,70 g, 2,2 mmol) wurde in abolutem $CH_2Cl_2$ (100 ml) gelöst. Es wurde 2-(3,4-Dichlorphenyl)acetylchlorid (1,1 g, 4,9 mmol) hinzugetropft und der Ansatz bei Raumtemperatur gerührt. Nach 4 Stunden wurde 2 N NaOH (4,5 ml) zugegeben und über Nacht kräftig gerührt. Die organische Phase wurde abgetrennt und zwei Mal mit 2 N NaOH gewaschen. Anschließend wurden die organische Phase über $Na_2SO_4$ getrocknet und im Vakuum eingedampft. Der Rückstand wurde zwei Mal flashchromatographisch (0 2 cm, EE /$Et_2NH$ 10:0,1, 1 = 15 cm, V = 5 ml) gereinigt. Es wurde ein hellgelbes Harz isoliert.

Beispiel 28

Untersuchungen zur Schmerzhemmung in vivo in der Maus

Die antinociceptive Wirksamkeit wurde im Phenylchinon-induzierten Writhing-Test an der Maus untersucht, wie in Hendershot, L. C.; Forsaith, J. J. Pharmacol. Exp. Ther. 1959, 125, 237-240 beschrieben.

Dazu wurden männliche NMRI-Mäuse (Charles River, Deutschland) mit einem Gewicht von 25 g bis 30 g verwendet. Gruppen von 10 Tieren wurde jeweils das Diastereomeren-Gemisch 2-(3,4-Dichlorphenyl)-1-{(4aRS,8SR, 8aSR)-8-[(3SR)-3-hydroxypyrrolidin-1-yl]perhydrochinoxalin-1-yl}ethan-1-on und 2-(3,4-Dichlorphenyl)-1-{(4aRS,8SR, 8aSR)-8-[(3RS)-3-hydroxypyrrolidin-1-yl]perhydrochinoxalin-1-yl}ethan-1-on in einer Konzentration von 3,16 mg/kg, 10 mg/kg oder 21,5 mg/kg gelöst in PEG 200 (Polyethylenglycol, Merck Schuhardt OHG) oder die Verbindung 2-(3,4-Dichlorphenyl)-1-[(4aRS,8SR,8aRS)-8-(pyrrolidin-1-yl)-perhydrochinoxalin-1-yl]-ethan-1-on in einer Konzentration von 10 mg/kg gelöst in PEG 200 intravenös appliziert. Nach 10 Minuten wurde 0,3 ml einer 0,02 %igen wässrigen Lösung von Phenylchinon (Phenyl-p-benzochinon, Fa. Sigma, Deisenhofen) intraperitoneal appliziert. Die Phenylchinon-Lösung wurde unter Zusatz von 5 % Ethanol hergestellt und im Wasserbad bei 45 C aufbewahrt.

Anschließend wurde die Anzahl an schmerz-induzierten Streckbewegungen, so genannten Writhing-Reaktionen (Anzahl n), über 10 Minuten gezählt. Als so genannte Writhingreaktionen wird das Durchdrücken des Körpers mit Abstrecken der Hinterextremitäten bezeichnet. Ist eine Substanz analgetisch wirksam, nimmt die Anzahl der Streckbewegungen im Vergleich zu einer Kontrollgruppe, die die Testsubstanz nicht erhalten hat, ab.

Hierzu wurden die Tiere einzeln in Beobachtungskäfige gesetzt. Mittels eines Drucktastenzählers wurde die Anzahl der Schmerz-induzierten Streckbewegungen 5-20 Minuten nach der Phenylchinon-Gabe während 15 Minuten ausgezählt. Als Kontrolle wurden Tiere mitgeführt, die PEG 200-Vehikellösung (intravenös, i. v.) und Phenylchinon (intraperitoneal, i. p.) erhielten.

Die prozentuale Hemmung der Writhingreaktionen durch das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-onoder 2-(3,4-Dichlorphenyl)-1-[(4aRS,8SR,8aRS)-8-(pyrrolidin-1-yl)-perhydrochinoxalin-1-yl]-ethan-1-on wurde nach folgender Formel (d) berechnet:

$$\text{Inhibition} = 100\% - \frac{\text{n (behandelte Tiere)}}{\text{n (Kontrolle)}} \cdot 100\% \qquad \text{(d)}$$

Es konnte festgestellt werden, dass bei einer Dosis von 10 mg/kg der Verbindung 2-(3,4-Dichlorphenyl)-1-[(4aRS,8SR,8aRS)-8-(pyrrolidin-1-yl)-perhydrochinoxalin-1-yl]-ethan-1-on eine Inhibition von etwa 9 % erzielt wurden. Nennenswerte Nebenwirkungen wurden nicht festgestellt.

Weiterhin konnte festgestellt werden, dass das Diastereomeren-Gemisch 2-(3,4-Dichlorphenyl)-1-{(4aRS,8SR, 8aSR)-8-[(3SR)-3-hydroxypyrrolidin-1-yl]perhydrochinoxalin-1-yl}ethan-1-on und 2-(3,4-Dichlorphenyl)-1-{(4aRS,8SR, 8aSR)-8-[(3RS)-3-hydroxypyrrolidin-1-yl]perhydrochinoxalin-1-yl}ethan-1-on bei einer Konzentration von 3,16 mg/kg 5 % der Writhing-Bewegungen inhibierte, bei einer Konzentration von 10 mg/kg 40 %, und bei einer Konzentration von 21,5 mg/kg 97 %.

Somit konnte eine analgetische Wirkung des Diastereomeren-Gemischs 2-(3,4-Dichlorphenyl)-1-{(4aRS,8SR, 8aSR)-8-[(3SR)-3-hydroxypyrrolidin-1-yl]perhydrochinoxalin-1-yl}ethan-1-on und 2-(3,4-Dichlorphenyl)-1-{(4aRS,8SR, 8aSR)-8-[(3RS)-3-hydroxypyrrolidin-1-yl]perhydrochinoxalin-1-yl}ethan-1-on und der Verbindung 2-(3,4-Dichlorphenyl)-1-[(4aRS,8SR,8aRS)-8-(pyrrolidin-1-yl)-perhydrochinoxalin-1-yl]-ethan-1-on festgestellt werden.

Beispiel 29

Untersuchungen zur Schmerzhemmung im visceralen Entzündungsschmerzmodell

Bei diesem Tierversuch wird eine durch Senföl induzierte nicht-neurogene Kolitis (Entzündung) in der Maus ausgelöst. Verschiedene Versuchsgruppen innerhalb der Experimente geben Aufschluss über periphere und zentral vermittelte Analgesie der untersuchten Verbindung 2-(3,4-Dichlorphenyl)-1-[(4aRS,8SR,8aRS)-8-(pyrrolidin-1-yl)-perhydrochinoxalin-1-yl]-ethan-1-on und des Diastereomeren-Gemischs 2-(3,4-Dichlorphenyl)-1-{(4aRS,BSR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl} ethan-1-on. Wenn nicht abweichend angegeben, wurden die Versuche durchgeführt wie in Christoph, T.; Kögel, B.; Schiene, K.; Méen, M.; De Vry, J.; Friedrichs, E. Eur. J. Pharmacol.

2005, 507, 87-98, beschrieben.

Durch das Verhalten der Tiere zwei bis zwölf Minuten nach rektaler Senföl-Gabe wurde hierbei der viscerale Spontanschmerz, beispielsweise durch Hüpfen, Zucken oder Vokalisation, quantitativ erfasst in Form eines Schmerzscore. Nach 20 bis 40 Minuten wurde die Bauchdecke der Tiere mechanisch stimuliert. Zentral vermittelte Allodynie und Hyperalgesie wurden durch von Frey Filamente (1 Nm bzw. 16 Nm) ermittelt.

Die Größe der Versuchsgruppen betrug n = 7 Mäuse. Einer Kontrollgruppe von Tieren wurde Polyethylenglycol, PEG200 (Merck Schuhardt OHG), rektal appliziert, bei einer zweiten Gruppe wurde eine Kolitis durch rektal verabreichtes Senföl ausgelöst. Weiteren Gruppen wurde Senföl (rektal) und das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on oder 2-(3,4-Dichlorphenyl)-1-[(4aRS,8SR,8aRS)-8-(pyrrolidin-1-yl)-perhydrochinoxalin-1-yl]-ethan-1-on, gelöst in PEG 200, intravenös verabreicht. Hierbei erhielt eine Versuchsgruppe die Verbindung 2-(3,4-Dichlorphenyl)-1-[(4aRS,8SR,8aRS)-8-(pyrrolidin-1-yl)-perhydrochinoxalin-1-yl]-ethan-1-on in einer Konzentration von 21,5 mg/kg verabreicht. Weitere Versuchsgruppen erhielten das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on in Konzentrationen von 1,0 mg/kg, 3,16 mg/kg und 10 mg/kg verabreicht. Als Applikationsvolumen wurden 10 ml/kg eingesetzt.

Eine verminderte Wirkung bei der Gruppe, die Senföl und das Diastereomeren-Gemisch oder 2-(3,4-Dichlorphenyl)-1-[(4aRS,8SR,8aRS)-8-(pyrrolidin-1-yl)-perhydrochinoxalin-1-yl]-ethan-1-on verabreicht erhielten, ist ein Hinweis auf eine analgetische Wirkung der Verbindung.

Die Versuchsdurchführung im Einzelnen:
Männliche NMRI Mäuse (Charles River, Deutschland) mit einem Körpergewicht von 20 g bis 35 g wurden auf einem Gitterrost in Plexiglaskäfigen (Grundfläche 14,5 x 14,5 cm, Höhe 10 cm) für etwa 30 Minuten habituiert.

Das Verhalten der Mäuse auf zehnmalige mechanische Stimulierung mittels von Frey Filamenten (Grünenthal GmbH) mit einer Kraft von 1 mN, 4 mN, 8 mN, 16 mN und 32 mN auf die Bauchdecke wurde als Vorwert erhoben. Das Verhalten wurde entweder über die Summe der Anzahl der nozifensiven Reaktionen oder über die Qualität dieser nozifensiven Reaktionen und deren Gewichtung durch Multiplikation der Anzahl der Reaktionen mit dem zugehörigen Faktor und anschließende Summenbildung analysiert. Die Faktoren waren dabei die Folgenden: Faktor 1: leichtes Anheben des Abdomens, Lecken an der Reizstelle, Weggehen; Faktor 2: Wegstrecken der Hinterpfoten, leichtes Weghüpfen, Zucken der Hinterpfoten, ruckartiges, starkes Lecken der Reizstelle; Faktor 3: Wegspringen, Vokalisation.

Anschließend erfolgte in den Versuchsgruppen die intravenöse Applikation des Diastereomeren-Gemischs 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on, der Verbindung 2-(3,4-Dichlorphenyl)-1-[(4aRS,8SR,8aRS)-8-(pyrrolidin-1-yl)-perhydrochinoxalin-1-yl]-ethan-1-on oder 10 ml/kg Lösemittel PEG 200.

Nach 5 Minuten wurde eine rektale Gabe von 50 μl einer 3,5 %igen Lösung von Senföl in PEG200 verabreicht.

Eine Kontrollgruppe von Tieren erhielt eine rektale Applikation von 50 μl PEG200.

2 bis 12 Minuten nach Senfölgabe zeigten die Tiere ein spontanes viszerales Schmerzverhalten, welches beobachtet wurde. Die Anzahl der Reaktionen wurde mit dem zugehörigen Faktor - Faktor 1: leichtes Anheben des Abdomens, Lecken an der Reizstelle, Weggehen; Faktor 2: Wegstrecken der Hinterpfoten, leichtes Weghüpfen, Zucken der Hinterpfoten, ruckartiges, starkes Lecken der Reizstelle; Faktor 3: Wegspringen - multipliziert und anschließend die Summe gebildet, welche den spontanen viszeralen Schmerzscore darstellt.

20 bis 40 Minuten nach Senfölgabe wurde erneut das Verhalten der Tiere auf zehnmalige mechanische Stimulierung mittels von Frey Filamenten mit 1 mN, 4 mN, 8 mN, 16 mN und 32 mN auf die Bauchdecke beobachtet und wie oben beschrieben quantifiziert.

Die übertragene mechanische Allodynie wurde hierbei aus der Summe der Reaktionen auf die Stimulierung mit dem von Frey Filament der Stärke 1 mN bestimmt. Die übertragene mechanische Hyperalgesie wurde aus der Summe der gewichteten, Reaktionen auf die Stimulierung mit dem von Frey Filament der Stärke 16 mN bestimmt.

Die Wirkung des Diastereomeren-Gemischs 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on und der Verbindung 2-(3,4-Dichlorphenyl)-1-[(4aRS,8SR,8aRS)-8-(pyrrolidin-1-yl)-perhydrochinoxalin-1-yl]-ethan-1-on wurde im Vergleich zur Kontrollgruppe beschrieben durch 1. Hemmung des spontanen viszeralen Schmerzverhaltens, 2. Hemmung der übertragenen mechanischen Allodynie und 3. Hemmung der übertragenen mechanischen Hyperalgesie.

Statistische Auswertungen erfolgten mit dem Programm SYSTAT, Version 11 für Windows.

Es konnte festgestellt werden, dass die Verbindung 2-(3,4-Dichlorphenyl)-1-[(4aRS,8SR,8aRS)-8-(pyrrolidin-1-yl)-perhydrochinoxalin-1-yl]-ethan-1-on im Spontanschmerz-Versuch eine sehr gute Wirkung zeigte. Der Schmerzscore entsprach dem der Kontrollgruppe. Dies weist auf eine gute periphere Schmerzhemmung hin.

In den Modellen der zentral vermittelten Schmerzarten, der Allodynie und Hyperalgesie, wurde keine Wirkung der Verbindung 2-(3,4-Dichlorphenyl)-1-[(4aRS,8SR,8aRS)-8-(pyrrolidin-1-yl)-perhydrochinoxalin-1-yl]-ethan-1-on festgestellt.

Es konnte weiter festgestellt werden, dass das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on im Spontanschmerz-Versuch bei den Applikationsformen mit zunehmender Dosis einen analgetischen Effekt zeigte. Bei 10 mg/kg war nahezu Schmerzfreiheit erreicht.

In den Modellen der zentral vermittelten Schmerzarten wurde keine signifikante Reduktion der Schmerzen festzustellen.

Dies zeigt, dass das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on und die Verbindung 2-(3,4-Dichlorphenyl)-1-[(4aRS,8SR,8aRS)-8-(pyrrolidin-1-yl)-perhydrochinoxalin-1-yl]-ethan-1-on eine gute periphere analgetische Wirkung aufweisen.

Beispiel 30

Bestimmung der κ-Rezeptor-Affinität

Die Affinität zu Rezeptoren lässt sich in vitro durch Rezeptor-Bindungsstudien bestimmen. Hierbei kommen Membranpräparationen, ein radioaktiv markierter Radioligand, der eine hohe Affinität und Selektivität zum Rezeptor aufweist, und die Testsubstanz, deren Affinität zu bestimmen ist, zum Einsatz.

Die Inkubation der Rezeptorpräparation mit einem Liganden L führt zu einem Gleichgewicht zwischen unbesetztem Rezeptor R und freiem Liganden L auf der einen Seite und dem Rezeptor-Ligand-Komplex RL auf der anderen Seite.

Daraus ergibt sich die Dissoziationskonstante $K_d$ gemäß folgender Gleichung (a):

$$K_d = \frac{k_2}{k_1} = \frac{[R]\cdot[L]}{[RL]} \quad (a)$$

Um die Affinität einer Testsubstanz zu bestimmen, wurden Kompetitionsexperimente durchgeführt. Dabei wurde das Rezeptormaterial mit dem Radioliganden und der zu untersuchenden Testsubstanz versetzt. Die beiden Liganden traten nun zueinander in Konkurrenz um die Bindungsstellen am Rezeptor. Nach Einstellung des Gleichgewichts wurde der ungebundene Radioligand abgetrennt und die Radioaktivität des Rezeptor-Ligand-Komplexes vermessen. Daraus lassen sich Rückschlüsse auf das Verhältnis von gebundenem Radioligand zu gebundener Testsubstanz ziehen und damit eine Aussage über die Affinität der Testsubstanz zum Rezeptor treffen. Die Radioaktivität wurde mit einem Szintillationszähler mit Hilfe eines Szintillationscocktails, der durch den tritiummarkierten Liganden Photonen emittiert, indirekt gemessen.

Die Messung erfolgte bei konstanter Rezeptor- und Radioligand-Konzentration, die Konzentration an zu bestimmender Testsubstanz wurde variiert. Zusätzlich wurden die Werte für die unspezifische und die maximale Bindung bestimmt. Die unspezifische Bindung des Radioliganden wurde durch Inkubation der Rezeptorpräparation mit Radioligand und einem großen Überschuss eines selektiven, nicht radioaktiv markierten Liganden bestimmt, wodurch die spezifischen Bindungsstellen des Rezeptors mit unmarkiertem Liganden abgesättigt wurden. Die gemessene Radioaktivität resultierte dann aus unspezifischer Bindung des Radioliganden an Membran, Filter etc. Die maximale Bindung wurde ermittelt, indem das Rezeptormaterial mit Radioligand ohne Testsubstanzen inkubiert wurde. Die prozentuale Restbindung des Radioliganden lässt sich berechnen nach Gleichung (b):

$$\%\ \text{Restbindung} = \frac{[\text{gemessene Bindung}] - [\text{unspezifische Bindung}]}{[\text{maximale Bindung}] - [\text{unspezifische Bindung}]} \cdot 100\% \quad (b)$$

Wird die Restbindung gegen den dekadischen Logarithmus der Substanzkonzentration grafisch aufgetragen, entsteht eine sigmoide Kurve. Aus ihr wurde diejenige Konzentration an Testsubstanz ermittelt, bei der die Bindung des Radioliganden an den Rezeptor um 50 % vermindert wurde. Diese wird als $IC_{50}$-Wert bezeichnet.

Aus dem ermittelten $IC_{50}$-Wert lässt sich mit bekannter Dissoziationskonstanten $K_d$ des Radioliganden die

Gleichgewichtskonstante $K_i$ berechnen gemäß folgender Gleichung (c) nach Cheng und Prusoff (Cheng, Y. C.; Prusoff, W. H. Biochem. Pharmacol. 1973, 22, 3099-3108):

$$K_i = \frac{IC_{50}}{1+\frac{[L]}{K_d}} \quad (c)$$

mit

$K_i$ Inhibitionskonstante der Testsubstanz
$IC_{50}$ Testsubstanzkonzentration, bei der 50 % des Radioliganden verdrängt werden
[L] Konzentration des Radioliganden
$K_d$ Dissoziationskonstante des Radioliganden.

Der $K_i$-Wert wurde nach der Methode gemäß Hunter et al., Br. J. Pharmacol. 1990, 1001. 183-189 und Smith et al., J. Neuoch. 1989, 53, 27-36 bestimmt, wobei eine Präparation aus Meerschweinchenganzhirn verwendet wurde und als Radioligand [$^3$H]-U-69,593 (Amersham) verwendet wurde.
Die $K_i$-Werte wurden aus $IC_{50}$-Werten berechnet, die aus Kompetitionskurven mit sechs verschiedenen Konzentrationen ermittelt wurden. Bei Verbindungen mit hoher Affinität wurden die $K_i$-Werte zwei bzw. drei Mal bestimmt und Mittelwerte und Standardabweichung (SEM, standard error of the mean) berechnet.

Testlösungen

Die Verbindung- 2-(3,4-Dichlorphenyl)-1-[(4aRS,8SR,8aRS)-8-(pyrrolidin-1-yl)-perhydrochinoxalin-1-yl]-ethan-1-on und das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl} ethan-1-on wurden jeweils in Dimethylsulfoxid (DMSO) ohne Wasserzusatz zu einer 10 mM Lösung gelöst. Anschließend wurde diese Stammlösung bei -80 °C eingefroren. Bei Bedarf wurde die Probe aufgetaut und mit Inkubationspuffer auf die erforderlichen Konzentrationen verdünnt.

Allgemeine Durchführung

Zuerst wurde ein Screening mit sechs unterschiedlichen Konzentrationen (Konzentration c = $10^{-5}$ mol/l, $10^{-6}$ mol/l, $10^{-7}$ mol/l, $10^{-8}$ mol/l, $10^{-9}$ mol/l, $10^{-10}$ mol/l) durchgeführt. Die Lösungen wurden jeweils zwei Mal vermessen. Anschließend wurden die Testläufe ebenfalls in sechs verschieden Konzentrationen durchgeführt. Diese wurden so gewählt, dass der abgeschätzte $IC_{50}$-Wert im mittleren Bereich der Konzentrationsspanne lag.
Die Auswertung der Verdrängungsexperimente erfolgte über nichtlineare Regression mit GraphPad Prism 3.0 (GraphPad Software). Die erhaltenen $IC_{50}$-Werte wurden nach der Gleichung von Cheng und Prusoff(Cheng, Y. C.; Prusoff, W. H. Biochem. Pharmacol. 1973, 22, 3099-3108) in $K_i$-Werte umgerechnet.
Die Testläufe wurden dreifach ausgeführt, und von den Triplikaten wurde der Mittelwert gebildet mit der Standardabweichung des Mittelwertes (SEM, "standard error of the mean"). Die jeweiligen Werte der Gleichgewichtsdissoziationskonstante der Radioliganden wurden der Literatur entnommen.

Standardisierung der Assays

Zur Standardisierung des Messverfahrens wurden die Rezeptorpräparationen in unterschiedlichen Konzentrationen mit dem jeweiligen Puffer verdünnt und sowohl die unspezifischen als auch die Gesamtbindungen gemessen. Die Verdünnungen der Rezeptorpräparationen wurden anschließend so gewählt, dass die unspezifische Bindung bei etwa 10 % der Gesamtbindung lag (ca. 30 von 300 cpm). Dadurch war eine Mindestkonzentration an gewünschtem Rezeptor in der Proteinsuspension gewährleistet. Anschließend folgte die Bestimmung der Proteinkonzentration nach Bradford (ca. 1,5 mg/ml bis 4,0 mg/ml).

Herstellung der κ-Rezeptorpräparation

Alle vorbereiteten Lösungen wurden auf Eis gekühlt. Fünf Meerschweinchenhirne wurden mit ca. 5 - 6facher Menge Saccharose-Lösung (0,32 M) versetzt und im Potter (Elvehjem-Potter, Fa. Braun) unter Eiskühlung homogenisiert

(ca. 800 bis 1000 Umdrehungen/Minute). Das Homogenisat wurde in Zentrifugengefäße (40 ml) gegeben und in der Hochleistungskühlzentrifuge (Sorvall RC-5, Fa. Thermo Fisher Scientific) zentrifugiert (2900 Umdrehungen/Minute, 4 °C, 10 min). Der Überstand wurde in Ultrazentrifugengefäße (40 ml) gegeben und erneut zentrifugiert (23500 g, 4 °C, 20 min, Sorvall RC-5, Fa. Thermo Fisher Scientific).

Der Überstand der Ultrazentrifugation wurde verworfen und das Pellet mit wenig eiskaltem TRIS-Puffer (50 mM, pH 8,0, 1,66 g Tris-Base, 5,72 g Tris-HCl, ad 1 1 Wasser). Durch kräftiges Schütteln (Vortexer) wurde das Pellet resuspendiert und 30 Minuten bei Raumtemperatur (22 °C) unter kontinuierlichem Schütteln inkubiert. Anschließend wurde die Suspension nochmals zentrifugiert (23500 g, 4 °C, 20 min). Der Überstand wurde verworfen und das Pellet mit etwas kaltem TRIS-Puffer aufgenommen. Nach Homogenisierung im Potter wurden die unspezifische und die Gesamtbindung bestimmt. Anschließend wurde die Proteinsuspension mit TRIS-Puffer verdünnt, so dass die unspezifische Bindung etwa 10 % der Gesamtbindung betrug, und eine Proteinbestimmung nach Bradford durchgeführt (Proteinstandard: Bovines Serum Albumin, Firma Sigma-Aldrich). Der Proteingehalt der Präparation lag in der Regel bei ca. 1,5 mg / ml. Das Homogenisat wurde in 2 ml-Eppendorfgefäße abgefüllt und bei -81 °C eingefroren.

Bestimmung der Affinität zum κ-Rezeptor

Ausgehend von der 10 mM Stammlösung des Diastereomeren-Gemischs 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on und der Verbindung 2-(3,4-Dichlorphenyl)-1-[(4aRS,8SR,8aRS)-8-(pyrrolidin-1-yl)-perhydrochinoxalin-1-yl]-ethan-1-on wurden durch Verdünnen mit Puffer Lösungen des Diastereomeren-Gemischs und der Verbindung 2-(3,4-Dichlorphenyl)-1-[(4aRS,8SR,8aRS)-8-(pyrrolidin-1-yl)-perhydrochinoxalin-1-yl]-ethan-1-on in verschiedenen Konzentrationen hergestellt. Als Radioligand wurde [$^3$H]-U69.593 (Amersham) in TRIS-Puffer (50 mM, pH 7,4) verwendet. Vor der Durchführung der Bindungsstudien wurden die Filtermatten (Filtermat A, Fa. Perkin-Elmer) 2,5 Stunden in Polyethylenimin-Lösung (0,2 %) eingelegt, um die unspezifische Bindung zu reduzieren. Die Gesamtbindung und die unspezifische Bindung wurden bei jedem Testlauf mitbestimmt. Zur Bestimmung der unspezifischen Bindung wurde der Assay in Gegenwart eines großen Überschusses unmarkierten U69.593 (10 μM) durchgeführt. Zur Messung der Gesamtbindung wurde der Assay ohne Testsubstanz durchgeführt und das fehlende Volumen durch Puffer ersetzt. In einem Gesamtvolumen von 200 μl wurden 50 μl TRIS-$MgCl_2$-Puffer, 50 μl Testsubstanz-Lösung, 50 μl Radioligand-Lösung (4 nM; entspricht 1 nM im Assay) und zuletzt 50 μL Proteinlösung (ca. 1,5 mg / ml) in eine Vertiefung einer Mikrotiterplatte (Standard 96-well Multititerplatte, Fa. Diagonal) pipettiert. Nach dem Füllen aller Vertiefungen wurde die Platte mit einem Deckel verschlossen und 2,5 Stunden bei 37 °C und ca. 500 Umdrehungen / Minute mit einem Schüttler (Eigenbau) geschüttelt. Nach der Inkubation wurde der Deckel abgenommen und die Platte mit Hilfe des Zellsammlers Unifilter 96 Harvester (Fa. Perkin-Elmer) durch eine Filtermatte abgesaugt. Die Vertiefungen wurden fünfmal unter vermindertem Druck mit Wasser gewaschen. Nach dem Waschen wurde die Filtermatte bei vermindertem Druck im geöffneten Zellsammler Unifilter 96 Harvester erst vorgetrocknet, danach im vorgeheizten Trockenschrank 5 Minuten bei 95 °C vollständig getrocknet. Anschließend wurde Meltilex-Schmelzszintillator (Meltilex A, Fa. Perkin-Elmer) auf die Filtermatte gelegt und im Trockenschrank ca. 2 - 3 Minuten bei 95 °C erhitzt, bis der Schmelzszintillator die Matte komplett durchdrungen hatte. Bei Raumtemperatur erstarrte der Szintillator innerhalb 1 Minute wieder vollständig, so dass die Filtermatte im Szintillationszähler (Microbeta TRILUX, Fa. Perkin-Elmer) vermessen werden konnte ([$^3$H]-Messprotokoll; 5 Minuten-Messzeit pro Vertiefung). Der $K_d$-Wert des Radioliganden [$^3$H]-U69.593 ($K_d$= 0,67 nM) wurde der Literatur entnommen.

Es konnte festgestellt werden, dass das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl)ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on und die Verbindung 2-(3,4-Dichlorphenyl)-1-[(4aRS,8SR,8aRS)-8-(pyrrolidin-1-yl)-perhydrochinoxalin-1-yl]-ethan-1-on jeweils eine hohe Affinität für den κ-Rezeptor von 8,7 ±1,1 nM und 2,1 ± 0,4 nM aufwiesen.

Beispiel 31

Bestimmung der κ-Rezeptor-Affinität

Die Bestimmung der κ-Rezeptor-Affinität für die in den Tabellen 1 und 2 aufgeführten Verbindungen wurde unter Verwendung dieser Verbindungen durchgeführt wie unter Beispiel 30 beschrieben. Es wurden die in der nachstehenden Tabellen 1 und 2 dargestellten Werte für die Affinität der Verbindungen zum κ-Rezeptor erhalten:

Tabelle 1

| Verbindung | κ: $K_i \pm$ SEM / nM |
|---|---|
| 1-[(*4aRS,8SR,8aRS*)-4-Benzoyl-8-(pyrrolidin-1-yl)-perhydrochinoxalin-1-yl]-2-(3,4-dichlorphenyl)ethan-1-on | 15 ± 3,4 |
| 1-[(*4aRS,8SR,8aRS*)-4-Acetyl-8-(pyrrolidin-1-yl)-perhydrochinoxalin-1-yl]-2-(3,4-dichlorphenyl) ethan-1-on | 24 ± 2,8 |
| 1-{(*4aRS,8SR,8aRS*)-1-[2-(3,4-Dichlorphenyl)acetyl]-8-(pyrrolidin-1-yl)-perhydrochinoxalin-4-yl}propan-1-on | 26 ± 1,3 |
| Methyl-{(*4aRS,8SR,8aRS*)-1-[2-(3,4-dichlorphenyl)acetyl]-8-(pyrrolidin-1-yl)-perhydrochinoxalin-4-yl}carboxylat | 9,7 ± 1,8 |
| Ethyl-{(*4aRS,8SR,8aRS*)-1-[2-(3,4-dichlorphenyl)acetyl]-8-(pyrrolidin-1-yl)-perhydrochinoxalin-4-yl}carboxylat | 15 ± 3,0 |
| 3-{(*4aRS,8SR,8aRS*)-1-[2-(3,4-Dichlorphenyl)acetyl]-8-(pyrrolidin-1-yl)-perhydrochinoxalin-4-yl}-3-oxopropionsäure | 169 ± 63 |
| 4-{(*4aRS,8SR,8aRS*)-1-[2-(3,4-Dichlorphenyl)acetyl]-8-(pyrrolidin-1-yl)perhydrochinoxalin-4-yl}-4-oxobuttersäure | 136 ± 31 |
| Methyl-3-{(*4aRS,8SR,8aRS*)-1-[2-(3,4-Dichlorphenyl)acetyl]-8-(pyrrolidin-1-yl)-perhydrochinoxalin-4-yl}-3-oxopropionat | 11 ± 5,6 |
| 1-{(*4aRS,8SR,8aRS*)-4-Benzoyl-8-[(*3SR*)- und (*3RS*)-3-hydroxypyrrolidin-1-yl]-perhydrochinoxalin-1-yl}-2-(3,4-dichlorphenyl)ethan-1-on | 22 ± 5,6 |
| Methyl-{(*4aRS,8SR,8aRS*)-1-[2-(3,4-dichlorphenyl)acetyl]-8-[(*3SR*)-und (*3RS*)-3-hydroxypyrrolidin-1 -yl]-perhydrochinoxalin-4-yl}carboxylat | 11 ± 2,8 |
| 3-{(*4aRS,8SR,8aRS*)-1-[2-(3,4-Dichlorphenyl)acetyl]-8-[(*3SR*)- und (*3RS*)-3-hydroxypyrrolidin-1-yl]-perhydrochinoxalin-4-yl}-3-oxopropionsäure | 482 ± 113 |
| Methyl-3-{(*4aRS,8SR,8aRS*)-1-[2-(3,4-Dichlor-phenyl)acetyl]-8-[(*3SR*)- und (*3RS*)-3-hydroxy-pyrrolidin-1-yl]-perhydrochinoxalin-4-yl} -3-oxopropionat | 18 ± 2,2 |

Tabelle 2

| Verbindung | κ: $K_i \pm$ SEM / nM |
|---|---|
| 2-(3,4-Dichlorphenyl)-1-[(*4aRS,8BSR,8aRS*)-4-methyl-8-(pyrrolidin-1-yl)-perhydrochinoxalin-1-yl]ethan-1-on | 2,7 ± 0,6 |
| 1-[(*4aRS,8SR,8aRS*)-4-Butyl-8-(pyrrolidin-1-yl)per-hydrochinoxalin-1-yl]-2-(3,4-dichlorphenyl)-ethan-1-on | 3,1 ± 1,8 |
| 1-[(*4aRS,8SR,8aRS*)-4-Benzyl-8-(pyrrolidin-1-yl)per-hydrochinoxalin-1-yl]-2-(3,4-dichlorphenyl)ethan-1-on | 9,4 ± 1,6 |
| 2-(3,4-Dichlorphenyl)-1-[(*4aRS,8SR,8aRS*)-4-(4-Methoxybenzyl)-8-(pyrrolidin-1-yl)-perhydro-chinoxalin-1-yl]ethan-1-on | 6,8 ± 2,0 |
| 2-(3,4-Dichlorphenyl)-1-{(*4aRS, 8SR,8aRS)-4-[*(pyridin-2-yl)methyl]-8-(pyrrolidin-1-yl) perhydrochinoxalin-1-yl}ethan-1-on | 4,2 ± 2,6 |
| 2-(3,4-Dichlorphenyl)-1-{(*4aRS,8SR,8aRS*)-4-[(pyridin-3-yl)methyl]-8-(pyrrolidin-1-yl) perhydrochinoxalin-1-yl}ethan-1-on | 0,13 ± 0,02 |
| 2-(3,4-Dichlorphenyl)-1-{(*4aRS,8*SR,*8aRS*)-4-[(1H-imidazol-5-yl)methyl]-8-(pyrrolidin-1-yl) perhydrochinoxalin-1-yl}ethan-1-on | 4,3 ± 2,0 |
| 2-(3,4-Dichlorphenyl)-1-{(*4aRS,8SR,8aRS*)-4-methyl-8-[(*3SR*)- und (*3RS*)-3-hydroxypyrrolidin-1-yl]-perhydrochinoxalin-1-yl}ethan-1-on | 5,4±0,8 |

(fortgesetzt)

| Verbindung | κ: $K_i$ ± SEM / nM |
|---|---|
| 1-{(*4aRS,8SR,8aSR*)-4-Benzyl-8-[(*3SR*)-und (*3RS*)-3-hydroxypyrrolidin-1-yl] perhydrochinoxalin-1-yl} -2-(3,4-dichlorphenyl)ethan-1-on | 6,6 ± 1,4 |
| 2-(3,4-Dichlorphenyl)-1-{(*4aRS,8SR,8aSR*)-4-[(pyridin-3-yl)methyl]-8-[(*3SR*)- und (*3RS*)-3-hydroxypyrrolidin-1-yl]perhydrochinoxalin-1-yl} ethan-1-on | 3,8 ± 0,7 |

Es konnte festgestellt werden, dass die Verbindungen jeweils eine hohe Affinität für den κ-Rezeptor aufwiesen.

Beispiel 32

Bestimmung der Selektivität der Bindung zum κ-Rezeptor

Die Bestimmung der Rezeptor-Affinität im Rahmen der Selektivitätsuntersuchungen wurde in humanem Rezeptormaterial durchgeführt. Hierbei wurde [$^3$H]- CI-977 (TRK945, Fa. Amersham, spezifische Aktivität ca. 48 Ci / mmol) für den κ-Rezeptor und [$^3$H]-Naloxon (N-allyl-2,3) (NET719, Fa. NEN, spezifische Aktivität ca. 60 Ci / mmol) für den μ-Rezeptor als Radioligand verwendet.

Allgemeine Durchführung (Bindung humanen κ- und μ-Opiatrezeptormembranen)

In Abweichung von der unter Beispiel 30 beschriebenen Versuchsdurchführung zur k-Opiatrezeptorbindung an Meerschweinchenhirnhomogenaten wurden zur Bestimmung der Bindung an humanen κ- und μ-Opiatrezeptormembranen jeweils ein Rezeptorscreening mit fünf unterschiedlichen Konzentrationen (Konzentration c = $10^{-5}$, $10^{-6}$ , $10^{-7}$, $10^{-8}$, $10^{-9}$ mol / 1 jeweils als Doppelwerte) in 2 voneinander unabhängigen Versuchen durchgeführt.

Die Auswertung und Bestimmung der jeweiligen $IC_{50}$-Werte wurde ebenfalls mittels nichtlinearer Regressionsberechnung durch die Software XLfit Version 4 eingebettet in die Auswertungssoftware ActivityBase Version 5.3.4.26 durchgeführt. Die Berechnung von $K_i$-Werten aus den jeweiligen $IC_{50}$-Werten erfolgte mit der unter Beispiel 30 aufgeführten Cheng-Prussof-Gleichung. Die jeweiligen Werte der Dissoziationskonstanten zur Berechnung der $K_i$-Werte nach der Cheng-Prussof-Gleichung wurden vorab für die verwendeten Rezeptormembranpräparationen unter den gleichen Rezeptorbindungsbedingungen durch Liganden-Rezeptor-Sättigungsversuche ermittelt.

Bestimmung der Affinität zum κ-Rezeptor

Die Rezeptormembranen des humanen κ-opioid Rezeptors aus HEK-293-Zellen (Fa. PerkinElmer Life Sciences (Best.-Nr. 6110558 #370-960-A)) wurden kurz (2 Minuten) vor der Verwendung in warmem (ca. 37 °C) Wasser aufgetaut, mit Assay-Puffer (50 mmol / l TRIS-HCl, pH 7,4) supplementiert mit 0,02 % bovinem Serumalbumin (Fa. Serva) im Verhältnis 1:34 verdünnt und im Potter homogenisiert. Wheatgerm agglutinin SPA ("Scintillation Proximity Assay") Beads, (Fa. Amersham (Best.-Nr. RPNQ0001)) wurden mit Assay-Puffer (50 mmol / 1 TRIS-HCl, pH 7,4) (70 ml / 500 mg Beads) versetzt und 1 Stunde auf einem Magnetrührer suspendiert. In die Vertiefungen einer Lumineszenzplatte (SPA-Platten, Fa. Costar) wurden nun jeweils 5 μl der Lösung des Diastereomeren-Gemischs 2-(3,4-Dichlorphenyl)-1-{(4aRS,8SR, 8aSR)-8-[(3SR)-3-hydroxypyrrolidin-1-yl]perhydrochinoxalin-1-yl}ethan-1-on und 2-(3,4-Dichlorphenyl)-1-{(4aRS,8SR, 8aSR)-8-[(3RS)-3-hydroxypyrrolidin-1-yl]perhydrochinoxalin-1-yl}ethan-1-on oder der Verbindung 2-(3,4-Dichlorphenyl)-1-[(4aRS,8SR,8aRS)-8-(pyrrolidin-1-yl)-perhydrochinoxalin-1-yl]-ethan-1-on, die jeweils in einer 50-fach höheren Konzentration (in 25 %igem wässrigen Dimethylsulfoxid (DMSO)) als der jeweiligen Testkonzentration im Reaktionsansatz gelöst vorlagen, 20 μl Radioligand [$^3$H]-CI-977 (TRK945, Fa. Amersham, spezifische Aktivität ca. 48 Ci / mmol) (12,5 nmol / 1 Assay-Puffer) und 225 μl einer vorinkubierten Mischung aus 88 μl der verdünnten Rezeptormembran und 137 μl Beadsuspension pipettiert. Zur Bestimmung der nicht-spezifischen Bindung wurde anstelle der Lösung des Diastereomeren-Gemischs 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on oder der Verbindung 2-(3,4-Dichlorphenyl)-1-[(4aRS,8SR,8aRS)-8-(pyrrolidin-1-yl)-perhydrochinoxalin-1-yl]-ethan-1-on5 μl Naloxon-HCl (500 μmol/ / 1 wässriger 25%iger DMSO-Lösung) und zur Bestimmung der Gesamtbindung 5 μl einer wässrigen 25 % DMSO-Lösung zugegeben. Somit enthielten alle Reaktionsansätze mit unterschiedlichen Konzentrationen des Diastereomeren-Gemischs oder der Verbindung 2-(3,4-Dichlorphenyl)-1-[(4aRS,8SR,8aRS)-8-(pyrrolidin-1-yl)-perhydrochinoxalin-1-yl]-ethan-1-onund die Ansätze zur Bestimmung der unspezifischen Bindung sowie der maximalen Bindung einen 0,5%igen DMSO-Lösemittelanteil im Endansatz. Die Ansätze wurden mit einem Minishaker durchmischt und für 90 Minuten bei Raumtemperatur inkubiert. Anschließend wurden die

Proben 20 Minuten bei $500^{-1}$ (60 g) abzentrifugiert (Omnifuge 2.0 RS Fa. Heraeus) und die an die SPA-Beads gebundene Radioaktivität mit dem Szintillationszähler (1450 Microbeta Trilux, Fa. Wallac/PerkinElmer Life Sciences) vermessen.

Es konnte festgestellt werden, dass das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on und die Verbindung 2-(3,4-Dichlorphenyl)-1-[(4aRS,8SR,8aRS)-8-(pyrrolidin-1-yl)-perhydrochinoxalin-1-yl]-ethan-1-onjeweils eine hohe Affinität für den κ-Rezeptor von 19 nM und 28 nM aufwiesen.

<u>Bestimmung der Affinität zum μ-Rezeptor.</u>

Rezeptormembranen des human μ-Opioid Rezeptors aus CHO-K1-Zellen (RBHOMM) (Fa. PerkinElmer Life Sciences) wurden kurz (2 Minuten) vor der Verwendung in warmem (37 °C) Wasser aufgetaut, mit Assay-Puffer (50 mmol / 1 Tris-HCl, pH 7,4) supplementiert mit 0,06 % bovinem Serumalbumin (Fa. Serva) verdünnt und im Potter homogenisiert. Wheatgerm agglutinin SPA ("Scintillation Proximity Assay") Beads, (Fa. Amersham (Best.-Nr. RPNQ0001)), wurden mit Assay-Puffer (50 mmol / 1 Tris-HCl pH 7,4) (100 ml / 500 mg Beads) versetzt und 1 Stunde auf einem Magnetrührer suspendiert. In die Vertiefungen einer Lumineszenzplatte (SPA-Platten, Fa. Costar) wurden nun jeweils 5 μl der Lösung des Diastereomeren-Gemischs 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl} ethan-1-on oder der Verbindung 2-(3,4-Dichlorphenyl)-1-[(4aRS,8SR, 8aRS)-8-(pyrrolidin-1-yl)-perhydrochinoxalin-1-yl]-ethan-1-on, die jeweils in einer 50-fach höheren Konzentration (in 25 %igem wässrigen Dimethylsulfoxid (DMSO)) als der jeweiligen Testkonzentration im Reaktionsansatz gelöst vorlagen, 25 μl Radioligand [$^3$H]-Naloxon (N-allyl-2,3) (NET719, Fa. NEN, spezifische Aktivität ca. 60 Ci / mmol) (10 nmol / 1 Assay-Puffer) und 220 μl einer vorinkubierten Mischung aus 20 μl Rezeptormembran und 200 μl Beadsuspension pipettiert. Zur Bestimmung der nicht-spezifischen Bindung wurde anstelle der Lösung des Diastereomeren-Gemischs 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on oder der Verbindung 2-(3,4-Dichlorphenyl)-1-[(4aRS,8SR,8aRS)-8-(pyrrolidin-1-yl)-perhydrochinoxalin-1-yl]-ethan-1-on5 μl Naloxon-HCl (500 μmol / 1 wässriger 25%iger DMSO-Lösung) und zur Bestimmung der Gesamtbindung 5 μl einer wässrigen 25 % DMSO-Lösung zugegeben. Somit enthielten alle Reaktionsansätze mit unterschiedlichen Konzentrationen des Diastereomeren-Gemischs oder der Verbindung 2-(3,4-Dichlorphenyl)-1-[(4aRS,8SR,8aRS)-8-(pyrrolidin-1-yl)-perhydrochinoxalin-1-yl]-ethan-1-onund die Ansätze zur Bestimmung der unspezifischen Bindung sowie der maximalen Bindung einen 0,5%igen DMSO-Lösemittelanteil im Endansatz. Die Ansätze wurden mit einem Minishaker durchmischt und für 90 Minuten bei Raumtemperatur inkubiert. Anschließend wurden die Proben 20 Minuten bei $500^{-1}$ (60 g) abzentrifugiert (Omnifuge 2.0 RS Fa. Heraeus) und die an die SPA-Beads gebundene Radioaktivität mit dem Szintillationszähler (1450 Microbeta Trilux, Fa. Wallac/PerkinElmer Life Sciences) vermessen.

Es konnte festgestellt werden, dass das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on und die Verbindung 2-(3,4-Dichlorphenyl)-1-[(4aRS,8SR,8aRS)-8-(pyrrolidin-1-yl)-perhydrochinoxalin-1-yl]-ethan-1-onjeweils eine Affinität für den μ-Rezeptor von 4900 nM und 2800 nM aufwiesen.

Im Vergleich liegt somit die Selektivität der Affinität der Bindung κ-Rezeptor im vergleich zum μ-Rezeptor für das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on bei 258:1 und für die Verbindung 2-(3,4-Dichlorphenyl)-1-[(4aRS,8SR,8aRS)-8-(pyrrolidin-1-yl)-perhydrochinoxalin-1-yl]-ethan-1-on bei 99:1. Dies zeigt, dass sich das Diastereomeren-Gemisch 2-(3,4-Dichlorphenyl)-1-{(4aRS,8SR,8aSR)-8-[(3SR)-3-hydroxypyrrolidin-1-yl]perhydrochinoxalin-1-yl}ethan-1-on und 2-(3,4-Dichlorphenyl)-1-{(4aRS,8SR,8aSR)-8-[(3RS)-3-hydroxypyrrolidin-1-yl]perhydrochinoxalin-1-yl}ethan-1-on und die Verbindung 2-(3,4-Dichlorphenyl)-1-[(4aRS,8SR,8aRS)-8-(pyrrolidin-1-yl)-perhydrochinoxalin-1-yl]-ethan-1-on durch eine Selektivität der Bindung an den κ-Rezeptor gegenüber der Bindung an den μ-Rezeptor auszeichnen.

Beispiel 33

Bestimmung der Selektivität der Bindung zum κ-Rezeptor

Die Bestimmung der Selektivität der Bindung zum κ-Rezeptor für die in den Tabellen 3 und 4 aufgeführten Verbindungen wurde unter Verwendung dieser Verbindungen durchgeführt wie unter Beispiel 32 beschrieben. Es wurden die in der nachstehenden Tabellen 3 und 4 dargestellten Werte für die Selektivität der Bindung der Verbindungen zum κ-Rezeptor gegenüber der Bindung an den μ-Rezeptor erhalten:

Tabelle 3

| Verbindung | Selektivität κ/μ |
|---|---|
| 1-[(*4aRS*,*8SR*,*8aRS*)-4-Benzoyl-8-(pyrrolidin-1-yl)-per-hydrochinoxalin-1-yl]-2-(3,4-dichlorphenyl)ethan-1-on | 14:1 |
| 1-[(*4aRS*,*8SR*,*8aRS*)-4-Acetyl-8-(pyrrolidin-1-yl)-per-ydrochinoxalin-1-yl]-2-(3,4-dichlorphenyl) ethan-1-on | 14:1 |
| 1-{(*4aRS,8SR,8aRS*)-1-[2-(*3*,4-Dichlorphenyl)acetyl]-8-(pyrrolidin-1-yl)-perhydrochinoxalin-4-yl} propan-1-on | 8:1 |
| Methyl-{(4*aRS,8SR,8aRS*)-1-[2-(3,4-dichlorphenyl)-acetyl]-8-(pyrrolidin-1-yl)-perhydrochinoxalin-4-yl}-carboxylat | 15:1 |
| Ethyl-{(*4aRS*,*8SR*,*8aRS*)-1-[2-(3,4-dichlorphenyl)-acetyl]-8-(pyrrolidin-1-yl)-perhydrochinoxalin-4-yl} carboxylat | 10:1 |
| Methyl-3-{(*4aRS,8SR,8aRS*)-1-[2-(3,4-Dichlorphenyl)-acetyl]-8-(pyrrolidin-1-yl)-perhydrochinoxalin-4-yl}-3-oxopropionat | 22:1 |
| 1-{(*4aRS,8SR,8aRS*)-4-Benzoyl-8-[(*3SR*)- und (*3RS*)-3-hydroxypyrrolidin-1-yl]-perhydrochinoxalin-1-yl}-2-(3,4-dichlorphenyl)ethan-1-on | 21:1 |
| Methyl-{(*4aRS,8SR,8aRS*)-1-[2-(3,4-dichlor-phenyl)acetyl]-8-[(*3SR*)- und (*3RS*)-3-hydroxy-pyrrolidin-1-yl]-perhydrochinoxalin-4-yl}carboxylat | 43:1 |
| Methyl-3-{(*4aRS,8SR,8aRS*)-1-[2-(3,4-Dichlor-phenyl)acetyl]-8-[(*3SR*)-und (*3RS*)-3-hydroxy-pyrrolidin-1-yl]-perhydrochinoxalin-4-yl}-3-oxopropionat | 33:1 |

Tabelle 4

| Verbindung | Selektivität κ/μ |
|---|---|
| 2-(3,4-Dichlorphenyl)-1-[(*4aRS*,*8SR*,*8aRS*)-4-methyl-8-(pyrrolidin-1-yl)-perhydrochinoxalin-1-yl] ethan-1-on | 131:1 |
| 1-[(*4aRS*,*8SR*,*8aRS*)-4-Butyl-8-(pyrrolidin-1-yl)per-hydrochinoxalin-1-yl]-2-(3,4-dichlorphenyl)-ethan-1-on | 177:1 |
| 1-[(*4aRS,8SR,8aRS*)-4-Benzyl-8-(pyrrolidin-1-yl)per-hydrochinoxalin-1-yl]-2-(3,4-dichlorphenyl) ethan-1-on | 153:1 |
| 2-(3,4-Dichlorphenyl)-1-[(*4aRS,8SR,8aRS*)-4-(4-Methoxybenzyl)-8-(pyrrolidin- 1-yl)-perhydro-chinoxalin-1-yl]ethan-1-on | 16:1 |
| 2-(3,4-Dichlorphenyl)-1-{(*4aRS,8SR,8aRS*)-4-[(pyridin-2-yl)methyl]-8-(pyrrolidin-1-yl) perhydrochinoxalin-1-yl}ethan-1-on | 96:1 |
| 2-(3,4-Dichlorphenyl)-1-{(*4aRS,8SR,8aRS*)-4-[(pyridin-3-yl)methyl]-8-(pyrrolidin-1-yl) perhydrochinoxalin-1-yl}ethan-1-on | 112:1 |
| 2-(3,4-Dichlorphenyl)-1-{(*4aRS,8SR,8aRS*)-4-[(1H-imidazol-5-yl)methyl]-8-(pyrrolidin-1-yl)per-hydrochinoxalin-1-yl}ethan-1-on | 108:1 |
| 2-(3,4-Dichlorphenyl)-1-{(*4aRS*,*8SR*,*8aRS*)-4-methyl-8-[(*3SR*)-und (*3RS*)-3-hydroxypyrrolidin-1-yl]-perhydrochinoxalin-1-yl}ethan-1-on | 122:1 |
| 1-{(*4aRS,8SR,8aSR*)-4-Benzyl-8-[(*3SR*)-und (*3RS*)-3-hydroxypyrrolidin-1-yl]perhydrochinoxalin-1-yl}-2-(3,4-dichlorphenyl)ethan-1-on | 112:1 |
| 2-(3,4-Dichlorphenyl)-1-{(*4aRS,8SR,8aSR*)-4-[(pyridin-3-yl)methyl]-8-[(*3SR*)-und (*3RS*)-3-hydroxypyrrolidin-1-yl]perhydrochinoxalin-1-yl-}ethan-1-on | 112:1 |
| 2-(3,4-Dichlorphenyl)-1-{(*4aRS,8SR,8aSR*)-4-[(1H-Imidazol-5-yl)methyl]-8-[(*3SR*)-und (*3RS*)-3-hydroxypyrrolidin-1-yl]perhydrochinoxalin-1-yl}ethan-1-on | 90:1 |

(fortgesetzt)

| Verbindung | Selektivität $\kappa/\mu$ |
|---|---|
| 〈(*3SR*)-und (*3RS*)-1-{(*4aRS,5RS,8aSR*)-4-[2-(3,4-dichlorphenyl)acetyl]-perhydrochinoxalin-5-yl} pyrrolidin-3-yl〉-2-(3,4-dichlorphenyl)acetat | 138:1 |

Es konnte festgestellt werden, dass die Verbindungen jeweils eine hohe Selektivität der Bindung zum $\kappa$-Rezeptor gegenüber der Bindung an den $\mu$-Rezeptor aufwiesen.

## Patentansprüche

**1.** Verbindungen gemäß der allgemeinen Formel (1) wie nachstehend angegeben und/oder deren Racemate, Enantiomere, Diastereomere, Solvate, Hydrate sowie deren pharmazeutisch verträgliche Salze und/oder Ester:

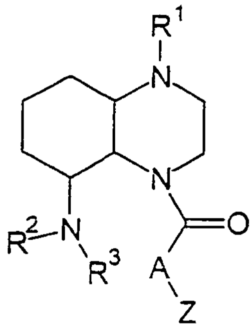

(1)

worin:

$R^1$ ist ausgewählt aus der Gruppe umfassend H; $C_1$-$C_{10}$-Alkyl; $C_3$-$C_{10}$-Cycloalkyl; COO($C_1$-$C_{10}$-Alkyl); $C_1$-$C_6$-Alkoxycabonyl; $C_1$-$C_6$-Oxocarbonyl; Phenylalkyl mit $C_1$-$C_6$-Alkyl wobei der Phenylrest mit einer oder mehreren gleichen oder verschiedenen Gruppen ausgewählt aus der Gruppe umfassend Halogen, $C_1$-$C_6$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N($C_1$-$C_5$-Alkyl)$_2$, $SO_2NH_2$, $SO_2$NH($C_1$-$C_5$-Alkyl), $SO_2$NH(Aryl), $SO_2$NH(Phenyl) und/oder $SO_2$NH(Heteroaryl) substituiert sein kann; $C_1$-$C_{10}$-Acyl; $C_3$-$C_{10}$-Cycloacyl; Phenylacyl, wobei der Acylrest ein $C_1$-$C_6$-Acylrest ist und der Phenylrest mit einer oder mehreren gleichen oder verschiedenen Gruppen ausgewählt aus der Gruppe umfassend Halogen, $C_1$-$C_6$-Alkyloxy, $NN_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N($C_1$-$C_5$-Alkyl)$_2$, $SO_2NH_2$, $SO_2$NH($C_1$-$C_5$-Alkyl), $SO_2$NH(Aryl), $SO_2$NH(Phenyl) und/oder $SO_2$NH(Heteroaryl) substituiert sein kann: mono-, bi- oder tricyclisches Heteroaryl umfassend ein, zwei, drei oder vier Heteroatome ausgewählt aus der Gruppe umfassend N, O und/oder S; mono-, bi- oder tricyclisches Heteroarylalkyl umfassend ein, zwei, drei oder vier Heteroatome ausgewählt aus der Gruppe umfassend N, O und/oder S, wobei der Alkylrest ein $C_1$-$C_6$-Alkylrest ist; mono-, bi- oder tricyclisches Heteroarylacyl umfassend ein, zwei, drei oder vier Heteroatome ausgewählt aus der Gruppe umfassend N, O und/oder S, wobei der Acylrest ein $C_1$-$C_6$-Acylrest ist; C(O)($C_1$-$C_{10}$-Alkyl); C(O)N($C_1$-$C_{10}$-Alkyl)$_2$; C(O)($C_3$-$C_{10}$-Cycloalkyl); COO($C_1$-$C_{10}$-Alkyl); COO(Aryl); COO($C_3$-$C_{10}$-Cycloalkyl); C(O)COO($C_1$-$C_{10}$-Alkyl); C(O)-($CH_2$)$_q$ -COOH wobei q ist 0, 1, 2, 3 oder 4; C(O)-($CH_2$)$_r$-COO($C_1$-$C_{10}$-Alkyl) wobei r ist 0, 1, 2, 3 oder 4; C(O)-CH ($NH_2$)-($CH_2$)$_5$-COOH wobei s ist 0, 1, 2, 3 oder 4; C(O)-CH($NH_2$)-($CH_2$)$_r$-COO($C_1$-$C_{10}$-Alkyl) wobei t ist 0, 1, 2, 3 oder 4; C(O)-($CH_2$)$_u$-CH($NH_2$)-COOH wobei u ist 0, 1,2,3 oder 4 und/oder C(O)-($CH_2$)$_v$-CH($NH_2$)-COO ($C_1$-$C_{10}$-Alkyl) wobei v ist 0, 1, 2, 3 oder 4; $R^2$, $R^3$ sind jeweils gleich oder unabhängig voneinander ausgewählt aus der Gruppe umfassend H; $C_1$-$C_{10}$-Alkyl; $C_3$-$C_{10}$-Cycloalkyl; Phenylalkyl mit $C_1$-$C_6$-Alkyl und wobei der Phenylrest mit einer oder mehreren gleichen oder verschiedenen Gruppen ausgewählt aus der Gruppe umfassend Halogen, $C_1$-$C_5$-Alkyl, $C_1$-$C_4$-Alkyloxy, $NH_2$, NH($C_1$-$C_5$-Alkyl), N($C_1$-$C_5$-Alkyl)$_2$, OH, COOH, COO($C_1$-$C_{10}$-Alkyl), $CONH_2$, CONH($C_1$-$C_{10}$-Alkyl), CON($C_1$-$C_{10}$-Alkyl)$_2$, $SO_2$($C_1$-$C_5$-Alkyl), $SO_2$HN($C_1$-$C_5$-Alkyl), $CF_3$, CN und/oder $NO_2$ substituiert sein kann, oder $R^2$ und $R^3$ bilden gemeinsam mit dem Stickstoff, an den sie gebunden sind, einen gesättigten 3- bis 8-gliedrigen N-Heterocyclus aus, wobei dieser substituiert sein kann mit einer oder mehreren gleichen oder verschiedenen Gruppen ausgewählt aus der Gruppe umfassend OH, $C_1$-$C_4$-Al-

kyloxy, Carbonyl-Sauerstoff, $NH_2$, NH($C_1$-$C_5$-Alkyl), N($C_1$-$C_5$-Alkyl)$_2$, COOH, COO($C_1$-$C_{10}$-Alkyl), $CONH_2$, CONH($C_1$-$C_{10}$-Alkyl), CON($C_1$-$C_{10}$-Alkyl)$_2$, $OPO_3H_2$, $OSO_3H$, $SO_2$($C_1$-$C_5$-Alkyl), $SO_2$HN($C_1$-$C_5$-Alkyl), CN, O-Arylacetyl, O-Phenylacetyl, Arylacetoxy und/oder Acetylbenzyl, das mit zwei Cl-Gruppen substituiert sein kann;
A ist $(CH_2)_n$ wobei n 1 ist ;
Z ist ausgewählt aus der Gruppe umfassend Phenyl, das unit einer oder mehreren gleichen oder verschiedenen Gruppen ausgewählt aus der Gruppe umfassend Halogen, $C_1$-$C_5$-Alkyl, $C_1$-$C_5$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N($C_1$-$C_5$-Alkyl)$_2$, $SO_2NH_2$, $SO_2$NH ($C_1$-$C_5$-Alkyl), $SO_2$NH(Aryl), $SO_2$NH(Phenyl) und/oder $SO_2$NH(Heteroaryl) substituiert sein kann; ein mono- oder bicyclisches Aryl oder Heteroaryl umfassend ein oder zwei Heteroatome ausgewählt aus der Gruppe umfassend N, O und/oder S, wobei die Aryl - oder Heteroarylgruppe substituiert sein kann mit einer oder mehreren gleichen oder verschiedenen Gruppen ausgewählt aus der Gruppe umfassend Halogen, $C_1$-$C_4$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N ($C_1$-$C_5$-Alkyl)$_2$, $SO_2NH_2$, $SO_2$NH($C_1$-$C_5$-Alkyl), $SO_2$NH(Aryl), $SO_2$NH(Phenyl) und/oder $SO_2$NH(Heteroaryl).

2. Verbindungen, nach Anspruch 1, **dadurch gekennzeichnet, dass** die Verbindungen die nachstehende allgemeine Formel (2) aufweisen:

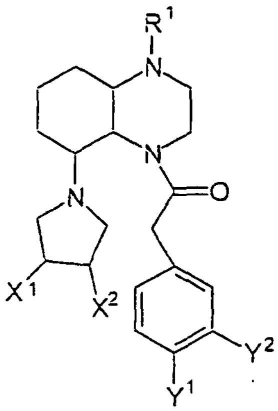

(2)

worin:

$R^1$ ist ausgewählt aus der Gruppe umfassend H; $C_1$-$C_{10}$-Alkyl; $C_3$-$C_{10}$-Cycloalkyl; COO($C_1$-$C_{lO}$-Alk.-yl); $C_1$-$C_6$-Alkoxycabonyl; $C_1$-$C_6$-Oxocarbonyl; Phenylalkyl mit $C_1$-$C_6$-Alkyl wobei der Phenylrest mit einer oder mehreren gleichen oder verschiedenen Gruppen ausgewählt aus der Gruppe umfassend Halogen, $C_1$-$C_6$-Alkyloxy, $NH_2$, NH($C_1$-$C_5$-Alkyl), N($C_1$-$C_5$-Alk-yl)$_2$, OH, $SO_2$($C_1$-$C_5$-Alkyl), SO($C_1$-$C_5$-Alkyl), $CF_3$, *CN,* $NO_2$, $SO_2$N($C_1$-$C_5$-Alkyl)$_2$, $SO_2NH_2$, $SO_2$NH($C_1$-$C_3$-Alkyl), $SO_2$NH(Aryl), $So_2$NH(Phenyl) und/oder $SO_2$NH(Heteroaryl) substituiert sein kann; $C_1$-$C_{10}$-Acyl; $C_3$-$C_{10}$-Cycloacyl; Phenylacyl, wobei der Acylrest ein $C_1$-$C_6$-Acylrest ist und der Phenylrest mit einer oder mehreren gleichen oder verschiedenen Gruppen ausgewählt aus der Gruppe umfassend Halogen, $C_1$-$C_6$-Alkyloxy, $NH_2$, NH($C_1$-$C_5$-Alkyl), N($C_1$-$C_5$-Alkyl)$_2$, OH, $SO_2$($C_1$-$C_5$-Alkyl), SO($C_1$-$C_5$-Alkyl), $CF_3$, CN, NO2, $SO_2$N($C_1$-$C_5$-Alkyl)$_2$, $SO_2NH_2$, $SO_2$NH($C_1$-$C_5$-Alkyl), $SO_2$NH(Aryl), $SO_2$-NH(Phenyl) und/oder $SO_2$NH (Heteroaryl) substituiert sein kann; mono-, bi- oder tricyclisches Heteroaryl umfassend ein, zwei, drei oder vier Heteroatome ausgewählt aus der Gruppe umfassend N, O und/oder S; mono-, bi- oder tricyclisches Heteroarylalkyl umfassend ein, zwei, drei oder vier Heteroatome ausgewählt aus der Gruppe umfassend N, O und/oder S, und der Alkylrest ein $C_1$-$C_6$-Alkylrest ist: mono-, bi- oder tricyclisches Heteroarylacyl umfassend ein, zwei, drei oder vier Heteroatome ausgewählt aus der Gruppe umfassend N, O und/oder S, und der Acylrest ein $C_1$-$C_6$-Acylrest ist; C(O)($C_1$-$C_{10}$-Alkyl); C(O)N($C_1$-$C_{10}$-Alkyl)$_2$; C(O)($C_3$-$C_{10l}$-Cycloalkyl); COO($C_1$-$C_{10}$-Alkyl); COO(Aryl); COO($C_3$-$C_{10}$-Cycloalkyl); C(O)COO($C_1$-$C_{10}$-Alkyl), C(O)-$(CH_2)_q$-COOH wobei q ist 0, 1, 2, 3 der 4, C(O)-$(CH_2)_r$COO($C_1$-$C_{10}$-Alkyl) wobei r ist 0, 1, 2, 3 oder 4, C(O)-CH($NH_2$)-$(CH_2)_s$-COOH wobei s ist 0, 1, 2, 3 oder 4, C(O)-CH($NH_2$)-$(CH_2)_t$COO($C_1$-$C_{10}$-Alkyl) wobei t ist 0, 1, 2, 3 oder 4, C(O)-$(CH_2)_u$-CH($NH_2$)-COOH wobei u ist 0, 1, 2, 3 oder 4, und/oder C(O)-$(CH_2)_v$-CH($NH_2$)-COO($C_1$-$C_{10}$-Alkyl) wobei v ist 0, 1, 2, 3 oder 4;
$X^1$, $X^2$ sind jeweils gleich oder unabhängig voneinander ausgewählt aus der Gruppe umfassend H, OH, Carbonyl-Sauerstoff, $NH_2$, NH($C_1$-$C_5$-Alkyl), N($C_1$-$C_5$-Alkyl)$_2$, COOH, COO($C_1$-$C_{10}$-Alkyl), $CONH_2$, CONH($C_1$-$C_{10}$-Alkyl), CON($C_1$-$C_{10}$-Alkyl)$_2$, $OPO_3H_2$, $OSO_3H$, $SO_2$($C_1$-$C_5$-Alkyl), $SO_2$HN($C_1$-$C_5$-Alkyl), $C_1$-$C_4$-Alkyloxy, O-Arylacetyl, O-Phenylacetyl, Arylacetoxy und/oder Acetylbenzyl, das mit zwei Cl Gruppen substituiert sein kann;

$Y^1$, $Y^2$ sind jeweils gleich oder unabhängig voneinander ausgewählt aus der Gruppe umfassend H, Halogen, $C_1$-$C_5$-Alkyl, $C_1$-$C_5$-Alkyloxy, $NH_2$, NH($C_1$-$C_5$-Alkyl), NH(Aryl), NH(Phenyl), NH(Heteroaryl), N($C_1$-$C_5$-Alkyl)$_2$, OH, $SO_2$($C_1$-$C_5$-Alkyl), SO($C_1$-$C_5$-Alkyl), $CF_3$, CN, $NO_2$, $SO_2$N($C_1$-$C_5$-Alkyl)$_2$, $SO_2NH_2$, $SO_2$NH($C_1$-$C_5$-Alkyl).

**3.** Verbindungen nach Anspruch 1 oder 2, **dadurch gekennzeichnet, dass** die Verbindungen die nachstehende allgemeine Formel (3) aufweisen:

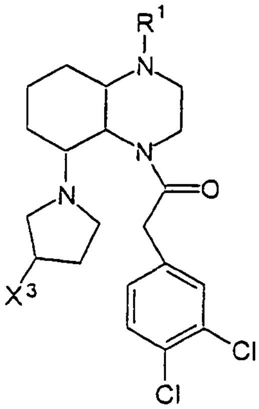

(3)

worin:

$R^1$ ist ausgewählt aus der Gruppe umfassend H; $C_1$-$C_5$-Alkyl; Phenylalkyl mit $C_1$-$C_4$-Alkyl und wobei der Phenylrest mit einer oder mehreren gleichen oder verschiedenen Gruppen ausgewählt aus der Gruppe umfassend Cl, OH und/oder $C_1$-$C_4$-Alkyloxy substituiert sein kann; N-Heteroarylalkyl, wobei der N-Heteroarylrest ausgewählt ist aus Pyridinyl, Imidazolyl, Pyrimidinyl, Pyrazinyl und/oder Pyrrolyl, und der Alkylrest ein $C_1$-$C_4$-Alkylrest ist; $C_1$-$C_5$-Acyl; Benzoyl; COO($C_1$-$C_5$-Alkyl); COO(Aryl); C(O)-$(CH_2)_q$-COOH wobei q ist 0, 1, 2, 3 oder 4 und/oder C(O)-$(CH_2)_t$-COO($C_1$-$C_5$-Alkyl) wobei r ist 0, 1, 2, 3 oder 4;

$X^3$ ist ausgewählt aus der Gruppe umfassend H, OH, Benzyl und/oder O-Arylacetyl, das mit zwei Cl-Gruppen substituiert sein kann.

**4.** Verbindungen nach einem der vorherigen Ansprüche, **dadurch gekennzeichnet, dass** die Verbindungen ein Gemisch umfassend Enantiomere gemäß den nachstehenden Formeln (1a) und/oder (1b):

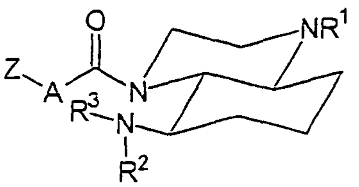

(1a)

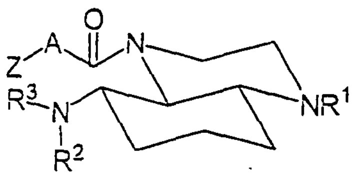

(1b)

umfassen.

**5.** Verbindung nach einem der vorherigen Ansprüche, **dadurch gekennzeichnet, dass** die Verbindung die nachstehende Formel (4) aufweist:

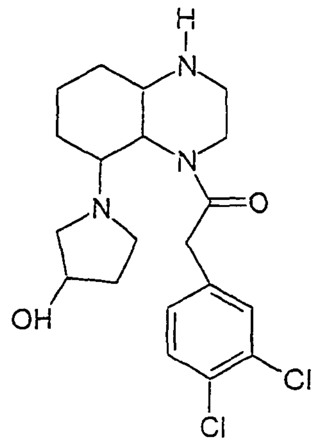

(4).

6. Verbindung nach einem der vorherigen Ansprüche, **dadurch gekennzeichnet, dass** die Verbindung die nachstehende Formel (6) aufweist:

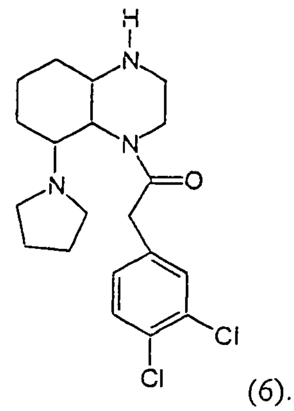

(6).

7. Verbindungen nach einem der vorherigen Ansprüche zur Verwendung als Arzneimittel.

8. Verbindungen zur Verwendung als Arzneimittel nach Anspruch 7 zur therapeutischen und/oder prophylaktischen Behandlung von Erkrankungen ausgewählt aus der Gruppe umfassend Schmerzerkrankungen: entzündliche Erkrankungen und/oder gastrointestinale Erkrankungen.

9. Verbindungen zur Verwendung als Arzneimittel nach Anspruch 8, **dadurch gekennzeichnet, dass** die Schmerzerkrankungen ausgewählt sind aus der Gruppe umfassend Rückenschmerzen, Gesichtsschmerzen, Kopfschmerzen, Gelenkschmerzen, muskuläre Schmerzsyndrome, entzündliche Schmerzerkrankungen, neuropathische Schmerzen, periphere Schrnerzen, periphere Nervenschädigungen, viscerale Schmerzen, Bauchschmerzen, Menstruationsbeschwerden, Nieren- und Gallensteinschmerzen, Juckreiz, Krebs- und Tumorschmerzen, sympatische Schmerzen, postoperative Schmerzen, postraumatische Schmerzen, Hyperalgesie und/oder inflammatorische Schmerzen.

10. Verbindungen zur Verwendung als Arzneimittel nach Anspruch 8, **dadurch gekennzeichnet, dass** die entzündlichen Erkrankungen ausgewählt sind aus der Gruppe umfassend entzündliche Erkrankungen des Gastrointestinaltraktes, entzündliche Darmerkrankungen wie Morbus Crohn und/oder Colitis ulcerosa, akute oder chronische entzündliche Veränderungen bei Gallenblarenentzündung, entzündliche Pseudopolypen, Colitis cystica profunda, Pneumatosis cystoides intestinales, Pankreatitis, Appendizitis, entzündlichen Erkrankungen der Gelenke wie rheumatoide Arthritis und/oder entzündliche Erkrankungen der Haut und der Augen.

11. Verbindungen zur Verwendung als Arzneimittel nach Anspruch 8, **dadurch gekennzeichnet, dass** die gastrointestinalen Erkrankungen ausgewählt sind aus der Gruppe umfassend Reizdarm, gastrische Läsionen, gastrointestinale

Ulzerationen, exogene und endogene Schädigungen der Masen-Darmmukosa, Fehlfunktionen des Gastrointestinaltraktes; Adenomen, insbesondere im Darm und/oder juvenile Polypen.

**12.** Verbindungen zur Verwendung als Arzneimittel nach Anspruch 7 zur therapeutischen und/oder prophylaktischen Behandlung von Juckreiz.

**13.** Arzneimittel umfassend wenigstens eine Verbindung nach einem der Anspruche 1-6 und/oder deren Racemate, Enantiomere, Diastereomere, Solvate, Hydrate sowie deren pharmazeutisch verträgliche Salze und/oder Ester.

**14.** Arzneimittel nach Anspruch 13, weiterhin umfassend wenigstens einen Opioid-Rezeptor-Antagonisten; vorzugsweise ausgewählt aus der Gruppe umfassend Naloxon. Naltrexon, Cyprodim; Naltrindol, Norbinaltorphimin, Nalmefen, Nalorphin, Nalbuphin, Naloxonazin, Methylnaltrexon und/oder Ketylcyclazocin.

**15.** Verfahren zur Herstellung einer Verbindung gemäß der allgemeinen Formel (1) nach einem der Ansprüche 1-6, **dadurch gekennzeichnet, dass** Verfahren die folgenden Schritte umfasst:

a) Cyclisierung von Nitromethan und Glutaraldehyd zu 2-Nitrocyclohexan-1,3-diol;
b) Aminierung des in Schritt a) erhaltenen Nitrodiols mit primären oder sekundären Amine;
c) Reduktion der Nitrogruppe des Nitrodiamins zu einem primären Amin;
d) Umsetzung des in Schritt c) erhaltenen Cyclohexantriamins mit Dialkyloxalat;
e) Abspaltung eines Aminrestes der in Schritt d) erhaltenen Verbindung;
f) Alkylierung der in Schritt e) erhaltenen Verbindung unter Einführen der Gruppen $R^2$ und $R^3$:
g) Reduktion des Perhydrochinoxalindion-Rings der in Schritt f) erhaltenen Verbindung zum Perhydrochinoxalin;
h) Acylierung des in Schritt g) erhaltenen sekundären Amins unter Einführen einer Gruppe C(O)-A-Z;
i) Einführung eines Restes $R^1$, vorzugsweise durch Alkylierung, Acylierung oder hydrogenolytische Einführung von H.

## Claims

**1.** A compound according to the general formula (I) as shown below and/or a racemate, enantiomer, diastereomer, solvate, hydrate thereof or a pharmaceutically acceptable salt and/or ester thereof:

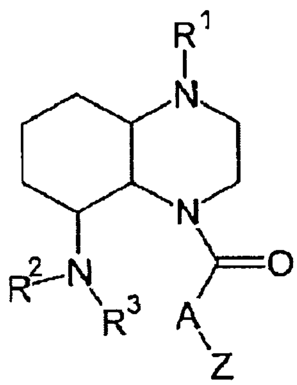

(1)

wherein:

$R^1$ is chosen from the group comprising H; $C_1$-$C_{10}$-alkyl; $C_3$-$C_{10}$-cycloalkyl; COO($C_1$-$C_{10}$-alkyl); $C_1$-$C_6$-alkoxycarbonyl; $C_1$-$C_6$-oxocarbonyl; phenylalkyl with $C_1$-$C_6$-alkyl, wherein the phenyl radical can be substituted by one or more identical or different groups chosen from the group comprising halogen, $C_1$-$C_6$-alkyloxy, $NH_2$, NH ($C_1$-$C_5$-alkyl), N($C_1$-$C_5$-alkyl)$_2$, OH, $SO_2$($C_1$-$C_5$-alkyl), SO($C_1$-$C_5$-alkyl) $CF_3$, CN,$NO_2$, $SO_2$N($C_1$-$C_5$-alkyl)$_2$, $SO_2$ $NH_2$, $SO_2$NH($C_1$-$C_5$-alkyl), $SO_2$NH (aryl), $SO_2$NH (phenyl) and/or $SO_2$NH (heteroaryl) ; $C_1$-$C_{10}$-acyl; $C_3$-$C_{10}$-cycloacyl; phenylacyl, wherein the acyl radical is a $C_1$-$C_6$-acyl radical and the phenyl radical can be substituted by one or more identical or different groups chosen from the group comprising halogen, $C_1$-$C_6$-alkyloxy, $NH_2$, NH($C_1$-$C_5$-alkyl), N($C_1$-$C_5$-alkyl)$_2$, OH, $SO_2$($C_1$-$C_5$-alkyl), SO($C_1$-$C_5$-alkyl), $CF_3$, CN, $NO_2$, $SO_2$N ($C_1$-$C_5$-alkyl)$_2$,

$SO_2NH_2$, $SO_2NH$ ($C_1$-$C_5$-alkyl), $SO_2NH$ (aryl), $SO_2NH$ (phenyl) and/or $SO_2NH$(heteroaryl); mono-, bi- or tricyclic heteroaryl containing one, two, three or four hetero atoms chosen from the group comprising N, O and/or S; mono-, bi- or tricyclic heteroarylalkyl containing one, two, three or four hetero atoms chosen from the group comprising N, O and/or S, wherein the alkyl radical is a $C_1$-$C_6$ alkyl radical; mono-, bi- or tricyclic heteroarylacyl containing one, two, three or four hetero atoms chosen from the group comprising N, O and/or S, wherein the acyl radical is a $C_1$-$C_6$-acyl radical; C(O) ($C_1$-$C_{10}$-alkyl) ; C(O)N($C_1$-$C_{10}$-alkyl)$_2$; C(O) ($C_3$-$C_{10}$-cycloalkyl) ; COO ($C_1$-$C_{10}$-alkyl); COO(aryl); COO ($C_3$-$C_{10}$-cycloalkyl) ; C(O)COO($C_1$-$C_{10}$-alkyl),; C(O)-$(CH_2)_q$-COOH, wherein q is 0, 1, 2, 3 or 4; C(O)-$(CH_2)_r$-COO($C_1$-$C_{10}$-alkyl), wherein r is 0, 1, 2, 3 or 4; C(O)-CH($NH_2$)-$(CH_2)_s$-COOH, wherein s is 0, 1, 2, 3 or 4; C(O)-CH($NH_2$)-$(CH_2)_t$-COO($C_1$-$C_{10}$-alkyl), wherein t is 0, 1, 2, 3 or 4; C(O)-$(CH_2)_u$-CH ($NH_2$)-COOH, wherein u is 0, 1, 2, 3 or 4 and/or C(O)-$(CH_2)_v$-CH($NH_2$)-COO($C_1$-$C_{10}$-alkyl), wherein v is 0, 1, 2, 3 or 4;

$R^2$, $R^3$ are in each case identical or independent of each other and are chosen from the group comprising H; $C_1$-$C_{10}$-alkyl; $C_3$-$C_{10}$-cycloalkyl; phenylalkyl with $C_1$-$C_6$-alkyl and wherein the phenyl radical can be substituted by one or more identical or different groups chosen from the group comprising halogen, $C_1$-$C_5$-alkyl, $C_1$-$C_4$-alkyloxy, $NH_2$, NH($C_1$-$C_5$-alkyl), N($C_1$-$C_5$-alkyl)$_2$, OH, COOH, COO($C_1$-$C_{10}$-alkyl), $CONH_2$, CONH($C_1$-$C_{10}$-alkyl), CON($C_1$-$C_{10}$-alkyl)$_2$, $SO_2$($C_1$-$C_5$-alkyl), $SO_2HN$($C_1$-$C_5$-alkyl), $CF_3$, CN and/or $NO_2$, or

$R^2$ and $R^3$ form, together with the nitrogen to which they are bonded, a saturated 3- to 8-membered N-heterocycle, wherein this can be substituted by one or more identical or different groups chosen from the group comprising OH, $C_1$-$C_4$-alkyloxy, carbonyl oxygen, $NH_2$, NH($C_1$-$C_5$-alkyl), N($C_1$-$C_5$-alkyl)$_2$, COOH, COO ($C_1$-$C_{10}$-alkyl), $CONH_2$, CONH($C_1$-$C_{10}$-alkyl), CON ($C_1$-$C_{10}$-alkyl)$_2$, $OPO_3H_2$, $OSO_3H$, $SO_2$($C_1$-$C_5$-alkyl), $SO_2HN$($C_1$-$C_5$-alkyl), CN, O-arylacetyl, O-phenylacetyl, arylacetoxy and/or acetylbenzyl, which can be substituted by two Cl groups;

A is $(CH_2)_n$, wherein n is 1;

Z is chosen from the group comprising phenyl, which can be substituted by one or more identical or different groups chosen from the group comprising halogen, $C_1$-$C_5$-alkyl, $C_1$-$C_5$-alkyloxy, $NH_2$, NH($C_1$-$C_5$-alkyl), N ($C_1$-$C_5$-alkyl)$_2$, OH, $SO_2$($C_1$-$C_5$-alkyl), SO($C_1$-$C_5$-alkyl), $CF_3$, CN, $NO_2$, $SO_2N$ ($C_1$-$C_5$-alkyl)$_2$, $SO_2NH_2$, $SO_2NH$ ($C_1$-$C_5$-alkyl), $SO_2NH$(aryl), $SO_2NH$(phenyl) and/or $SO_2NH$(heteroaryl); a mono- or bicyclic aryl or heteroaryl containing one or two hetero atoms chosen from the group comprising N, O and/or S, wherein the aryl or heteroaryl group can be substituted by one or more identical or different groups chosen from the group comprising halogen, $C_1$-$C_4$-alkyloxy, $NH_2$, NH($C_1$-$C_5$-alkyl), N($C_1$-$C_5$-alkyl)$_2$, OH, $SO_2$($C_1$-$C_5$-alkyl), SO($C_1$-$C_5$-alkyl), $CF_3$, CN, $NO_2$, $SO_2N$($C_1$-$C_5$-alkyl)$_2$, $SO_2NH_2$, $SO_2NH$($C_1$-$C_5$-alkyl), $SO_2NH$(aryl), $SO_2NH$(phenyl) and/or $SO_2NH$ (heteroaryl).

2. The compound as claimed in claim 1, **characterized in that** the compound has the following general formula (2):

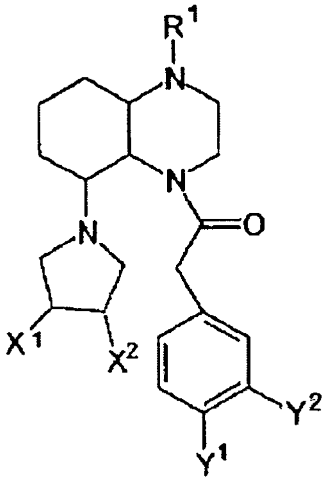

(2)

wherein:

$R^1$ is chosen from the group comprising H; $C_1$-$C_{10}$-alkyl; $C_3$-$C_{10}$-cycloalkyl; COO($C_1$-$C_{10}$-alkyl); $C_1$-$C_6$-alkoxycarbonyl; $C_1$-$C_6$-oxocarbonyl; phenylalkyl with $C_1$-$C_6$-alkyl, wherein the phenyl radical can be substituted by one or more identical or different groups chosen from the group comprising halogen, $C_1$-$C_6$-alkyloxy, $NH_2$, NH ($C_1$-$C_5$-alkyl), N($C_1$-$C_5$-alkyl)$_2$, OH, $SO_2$($C_1$-$C_5$-alkyl), SO($C_1$-$C_5$-alkyl), $CF_3$, CN, $NO_2$, $SO_2N$($C_1$-$C_5$-alkyl)$_2$,

$SO_2NH_2$, $SO_2NH$ ($C_1$-$C_5$-alkyl), $SO_2NH$ (aryl), $SO_2NH$ (phenyl) and/or $SO_2NH$ (heteroaryl) ; $C_1$-$C_{10}$-acyl; $C_3$-$C_{10}$-cycloacyl; phenylacyl, wherein the acyl radical is a $C_1$-$C_6$-acyl radical and the phenyl radical can be substituted by one or more identical or different groups chosen from the group comprising halogen, $C_1$-$C_6$-alkyloxy, $NH_2$, $NH(C_1$-$C_5$-alkyl), $N(C_1$-$C_5$-alkyl$)_2$, OH, $SO_2(C_1$-$C_5$-alkyl),$SO(C_1$-$C_5$-alkyl),$CF_3$, CN, $NO_2$, $SO_2N$ ($C_1$-$C_5$-alkyl$)_2$, $SO_2NH_2$, $SO_2NH(C_1$-$C_5$-alkyl), $SO_2NH$(aryl), $SO_2NH$(phenyl) and/or $SO_2NH$ (heteroaryl) ; mono-, bi- or tricyclic heteroaryl containing one, two, three or four hetero atoms chosen from the group comprising N, O and/or S; mono-, bi- or tricyclic heteroarylalkyl containing one, two, three or four hetero atoms chosen from the group comprising N, O and/or S, and the alkyl radical is a $C_1$-$C_6$-alkyl radical; mono-, bi- or tricyclic heteroarylacyl containing one, two, three or four hetero atoms chosen from the group comprising N, O and/or S, and the acyl radical is a $C_1$-$C_6$-acyl radical; C(O) ($C_1$-$C_{10}$-alkyl; C(O)N($C_1$-$C_{10}$-alkyl$)_2$; C(O) ($C_3$-$C_{10}$-cycloalkyl) ; COO ($C_1$-$C_{10}$-alkyl) ; COO(aryl) ; COO($C_3$-$C_{10}$-cycloalkyl); C(O)COO($C_1$-$C_{10}$-alkyl), C(O)-$(CH_2)_q$ -COOH, wherein q is 0, 1, 2, 3 or 4, C(O)-$(CH_2)_r$-COO($C_1$-$C_{10}$-alkyl), wherein r is 0, 1, 2, 3 or 4, C(O)-CH ($NH_2$)-$(CH_2)_s$-COOH, wherein s is 0, 1, 2, 3 or 4, C(O)-CH($NH_2$)-$(CH_2)_t$-COO($C_1$-$C_{10}$-alkyl), wherein t is 0, 1, 2, 3 or 4, C(O)-$(CH_2)_u$-CH($NH_2$)-COOH, wherein u is 0, 1, 2, 3 or 4, and/or C(O)-$(CH_2)_v$-CH($NH_2$)-COO ($C_1$-$C_{10}$-alkyl), wherein v is 0, 1, 2, 3 or 4;

$X^1$, $X^2$ are in each case identical or independent of each other and are chosen from the group comprising H, OH, carbonyl oxygen, $NH_2$, NH($C_1$-$C_5$-alkyl), N ($C_1$-$C_5$-alkyl$)_2$, COOH, COO ($C_1$-$C_{10}$-alkyl), $CONH_2$, CONH ($C_1$-$C_{10}$-alkyl), CON ($C_1$-$C_{10}$-alkyl$)_2$, $OPO_3H_2$, $OSO_3H$, $SO_2$($C_1$-$C_5$-alkyl), $SO_2HN$ ($C_1$-$C_5$-alkyl), $C_1$-$C_4$-alkyloxy, O-arylacetyl, O-phenylacetyl, arylacetoxy and/or acetylbenzyl, which can be substituted by two Cl groups;

$Y^1$, $Y^2$ are in each case identical or independent of each other and are chosen from the group comprising H, halogen, $C_1$-$C_5$-alkyl, $C_1$-$C_5$-alkyloxy, $NH_2$, NH ($C_1$-$C_5$-alkyl), NH(aryl), NH(phenyl), NH(heteroaryl), N($C_1$-$C_5$-alkyl$)_2$, OH, $SO_2$($C_1$-$C_5$-alkyl), SO($C_1$-$C_5$-alkyl), $CF_3$, CN, $NO_2$, $SO_2N$($C_1$-$C_5$-alkyl$)_2$, $SO_2NH_2$, $SO_2NH$($C_1$-$C_5$-alkyl).

3. The compound as claimed in claim 1 or 2, **characterized in that** the compound has the following general formula (3):

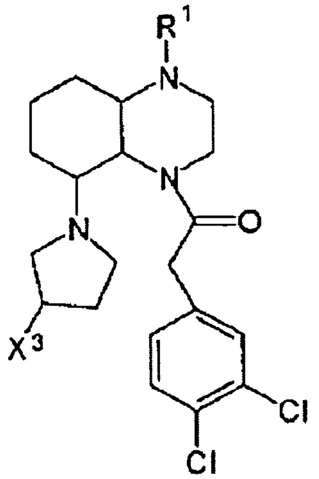

(3)

wherein:

$R^1$ is chosen from the group comprising H; $C_1$-$C_5$-alkyl; phenylalkyl with $C_1$-$C_4$-alkyl and wherein the phenyl radical can be substituted by one or more identical or different groups chosen from the group comprising Cl, OH and/or $C_1$-$C_4$-alkyloxy; N-heteroarylalkyl, wherein the N-heteroaryl radical is chosen from pyridinyl, imidazolyl, pyrimidinyl, pyrazinyl and/or pyrrolyl, and the alkyl radical is a $C_1$-$C_4$-alkyl radical; $C_1$-$C_5$-acyl; benzoyl; COO ($C_1$-$C_5$-alkyl) ; COO (aryl); C(O)-$(CH_2)_q$-COOH, wherein q is 0, 1, 2, 3 or 4 and/or C(O)-$(CH_2)_r$-COO ($C_1$-$C_5$-alkyl), wherein r is 0, 1, 2, 3 or 4;

$X^3$ is chosen from the group comprising H, OH, benzyl and/or O-arylacetyl, which can be substituted by two Cl groups.

4. The compound as claimed in one of the preceding claims, **characterized in that** the compound includes a mixture comprising enantiomers according to the following formulae (1a) and/or (1b):

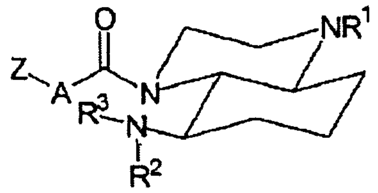
(1a)
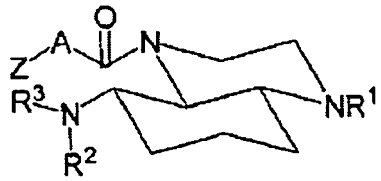
(1b).

5. The compound as claimed in one of the preceding claims, **characterized in that** the compound has the following formula (4):

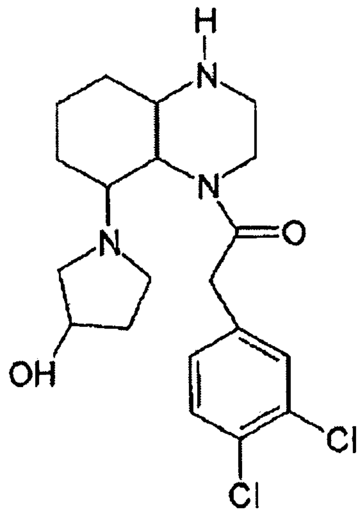
(4).

6. The compound as claimed in one of the preceding claims, **characterized in that** the compound has the following formula (6):

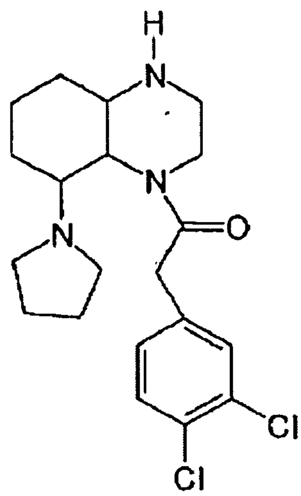
(6).

7. The compound as claimed in one of the preceding claims for use as a medicament.

8. The compound for use as a medicament as claimed in claim 7 for therapeutic and/or prophylactic treatment of

diseases chosen from the group comprising pain-related diseases, inflammatory diseases and/or gastrointestinal diseases.

**9.** The compound for use as a medicament as claimed in claim 8, **characterized in that** the pain-related diseases are chosen from the group comprising back pain, facial pain, headaches, joint pain, muscular pain syndromes, inflammatory pain-related diseases, neuropathic pain, peripheral pain, peripheral nerve damage, visceral pain, abdominal pain, menstruation symptoms, kidney- and gallstone pain, itching, cancer and tumor pain, sympathetic pain, postoperative pain, postraumatic pain, hyperalgesia and/or inflammatory pain.

**10.** The compound for use as a medicament as claimed in claim 8, **characterized in that** the inflammatory diseases are chosen from the group comprising inflammatory diseases of the gastrointestinal tract, inflammatory intestinal diseases, such as Crohn's disease and/or colitis ulcerosa, acute or chronic inflammatory changes with inflammation of the gall bladder, inflammatory pseudopolyps, colitis cystica profunda, pneumatosis cystoides intestinales, pancreatitis, appendicitis, inflammatory diseases of the joints, such as rheumatoid arthritis, and/or inflammatory diseases of the skin and of the eyes.

**11.** The compound for use as a medicament as claimed in claim 8, **characterized in that** the gastrointestinal diseases are chosen from the group comprising irritable bowel syndrome, gastric lesions, gastrointestinal ulcerations, exogenous and endogenous damage to the gastrointestinal mucosa, malfunctions of the gastrointestinal tract, adenomas, in particular in the intestine, and/or juvenile polyps.

**12.** The compound for use as a medicament as claimed in claim 7 for therapeutic and/or prophylactic treatment of itching.

**13.** A medicament comprising at least one compound as claimed in one of claims 1 to 6 and/or a racemate, enantiomer, diastereomer, solvate, hydrate thereof or a pharmaceutically acceptable salt and/or ester thereof.

**14.** The medicament as claimed in claim 13, further comprising at least one opioid receptor antagonist, preferably chosen from the group comprising naloxone, naltrexone, cyprodime, naltrindole, norbinaltorphimine nalmefene, nalorphine, nalbuphine, naloxonazine, methylnaltrexone and/or ketylcyclazocine.

**15.** A process for the preparation of a compound according to the general formula (1) as claimed in one of claims 1 to 6, **characterized in that** the process comprises the following steps:

a) cyclization of nitromethane and glutaraldehye to give nitrocyclohexane-1,3-diol;
b) amination of the nitrodiol obtained in step a) with primary or secondary amines;
c) reduction of the nitro group of the nitrodiamine to give a primary amine;
d) reaction of the cyclohexanetriamine obtained in step c) with dialkyl oxalate;
e) splitting off of an amine radical of the compound obtained in step d);
f) alkylation of the compound obtained in step e), with introduction of the groups $R^2$ and $R^3$;
g) reduction of the perhydroquinoxalinedione ring of the compound obtained in step f) to give the perhydroquinoxaline;
h) acylation of the secondary amine obtained in step g), with introduction of a group C(O)-A-Z;
i) introduction of a radical $R^1$, preferably by alkylation, acylation or hydrogenolytic introduction of H.

## Revendications

**1.** Composés selon la formule générale (1) telle qu'indiquée ci-après et/ou leurs racémiques, énantiomères, diastéréoisomères, solvates, hydrates ainsi que leurs sels et/ou esters pharmaceutiquement acceptables :

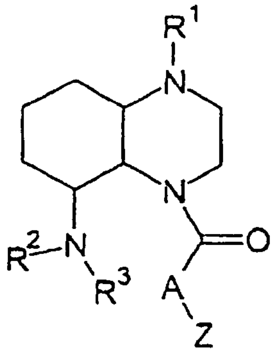

(1)

dans laquelle :

$R^1$ est choisi dans le groupe comprenant H ; alkyle en $C_1$-$C_{10}$ ; cycloalkyle en $C_3$-$C_{10}$ ; COO (alkyle en $C_1$-$C_{10}$); alkoxycarbonyle en $C_1$-$C_6$ ; oxocarbonyle en $C_1$-$C_6$ ; phénylalkyle avec alkyle en $C_1$-$C_6$, le radical phényle pouvant être substitué avec un ou plusieurs groupements identiques ou différents choisis dans le groupe comprenant halogène, alkyloxy en $C_1$-$C_6$, $NH_2$, NH (alkyle en $C_1$-$C_5$), N (alkyle en $C_1$-$C_5$)$_2$, OH, $SO_2$ (alkyle en $C_1$-$C_5$), SO(alkyle en $C_1$-$C_5$), $CF_3$, CN, $NO_2$, $SO_2$N(alkyle en $C_1$-$C_5$)$_2$, $SO_2NH_2$, $SO_2$NH (alkyle en $C_1$-$C_5$), $SO_2$NH (aryle), $SO_2$NH(phényle) et/ou $SO_2$NH (hétéroaryle) ; acyle en $C_1$-$C_{10}$ ; cycloacyle $C_3$-$C_{10}$ ; phénylacyle, le radical acyle étant un radical acyle en $C_1$-$C_6$ et le radical phényle pouvant être substitué avec un ou plusieurs groupements identiques ou différents choisis dansle groupe comprenant halogène, alkyloxy en $C_1$-$C_6$, $NH_2$, NH(alkyle en $C_1$-$C_5$), N(alkyle en $C_1$-$C_5$)$_2$, OH, $SO_2$(alkyle en $C_1$-$C_5$), SO(alkyle en $C_1$-$C_5$), $CF_3$, CN, $NO_2$, $SO_2$N(alkyle en $C_1$-$C_5$)$_2$, $SO_2NH_2$, $SO_2$NH (alkyle en $C_1$-$C_5$), $SO_2$NH(aryle), $SO_2$NH (phényle) et/ou $SO_2$NH (hétéroaryle) ; un hétéroaryle mono-, bi- ou tricyclique comprenant un, deux, trois ou quatre hétéroatomes choisis dans le groupe comprenant N, O et/ou S ; un hétéroarylalkyle mono-, bi- ou tricyclique comprenant un, deux, trois ou quatre hétéroatomes choisis dans le groupe comprenant N, O et/ou S, le radical alkyle étant un radical alkyle en $C_1$-$C_6$ ; un hétéroarylacyle mono-, bi- ou tricyclique comprenant un, deux, trois ou quatre hétéroatomes choisis dans le groupe comprenant N, O et/ou S, le radical acyle étant un radical acyle en $C_1$-$C_6$ ; C(O) (alkyle en $C_1$-$C_{10}$) ; C(O)N(alkyle en $C_1$-$C_{10}$)$_2$ ; C(O)(cycloalkyle en $C_3$-$C_{10}$) ; COO (alkyle en $C_1$-$C_{10}$) ; COO(aryle) ; COO(cycloalkyle en $C_3$-$C_{10}$) : C(O)COO(alkyle en $C_1$-$C_{10}$) ; C(O)-$(CH_2)_q$-COOH, q étant 0, 1, 2, 3 ou 4 ; C(O)-$(CH_2)_r$-COO(alkyle en $C_1$-$C_{10}$), r étant 0, 1, 2, 3 ou 4 ; C(O)-CH($NH_2$)-$(CH_2)_s$-COOH, s étant 0, 1, 2, 3 ou 4 ; C(O)-CH($NH_2$)-$(CH_2)_t$-COO(alkyle en $C_1$-$C_{10}$), t étant 0, 1, 2, 3 ou 4 ; C(O)-$(CH_2)_u$-CH($NH_2$)-COOH, u étant 0, 1, 2, 3 ou 4, et/ou C(O)-$(CH_2)_v$-CH($NH_2$)-COO(alkyle en $C_1$-$C_{10}$), v étant 0, 1, 2, 3 ou 4 ;

$R^2$, $R^3$ étant choisis, indépendamment l'un de l'autre, dans le groupe comprenant H ; alkyle en $C_1$-$C_{10}$ ; cycloalkyle en $C_1$-$C_{10}$ ; phénylalkyle avec alkyle en $C_1$-$C_6$, le radical phényle pouvant être substitué avec un ou plusieurs groupements identiques ou différents choisis dans le groupe comprenant halogène, alkyle en $C_1$-$C_5$, alkyloxy en $C_1$-$C_4$, $NH_2$, NH (alkyle en $C_1$-$C_5$), N (alkyle en $C_1$-$C_5$)$_2$, OH, COOH, COO(alkyle en $C_1$-$C_{10}$), $CONH_2$, CONH (alkyle en $C_1$-$C_{10}$), CON (alkyle en $C_1$-$C_{10}$)$_2$, $SO_2$(alkyle en $C_1$-$C_5$), $SO_2$NH(alkyle en $C_1$-$C_5$), $CF_3$, CN et/ou $NO_2$,

ou

$R^2$ et $R^3$ forment, ensemble avec l'azote auquel ils sont liés, un hétérocycle saturé constitué de 3 à 8 chaînons, ce dernier pouvant être substitué avec un ou plusieurs groupements identiques ou différents choisis dans le groupe comprenant OH, alkyloxy en $C_1$-$C_4$, carbonyl-oxygène, $NH_2$, NH(alkyle en $C_1$-$C_5$), N(alkyle en $C_1$-$C_5$)$_2$, COOH, COO(alkyle en $C_1$-$C_{10}$), $CONH_2$, CONH (alkyle en $C_1$-$C_{10}$), CON (alkyle en $C_1$-$C_{10}$)$_2$, $OPO_3H_2$, $OSO_3H$, $SO_2$(alkyle en $C_1$-$C_5$), $SO_2$HN(alkyle en $C_1$-$C_5$), CN, O-arylacétyle, O-phénylacetyle, arylacétoxy et/ou acétylbenzyle lequel être substitué avec deux groupements Cl ;

A est $(CH_2)_n$, n étant 1 ;

Z est choisi dans le groupe comprenant phényle pouvant être substitué avec un ou plusieurs groupements identiques ou différents choisis dans le groupe comprenant halogène, alkyle en $C_1$-$C_5$, alkyloxy en $C_1$-$C_5$, $NH_2$, NH(alkyle en $C_1$-$C_5$), N (alkyle en $C_1$-$C_5$)$_2$, OH, $SO_2$(alkyle en $C_1$-$C_5$), SO(alkyle en $C_1$-$C_5$), $CF_3$, CN, $NO_2$, $SO_2$N(alkyle en $C_1$-$C_5$)$_2$, $SO_2NH_2$, $SO_2$NH(alkyle en $C_1$-$C_5$), $SO_2$NH(aryle), $SO_2$NH (phényle) et/ou $SO_2$NH (hétéroaryle) ; un aryle ou hétéroaryle mono- ou bicyclique comprenant un ou deux hétéroatomes choisis dans le groupe comprenant N, O et/ou S, le groupement aryle ou hétéroaryle pouvant être substitué avec un ou plusieurs groupements identiques ou différents choisis dans le groupe comprenant halogène, alkyloxy en $C_1$-$C_4$, $NH_2$, NH(alkyle en $C_1$-$C_5$), N(alkyle en $C_1$-$C_5$)$_2$, OH, $SO_2$(alkyle en $C_1$-$C_5$), SO(alkyle en $C_1$-$C_5$), $CF_3$, CN, $NO_2$,

$SO_2N$(alkyle en $C_1$-$C_5$)$_2$, $SO_2NH_2$, $SO_2NH$(alkyle en $C_1$-$C_5$), $SO_2NH$(aryle), $SO_2NH$(phényle) et/ou $SO_2NH$ (hétéroaryle).

2. Composés selon la revendication 1, **caractérisé en ce que** lesdits composés présentent la formule générale (2) ci-après :

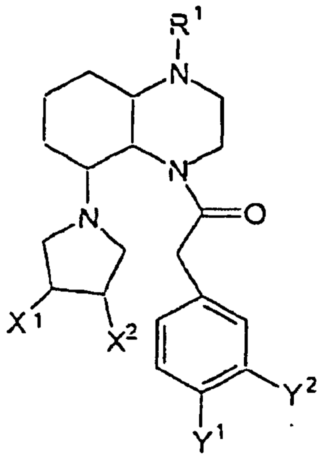

(2)

dans laquelle :

$R^1$ est choisi dans le groupe comprenant H ; alkyle en $C_1$-$C_{10}$ ; cycloalkyle en $C_3$-$C_{10}$ ; COO (alkyle en $C_1$-$C_{10}$) ; alkoxycarbonyle en $C_1$-$C_6$ ; oxycarbonyle en $C_1$-$C_6$ ; phénylalkyle avec alkyle en $C_1$-$C_6$, le radical phényle pouvant être substitué avec un ou plusieurs groupements identiques ou différents choisis dans le groupe comprenant halogène, alkyloxy en $C_1$-$C_6$, $NH_2$, NH(alkyle en $C_1$-$C_5$), N (alkyle en $C_1$-$C_5$)$_2$, OH, $SO_2$(alkyle en $C_1$-$C_5$), SO(alkyle en $C_1$-$C_5$), $CF_3$, CN, $NO_2$, $SO_2N$ (alkyle en $C_1$-$C_5$)$_2$, $SO_2NH_2$, $SO_2NH$(alkyle en $C_1$-$C_5$), $SO_2NH$ (aryle), $SO_2NH$(phényle) et/ou $SO_2NH$(hétéroaryle) ; acyle en $C_1$-$C_{10}$ ; cycloacyle $C_3$-$C_{10}$ ; phénylacyle, le radical acyle étant un radical acyle en $C_1$-$C_6$ et le radical phényle pouvant être substitué avec un ou plusieurs groupements identiques ou différents choisis dans le groupe comprenant halogène, alkyloxy en $C_1$-$C_6$, $NH_2$, NH(alkyle en $C_1$-$C_5$), N(alkyle en $C_1$-$C_5$)$_2$, OH, $SO_2$(alkyle en $C_1$-$C_5$), SO(alkyle en $C_1$-$C_5$), $CF_3$, CN, $NO_2$, $SO_2N$(alkyle en $C_1$-$C_5$)$_2$, $SO_2NH_2$, $SO_2NH$(alkyle en $C_1$-$C_5$), $SO_2NH$(aryle), $SO_2NH$ (phényle) et/ou $SO_2NH$ (hétéroaryle) ; un hétéroaryle mono-, bi- ou tricyclique comprenant un, deux, trois ou quatre hétéroatomes choisis dans le groupe comprenant N, O et/ou S ; un hétéroarylalkyle mono-, bi- ou tricyclique comprenant un, deux, trois ou quatre hétéroatomes choisis dans le groupe comprenant N, O et/ou S, et le radical alkyle étant un radical alkyle en $C_1$-$C_6$ ; un hétéroarylacyle mono-, bi- ou tricyclique comprenant un, deux, trois ou quatre hétéroatomes choisis dans le groupe comprenant N, O et/ou S, et le radical acyle étant un radical acyle en $C_1$-$C_6$ ; C(O) (alkyle en $C_1$-$C_{10}$) ; C(O)N(alkyle en $C_1$-$C_{10}$)$_2$ ; C(O)(cycloalkyle en $C_3$-$C_{10}$) ; COO(alkyle en $C_1$-$C_{10}$) ; COO(aryle) ; COO(cycloalkyle en $C_3$-$C_{10}$) ; C(O)COO(alkyle en $C_1$-$C_{10}$) ; C(O)-$(CH_2)_q$-COOH, q étant 0, 1, 2, 3 ou 4 ; C(O)-$(CH_2)_r$-COO (alkyle en $C_1$-$C_{10}$), r étant 0, 1, 2, 3 ou 4 ; C(O)-CH($NH_2$)-$(CH_2)_s$-COOH, s étant 0, 1, 2, 3 ou 4 ; C(O)-CH($NH_2$)-$(CH_2)_t$-COO(alkyle en $C_1$-$C_{10}$), t étant 0, 1, 2, 3 ou 4 ; C(O)-$(CH_2)_u$-CH ($NH_2$)-COOH, u étant 0, 1, 2, 3 ou 4, et/ou C(O)-$(CH_2)_v$-CH($NH_2$)-COO(alkyle en $C_1$-$C_{10}$), v étant 0, 1, 2, 3 ou 4 ;
$X^1$, $X^2$ sont identiques ou, indépendamment l'un de l'autre, choisis dans le groupe comprenant H, OH, carbonyloxygène, $NH_2$, NH(alkyle en $C_1$-$C_5$), N(alkyle en $C_1$-$C_5$)$_2$, COOH, COO (alkyle en $C_1$-$C_{10}$), $CONH_2$, CONH (alkyle en $C_1$-$C_{10}$), CON(alkyle en $C_1$-$C_{10}$)$_2$, $OPO_3H_2$, $OSO_3H$, $SO_2$(alkyle en $C_1$-$C_5$), $SO_2HN$ (alkyle en $C_1$-$C_5$), alkyloxy en $C_1$-$C_4$, O-arylacétyle, O-phénylacetyle, arylacétoxy et/ou acétylbenzyle lequel être substitué avec deux groupement Cl ;
$Y^1$, $Y^2$ sont identiques ou, indépendamment l'un de l'autre, choisis dans le groupe comprenant H, halogène, alkyle en $C_1$-$C_5$, alkyloxy en $C_1$-$C_5$, $NH_2$, NH (alkyle en $C_1$-$C_5$), NH(aryle), NH(phényle), NH(hétéroaryle), N (alkyle en $C_1$-$C_5$)$_2$, OH, $SO_2$(alkyle en $C_1$-$C_5$), SO(alkyle en $C_1$-$C_5$), $CF_3$, CN, $NO_2$, $SO_2N$(alkyle en $C_1$-$C_5$)$_2$, $SO_2NH_2$, $SO_2NH$ (alkyle en $C_1$-$C_5$).

3. Composés selon les revendications 1 ou 2, **caractérisés en ce que** lesdits composés présentent la formule générale (3) ci-après :

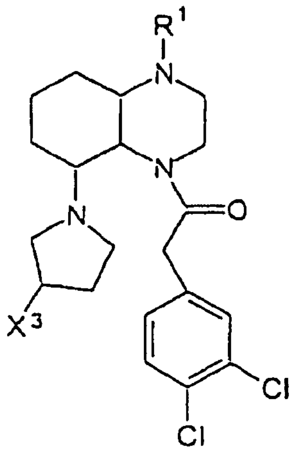

(3)

dans laquelle :

$R^1$ est choisi dans le groupe comprenant H ; alkyle en $C_1$-$C_5$ ; phénylalkyle avec alkyle en $C_1$-$C_4$, et le radical phényle pouvant être substitué avec un ou plusieurs groupements identiques ou différents choisis dans le groupe comprenant Cl, OH et/ou alkyloxy en $C_1$-$C_4$ ; N-hétéroarylalkyle, le radical N-hétéroarylalkyle pouvant être choisi parmi pyridinyle, imidazolyle, pyrimidinyle, pyrazinyle et/ou pyrrolyle, et le radical alkyle étant un radical alkyle en $C_1$-$C_4$ ; acyle en $C_1$-$C_5$ ; benzoyle ; COO(alkyle en $C_1$-$C_5$) ; C(O)-$(CH_2)_q$-COOH, q étant 0, 1, 2, 3 ou 4, et/ou C(O)-$(CH_2)_r$-COO(alkyle en $C_1$-$C_5$), r étant 0, 1, 2, 3 ou 4.
$X^3$ est choisi dans le groupe comprenant H, OH, benzyle et/ou O-arylacétyle, lequel peut être substitué avec deux groupes Cl.

4. Composés selon l'une des revendications précédentes, **caractérisés en ce que** lesdits composés comprennent un mélange comprenant des énantiomères selon les formules (1a) et/ou (1b) ci-après :

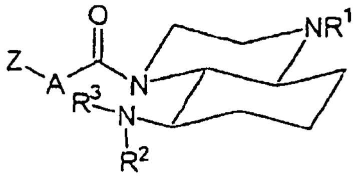

(1a)

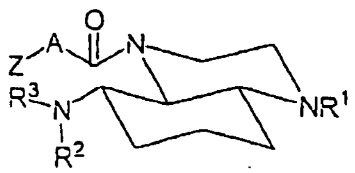

(1b)

5. Composé selon l'une des revendications précédentes, **caractérisé en ce que** ledit composé présente la formule (4) ci-après :

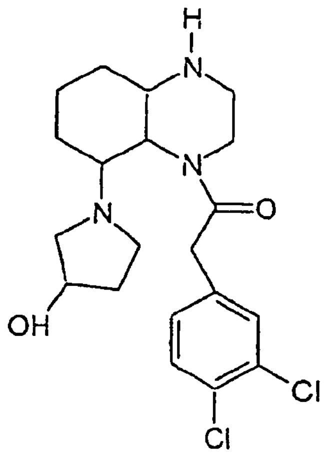

(4).

**6.** Composé selon l'une des revendications précédentes, **caractérisé en ce que** ledit composé présente la formule (6) ci-après :

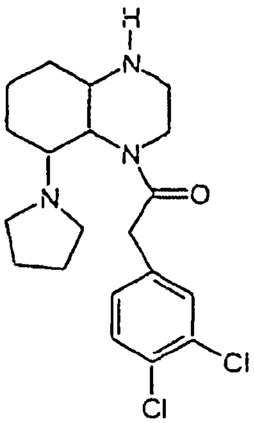

(6).

**7.** Composés selon l'une des revendications précédentes, destinés à être utilisés en tant que médicament.

**8.** Composés destinés à être utilisés en tant que médicament selon la revendication 7, destinés au traitement thérapeutique et/ou prophylactique d'affections choisies dans le groupe comprenant les affections douloureuses, les affections inflammatoires et/ou les affections gastro-intestinales.

**9.** Composés destinés à être utilisés en tant que médicament selon la revendication 8, **caractérisés en ce que** lesdites affections douloureuses sont choisies dans le groupe comprenant le mal de dos, les douleurs faciales, les céphalées, les anthralgies, les syndromes douloureux musculaires, les affections douloureuses inflammatoires, les douleurs neuropathiques, les douleurs périphériques, les lésions neuronales, les douleurs viscérales, les douleurs abdominales, la dysménorrhée, les douleurs dues aux calculs rénales et biliaires, le prurit, les douleurs dues aux cancers et tumeurs, les causalgies, les douleurs postopératoires, les douleurs post-traumatiques, l'hyperalgésie et/ou les douleurs inflammatoires.

**10.** Composés destinés à être utilisés en tant que médicament selon la revendication 8, **caractérisés en ce que** lesdites affections inflammatoires sont choisies dans le groupe comprenant les affections inflammatoires du système gastro-intestinal, les affections inflammatoires de l'intestin telles que la maladie de Crohn et/ou la rectocolite hémorragique, les évolutions inflammatoires aiguës ou chroniques associées à la cholécystite, les inflammations dues aux pseudopolypes, la colite kystique profonde, la pneumatose kystique intestinale, la pancréatite, l'appendicite, les affections

inflammatoires des articulations telles que la polyarthrite rhumatoïde et/ou les affections inflammatoires de la peau et des yeux.

**11.** Composés destinés à être utilisés en tant que médicament selon la revendication 8, **caractérisés en ce que** lesdites affections gastro-intestinales sont choisies dans le groupe comprenant le côlon irritable, les ulcérations gastro-intestinales, les lésions exogènes et endogènes de la muqueuse gastrique, les dysfonctionnements du système gastro-intestinal, les adénomes situés notamment dans intestin, et/ou les polypes juvéniles.

**12.** Composés destinés à être utilisés en tant que médicament selon la revendication 7, destiné au traitement thérapeutique et/ou prophylactique du prurit.

**13.** Médicament comprenant au moins un composé selon l'une des revendications 1 à 6 et/ou leurs racémiques, énantiomères, diastéréoisomères, solvates, hydrates ainsi que leurs sels et/ou esters pharmaceutiquement acceptables.

**14.** Médicament selon la revendication 13, comprenant en outre au moins un antagoniste de récepteurs opiacés, choisi de préférence dans le groupe comprenant la naloxone, la naltrexone, le cyprodime, le naltrindole, la norbinaltorphimine, le nalméfène, la nalorphine, la nalbuphine, la méthylnaltrexone et/ou la cétylcyclazocine.

**15.** Procédé destiné à la préparation d'un composé selon la formule générale (1) selon l'une des revendications 1 à 6, **caractérisé en ce que** ledit procédé comprend les étapes suivantes :

a) la cyclisation de nitrométhane et de glutaraldéhyde pour obtenir le 2-nitrocyclohexane-1,3-diol ;
b) l'amination du nitrodiol obtenu à l'étape a) en mettant en oeuvre des amines primaires ou secondaires ;
c) la réduction du groupement nitro de la nitrodiamine pour obtenir une amine primaire ;
d) la réalisation d'une réaction entre la cyclohexanetriamine obtenue à l'étape c) et l'oxalate de dialkyle ;
e) l'élimination d'un radical aminé que renferme le composé obtenu à l'étape d) ;
f) l'alkylation du composé obtenu à l'étape e) en introduisant les groupements $R^2$ et $R^3$ ;
g) la réduction du cycle de perhydroquinoxalindione du composé obtenu à l'étape f) pour obtenir de la perhydroquinoxaline ;
h) l'acylation de l'amine secondaire obtenue à l'étape g) en introduisant un groupement C(O)-A-Z;
i) l'introduction du radical $R^1$, de préférence par alkylation, acylation ou introduction hydrogénolytique de H.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20020042399 A1
- US 6303611 B1


### In der Beschreibung aufgeführte Nicht-Patentliteratur


- **REES et al.** *Journal of hetereocyclic chemistry,* 01. Januar 1987, vol. 24, 1297-1300
- **HUNTER et al.** *Br. J. Pharmacol.,* 1990, vol. 1001, 183-189
- **SMITH et al.** *J. Neuoch.,* 1989, vol. 53, 27-36
- **C. HENDERSHOT ; J. FORSAITH.** *J. Pharmacol. Exp. Ther.,* 1959, vol. 125, 237-240
- **GATTERMANN, L ; WIELAND, H. ; WIELAND, T. ; SUCROW, W.** Die Praxis des organischen Chemikers. Walter de Gryter, 1982, 555
- **HENDERSHOT, L. C. ; FORSAITH, J.** *J. Pharmacol. Exp. Ther.,* 1959, vol. 125, 237-240
- **CHRISTOPH, T. ; KÖGEL, B. ; SCHIENE, K. ; MÉEN, M. ; DE VRY, J. ; FRIEDRICHS, E.** *Eur. J. Pharmacol.,* 2005, vol. 507, 87-98
- **CHENG, Y. C. ; PRUSOFF, W. H.** *Biochem. Pharmacol.,* 1973, vol. 22, 3099-3108